# EUROPEAN PATENT APPLICATION

(11) **EP 4 552 639 A1**
(43) Date of publication of application: **14.05.2025**
(21) Application number: 23209032.4
(22) Date of filing: 10.11.2023
(51) Int. Cl.: A61K 31/381, A61K 31/44, A61K 31/4409, A61K 31/445, A61K 31/4462, A61K 31/4965, A61K 31/5375, A61K 31/55, A61P 35/00

(54) **COMPOUNDS FOR USE IN THE TREATMENT OF GASTROINTESTINAL STROMAL TUMOR (GIST)**

(71) Applicant: Universität Duisburg-Essen, 45141 Essen (DE); Technische Universität Dortmund, 44227 Dortmund (DE); Max-Planck-Gesellschaft zur Förderung der Wissenschaften e.V., 80539 München (DE)
(72) Inventor: BAUER, Sebastian, 45122 Essen (DE); MUEHLENBERG, Thomas, 45472 Mülheim an der Ruhr (DE); RAUH, Daniel, 44227 Dortmund (DE); SIEVERS, Sonja, 44143 Dortmund (DE); TEUBER, Alina, 44143 Dortmund (DE); SCHULZ, Tom, 44379 Dortmund (DE); WARMUTH, Jonas, 45699 Herten (DE); LATEGHAN, Jonas, 44141 Dortmund (DE)
(74) Representative: Maiwald GmbH

(57) **Abstract**

The present invention relates to method of treating a patient suffering from a gastrointestinal stromal tumor (GIST), comprising administering to said patient a pharmaceutically effective amount of a compound of formula (I) or a pharmaceutically acceptable salt thereof. Furthermore, the present invention relates to a compound of formula (I) or a pharmaceutically acceptable salt thereof for use in the treatment of a gastrointestinal stromal tumor (GIST) in a patient.

## Description

### Field of the invention

The present invention relates to method of treating a patient suffering from a gastrointestinal stromal tumor (GIST), comprising administering to said patient a pharmaceutically effective amount of a compound of formula (I) or a pharmaceutically acceptable salt thereof. Furthermore, the present invention relates to a compound of formula (I) or a pharmaceutically acceptable salt thereof for use in the treatment of a gastrointestinal stromal tumor (GIST) in a patient.

### Background of the invention

Gastrointestinal stromal tumors (GIST) comprise less than 1 % of all gastrointestinal (GI) tumors but constitute the most common mesenchymal tumors and soft tissue sarcomas of the GI tract. They occur anywhere along the GI tract but are found most often in the stomach (60 %) or small intestine (30 %) and less frequently in the rectum, colon, or mesentery. In the United States, around 3300 to 6000 new cases of GIST are diagnosed each year. The vast majority of cases are sporadic, and older age is a recognized risk factor. Mutations in tyrosine kinase (KIT) and platelet-derived growth factor receptor-alpha (PDGFRA) are found in over 80 % of all primary GISTs. Alterations in neurofibromatosis type 1 gene (NF1) and succinate dehydrogenase (SDH) complex (SDHC) genes as well as altered methylation of SDHC promoter have been described as oncogenic drivers in GIST without activating mutations in KIT or PDGFRA, and they have been linked to familial and heritable syndromes (NF1 and Carney-Stratakis syndrome).

Despite a wide variation in tumor size, location, and histologic subtypes (spindle cell, epithelioid cells, and mixed type), approximately 85 % of all GISTs share oncogenic mutations in 1 of 2 receptor tyrosine kinases (TKs): KIT or PDGFRA. Constitutive activation of either of these TKs plays a central role in the oncogenic behavior of GIST. The early characterization of GIST mutational status is important in both the localized and metastatic settings to identify drug-resistant mutations, for example imatinib-resistant mutations, such as some primary KIT exon 17 mutations or PDGFRA D842V, or mutations that require a higher dose of a kinase inhibitor drug, *e.g.,* imatinib. Characterization of the mutational status can also be helpful to discriminate against other less frequent mutations that may be present in these patients and drive GIST development, with the largest group represented by SDH-deficiency frequently associated with Carney or Carney-Stratakis-Syndrome. Other subtypes have mutations in NF1 (usually associated with neurofibromatosis type I) or in BRAF or KRAS. Very recently, casuistic cases of GIST-like tumors harboring NTRK translocations have further expanded the spectrum of molecular subtypes.

In the pre-tyrosine kinase inhibitor era, GISTs (often categorized as gastric leiomyosarcomas or leiomyoblastomas) were treated within the subtype of agnostic sarcoma trials and lacked an effective systemic therapy. However, a deeper understanding of the molecular pathogenesis and driving role of the protooncogenes KIT and PDGFRA has transformed the treatment of both localized and metastatic diseases. Localized and resectable tumors are treated surgically which remains the mainstay of curative therapy for localized disease. Resected high-risk GIST is typically treated with adjuvant imatinib, whereas low-risk GIST is managed with surgery alone. Intermediate-risk GIST is managed on a per-case basis. In an advanced/metastatic setting, the established and approved treatment is imatinib 400 mg daily, with dose escalation to 800 mg at the time of progression. Said treatment regimen has been shown to provide for dramatic increases in disease control. Imatinib-refractory patients are currently treated with sunitinib as a second-line therapy and regorafenib as third-line therapy, the latter in particular in cases of resistance or intolerance to sunitinib.

At diagnosis, a mutation in the KIT gene is found in 80 % of GISTs and usually occurs in exon 11 and less commonly in exon 9. Both mutations cause ligand-independent receptor activation, which leads to uncontrolled cell growth and transformation. Primary mutations are typically a gain-of-function mutation in the juxtamembrane (JM) domain and lead to a shift in equilibrium toward a Type I active or on-state conformation of KIT and away from a Type II inactive or off-state conformation of KIT. Exon 11 primary mutations are the ones most commonly seen in GISTs (around 70 % of cases) and patients derive significant benefit from treatment with imatinib in both the adjuvant and metastatic settings, achieving a 5-year overall survival of 89-100% (depending on type of exon 11 mutation) in the adjuvant setting, and a median event-free survival just under 2 years in the metastatic setting. Primary mutations (in treatment-naive patients) in exon 9 affect the extracellular (EC) domain of KIT, mimicking conformational changes induced by ligand binding and triggering KIT receptor homodimerization. This dimerization leads to the activation of specific intracellular signaling pathways which can lead to cancer cell proliferation, survival, and resistance. Although less common than exon 11 mutations, exon 9 mutations (10 % - 15 % of newly diagnosed cases) are most commonly seen in GISTs arising from the small intestine. Unlike exon 11 mutations, they benefit less from imatinib in both the adjuvant and metastatic setting.

Despite significant improvement in outcomes compared with those in the pre-mutation-driven/tyrosine kinase inhibitor therapy era, response to imatinib is not experienced by all patients, and most patients with GIST will ultimately develop resistance to imatinib, most commonly due to the development of secondary mutations in KIT. Secondary resistance mutations usually arise in the catalytic domain of the kinase: 1) at the ATP-binding pocket, which typically occur in KIT exons 13 and 14 and impede drug binding by decreasing van der Waals interactions thereby reducing binding affinity or by steric repulsion due to the more demanding and lipophilic isoleucine; and 2) in the activation loop (AL) encoded by KIT exons 17 and 18 and PDGFRA 18. AL mutations act by shifting the kinase into an activated Type I or on-state conformation which precludes imatinib and sunitinib from occupying the binding pocket. In contrast, ripretinib can is highly effective to inhibit secondary KIT mutations involving the activation loop. Although uncommon in primary GIST (1 % - 2 % of newly diagnosed cases), mutations in exons 13, 14 and 17 are often responsible for acquired imatinib resistance, with exon 17 mutations alone accounting for as many as 50 % of the acquired resistance cases to imatinib, and later to sunitinib.

Very recently, a novel mechanism of late resistance has been described. As a consequence of effective inhibition of KIT AL mutations by ripretinib, compound mutations may emerge that consist of AL and AP mutation occurring on the same allele ("in cis"). These mutations are resistant to all approved KIT inhibitors or experimental KIT inhibitors currently under clinical investigation. These may occur as triple-in-cis (primary KIT exon 11 mutations) or double-in-cis mutations (primary KIT exon 17 or PDGFRA exon 18 mutations) depending on the location of the primary mutation.

An unmet need exists for tyrosine kinase inhibitors (TKIs) that can broadly inhibit KIT and PDGFRA having clinically relevant mutations, particularly secondary and tertiary mutations resulting in acquired drug resistance to established TKIs in the treatment of GIST, such as imatinib and sunitinib.

### Summary of the invention

The existing demand for a TKI that can inhibit KIT and PDGFRA having clinically relevant mutations that cause resistance to existing TKIs is met by the present invention and is based on the finding that the compounds disclosed herein are highly effective in inhibiting KIT and PDGFRA having accumulated clinically relevant mutations that commonly lead to escape from established treatment regimens.

In a first aspect, provided herein are therefore methods of treating a patient suffering from a gastrointestinal stromal tumor (GIST), comprising administering to the patient a therapeutically effective amount of a compound of formula (I) or a pharmaceutically acceptable salt thereof, 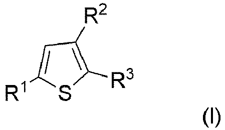 wherein
R¹ is selected from aryl and heterocyclyl, provided R¹ is not thienyl, and wherein R¹ can be optionally substituted on one or more carbon atoms by one or more R⁶; and further wherein if said heterocyclyl contains an -NH- moiety, the nitrogen of said moiety can be optionally substituted by a group selected from R7;
R² is -NHC(=O)NH₂;
R³ is -C(=O)NR⁴R⁵;
R⁴ is cycloalkyl or a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷;
R⁵ is H or taken together with R⁴ and the nitrogen atom to which it is attached form a heterocyclic ring, wherein said heterocyclic ring is selected from the group consisting of 4-, 5-, 6-, and 7-membered heterocyclic rings, wherein said heterocyclic ring can be optionally substituted on one or more carbon atoms by one or more R⁶, and wherein if said heterocyclyl contains a -NH- moiety, the nitrogen of said moiety can be optionally substituted by a group selected from R⁷;
each R⁶ and R⁷ is independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHSO₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁸R⁹, mercapto, -S(C₁₋₆ alkyl), -SO(C₁₋₆ alkyl), -S0₂(C₁₋₆ alkyl), -SO₂-NR⁸R⁹, wherein R⁶ and R⁷ independently of each other can be optionally substituted on one or more carbon atoms by one or more R¹⁰ and on nitrogen of any moiety that contains an NH or NH₂ by R¹¹;
R⁸ and R⁹ are each independently selected from halo, nitro, cyano, isocyano, C₁₋₆ alkyl, C₂₋₆ alkenyl, C₂₋₆ alkynyl, aryl, cycloalkyl, heterocyclyl, hydroxy, oxo(=O), -O(C1-₆ alkyl), -Oaryl, - OC(=O)alkyl, -CHO, -C(=O)NR¹²R¹³, -C(=O)(C₁₋₆ alkyl), -C(=O)heterocyclyl, -C(=O)cycloalkyl, -COOH, - C(=O)O(C₁₋₆ alkyl), -C(=O)Oaryl, -C(=O)ONR¹²R¹³, mercapto, -S(C₁₋₆ alkyl), -SO(C₁₋₆ alkyl), -S0₂(C₁₋₆ alkyl), -SO₂-NR¹²R¹³;
R¹⁰ and R¹¹ are independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), - NHC(=O)OH, -N(C₁₋₆ alkyl)C(=O)(C₁₋₆ alkyl), -NHS0₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁷R⁸, mercapto, -S(C₁₋₆ alkyl), -SO(C₁₋₆ alkyl), -S0₂(C₁₋₆ alkyl), -SO₂-NR⁸R⁹; and
R¹² and R¹³ are independently selected from H, C₁₋₆ alkyl, cycloalkyl, aryl, heterocyclyl; alternatively R¹² and R¹³ together with the nitrogen atom to which they are attached form a heterocyclic ring.

In various embodiments, R¹ is selected from aryl optionally substituted on one or more carbon atoms by one or more R⁶.

In various embodiments, R¹ is phenyl optionally substituted on one or more carbons atoms by one or more R⁶, wherein R⁶ is preferably halo, more preferably fluoro.

In various embodiments, R¹ is 3-fluorophenyl.

In some embodiments, R⁵ is H.

In various embodiments, R⁴ is a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷.

In various further embodiments, R⁴ is a 6-membered heterocyclyl ring, preferably 3-piperidinyl, optionally substituted on one or more carbon atoms by one or more R⁶, and wherein the nitrogen of the a -NH- moiety can be optionally substituted by a group selected from R⁷.

In some embodiments, the compound is 5-(3-fluorophenyl)-N-(piperidin-3-yl)-3-ureidothiophene-2-carboxamide or a pharmaceutically acceptable salt thereof, preferably (S)-5-(3-fluorophenyl)-N-(piperidin-3-yl)-3-ureidothiophene-2-carboxamide or a pharmaceutically acceptable salt thereof, optionally the hydrochloride salt thereof.

In various embodiments, the tumor has a drug resistant mutation.

In some embodiments, the tumor has an imatinib resistant mutation, sunitinib resistant mutation, regorafenib resistant mutation, ripretinib resistant mutation, avapritinib resistant mutation, or any combination thereof.

In some embodiments, the tumor has a KIT 8 exon mutation, a KIT exon 9 mutation, a KIT exon 10 mutation, a KIT exon 11 mutation, a KIT exon 13 mutation, a KIT exon 14 mutation, a KIT exon 17 mutation, a KIT exon 18 mutation, a PDGFRA exon 12 mutation, a PDGFRA exon 13 mutation, a PDGFRA exon 14 mutation, a PDGFRA exon 15 mutation, or a PDGFRA exon 18 mutation, wherein optionally the mutation is a missense mutation, insertion mutation, a deletion mutation, or a combination thereof.

In some embodiments, the mutation is a primary mutation.

In various embodiments, the tumor has:
- a KIT exon 9 mutation selected from the group consisting of p.A502_Y503dup, p.Y503_F504insAY, and p.S464L;
- a KIT exon 11 mutation selected from the group consisting of deletions involving codons 557/558, deletions upstream of codons 557/558, deletions downstream of codons 557/558, and deletions affecting codons 557/558;
- as a KIT exon 13 mutation selected from K642E, V65A, and T670I;
- a KIT exon 17 mutation selected from mutations involving codons 809, 816, 820, 822, or 823;
- a KIT exon 18 mutation selected from mutations involving codon 829,
- a PDGFRA exon 12 mutation selected from the group consisting of p.W559_R560del and p.581S;
- a PDGFRA exon 18 mutation selected from the group consisting of p.D842_M84del, p.D842_I843delinsV, and p.D842V.

In some embodiments, the tumor has one or more PDGFRA mutations selected from the group consisting of V598F, G605A, G652E, P653Q, V658A, N659K/T, T674R/I, E675G, Y676C, G680R/E, D842V, and 1843_D846del.

In some further embodiments, the tumor has the PDGFRA mutation D842V.

In some embodiments, the tumor has the PDGFRA mutation G680R.

In some embodiments, the tumor has the PDGFRA mutation G652E.

In some embodiments, the tumor has the PDGFRA mutation V658A.

In various embodiments, the tumor has one or more KIT mutations selected from the group consisting of S476I, F522C, K642E, V654A, N655K, T670E/I, D816A/E/G/H/N/V, D820A/E/G/H/V/Y, N822I/K/Y, Y823C/D, and A829P.

In some embodiments, the tumor has the KIT mutation T670I.

In some embodiments, the tumor has the KIT mutation V654A.

In some embodiments, the tumor has the KIT mutation D816A.

In some embodiments, the tumor has the KIT mutation D820A.

In some embodiments, the tumor has the KIT mutation N822K.

In some embodiments, the tumor has the KIT mutation A829P.

In various embodiments, the tumor has at least one primary and at least one secondary mutation.

In some embodiments, the tumor has a primary KIT exon 9 mutation and a secondary KIT exon 13 mutation, a secondary KIT exon 14 mutation, a secondary KIT exon 17 mutation or a secondary KIT exon 18 mutation.

In some embodiments, the tumor has a primary KIT exon 11 mutation and a secondary KIT exon 13 mutation, a secondary KIT exon 14 mutation, a secondary KIT exon 17 mutation or a secondary KIT exon 18 mutation.

In some embodiments, the tumor has a primary KIT exon 13 mutation and a, a secondary KIT exon 14 mutation, a secondary KIT exon 17 mutation or a secondary KIT exon 18 mutation.

In some embodiments, the tumor has a primary KIT exon 17 mutation and a secondary KIT exon 13 mutation, a secondary KIT exon 14 mutation, or a secondary KIT exon 18 mutation.

In some embodiments, the tumor has a primary PDGFRA exon 12 mutation and, a secondary PDGFRA exon 13 mutation, a secondary PDGFRA exon 14 mutation, a secondary PDGFRA exon 15 mutation, or a secondary PDGFRA exon 18 mutation.

In some embodiments, the tumor has a primary PDGFRA exon 18 mutation and a secondary PDGFRA exon 12 mutation, a secondary PDGFRA exon 13 mutation, a secondary PDGFRA exon 14 mutation, and a secondary PDGFRA exon 15 mutation.

In various embodiments, the tumor has compound mutations selected from double-in-cis mutations or triple-in-cis mutations.

In some embodiments, the tumor has a primary KIT exon 9 mutation and at least one acquired mutation selected from mutations in KIT exon 13, KIT exon 14, KIT exon 17, and KIT exon 18.

In some embodiments, the tumor has a primary KIT exon 9 mutation and at least one acquired mutation in KIT exon 13 or KIT exon 14 and at least one acquired mutation in KIT exon 17 or KIT exon 18.

In some embodiments, the tumor has a primary KIT exon 11 mutation and at least one acquired mutation selected from mutations in KIT exon 13, KIT exon 14, KIT exon 17, and KIT exon 18.

In some embodiments, the tumor has a primary KIT exon 11 mutation and at least one acquired mutation in KIT exon 13 or KIT exon 14 and at least one acquired mutation in KIT exon 17 or KIT exon 18.

In various embodiments, the patient was previously treated with one or more kinase inhibitors selected from imatinib, sunitinib, regorafenib, lapatinib, gefinitib, erlotinib, vatlanib, crenolanib, ripretinib, nintedanib, ponartinib, nilotinib, axitinib, cabozantinib, pazopanib, pexidartinib, bezuclastinib, dasatinib, sorafenib, and avapritinib, or a pharmaceutically acceptable salt thereof.

In some embodiments, the patient was previously treated with imatinib, sunitinib, regorafenib, ripretinib and/or avapritinib.

In some embodiments, the patient was previously treated with imatinib.

In some embodiments, the patient was previously treated with sunitinib.

In some embodiments, the patient was previous treated with regorafenib.

In some embodiments, the patient was previously treated with ripretinib.

In some embodiments, the patient was previously treated with avapritinib.

In various embodiments, the tumor is an advanced gastrointestinal stromal tumorthat has progressed from, or the patient was intolerant to, administration of imatinib, administration of sunitinib, administration of regorafenib, administration of ripretinib and/or administration of avapritinib, or wherein the patient has a documented intolerance to one or more of imatinib, sunitinib, regorafenib, ripretinib, and avapritinib.

In various embodiments, the tumor is an advanced gastrointestinal stromal tumorthat has progressed from, or the patient was intolerant to, a first line administration of imatinib, a second line administration of sunitinib, and an optional third line administration of regorafenib or ripretinib, or wherein the patient has a documented intolerance to one or more of imatinib, sunitinib, regorafenib and/or ripretinib.

In various embodiments, the tumor has a KIT 8 exon mutation, a KIT exon 9 mutation, a KIT exon 10 mutation, a KIT exon 11 mutation, a KIT exon 13 mutation, a KIT exon 14 mutation, a KIT exon 17 mutation, or a KIT exon 18 mutation, preferably a KIT exon 9 mutation selected from the group consisting of p.A502_Y503dup, p.Y503_F504insAY, and p.S464L;
a KIT exon 11 mutation selected from the group consisting of deletions involving codons 557/558, deletions upstream of codons 557/558, deletions downstream of codons 557/558, and deletions affecting codons 557/558;
a KIT exon 13 mutation selected from K642E, V65A, and T670I; or
a KIT exon 17 mutation selected from mutations involving codons 816, 820 or 823.

In some embodiments, the tumor is an advanced gastrointestinal stromal tumor that has progressed from, or the patient was intolerant to, administration of avapritinib, or wherein the patient has a documented intolerance to avapritinib.

In various embodiments, the tumor has a PDGFRA exon 12 mutation, a PDGFRA exon 13 mutation, a PDGFRA exon 14 mutation, a PDGFRA exon 15 mutation, or a PDGFRA exon 18 mutation, preferably a PDGFRA exon 12 mutation selected from the group consisting of p.W559_R560del and p.P581S; or a PDGFRA exon 18 mutation selected from the group consisting of p.D842_M84del, p.D842_I843delinsV, and p.D842V.

In some embodiments, the patient is a human patient.

In various embodiments, the compound of formula (I) is comprised in a pharmaceutical composition together with a pharmaceutically acceptable carrier.

In some embodiments, the compound of formula (I) is administered orally, parenterally, buccally, vaginally, rectally, by inhalation, by insufflation, sublingually, intramuscularly, subcutaneously, topically, intranasally, intraperitoneally, intrathoracially, intravenously, epidurally, intrathecally, intracerebroventricularly, or by injection into a joint.

In another aspect, the present invention relates to a compound of formula (I) or a pharmaceutically acceptable salt thereof for use in the treatment of a gastrointestinal stromal tumor (GIST) in a patient, 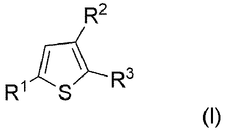 wherein
R¹ is selected from aryl and heterocyclyl, provided R¹ is not thienyl, and wherein R¹ can be optionally substituted on one or more carbon atoms by one or more R⁶; and further wherein if said heterocyclyl contains an -NH- moiety, the nitrogen of said moiety can be optionally substituted by a group selected from R7;
R² is -NHC(=O)NH₂;
R³ is -C(=O)NR⁴R⁵;
R⁴ is cycloalkyl or a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷;
R⁵ is H or taken together with R⁴ and the nitrogen atom to which it is attached form a heterocyclic ring, wherein said heterocyclic ring is selected from the group consisting of 4-, 5-, 6-, and 7-membered heterocyclic rings, wherein said heterocyclic ring can be optionally substituted on one or more carbon atoms by one or more R⁶, and wherein if said heterocyclyl contains a -NH- moiety, the nitrogen of said moiety can be optionally substituted by a group selected from R⁷;
each R⁶ and R⁷ is independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C1-₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHSO₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁸R⁹, mercapto, -S(C₁₋₆ alkyl), -SO(C₁₋₆ alkyl), -S0₂(C₁₋₆ alkyl), -SO₂-NR⁸R⁹, wherein R⁶ and R⁷ independently of each other can be optionally substituted on one or more carbon atoms by one or more R¹⁰ and on nitrogen of any moiety that contains an NH or NH₂ by R¹¹;
R⁸ and R⁹ are each independently selected from halo, nitro, cyano, isocyano, C₁₋₆ alkyl, C₂₋₆ alkenyl, C₂₋₆ alkynyl, aryl, cycloalkyl, heterocyclyl, hydroxy, oxo(=O), -O(C1-₆ alkyl), -Oaryl, -OC(=O)alkyl, -CHO, -C(=O)NR¹²R¹³, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR¹²R¹³, mercapto, -S(C₁₋₆ alkyl), -SO(C₁₋₆ alkyl), -S0₂(C₁₋₆ alkyl), -SO₂-NR¹²R¹³;
R¹⁰ and R¹¹ are independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C1-₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHS0₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁷R⁸, mercapto, -S(C₁₋₆ alkyl), -SO(C₁₋₆ alkyl), -S0₂(C₁₋₆ alkyl), -SO₂-NR⁸R⁹,
R¹² and R¹³ are independently selected from H, C₁₋₆ alkyl, cycloalkyl, aryl, heterocyclyl; alternatively R¹² and R¹³ together with the nitrogen atom to which they are attached form a heterocyclic ring.

These and other aspects, embodiments, features, and advantages of the invention become apparent to the person skilled in the art in the following detailed description, claims and figures. Each feature from one aspect of the invention can be used in any other aspect of the invention. Furthermore, the examples contained herein are intended to describe and illustrate the invention, but do not restrict it. In particular, the invention is not limited to these examples.

### Brief description of the drawings

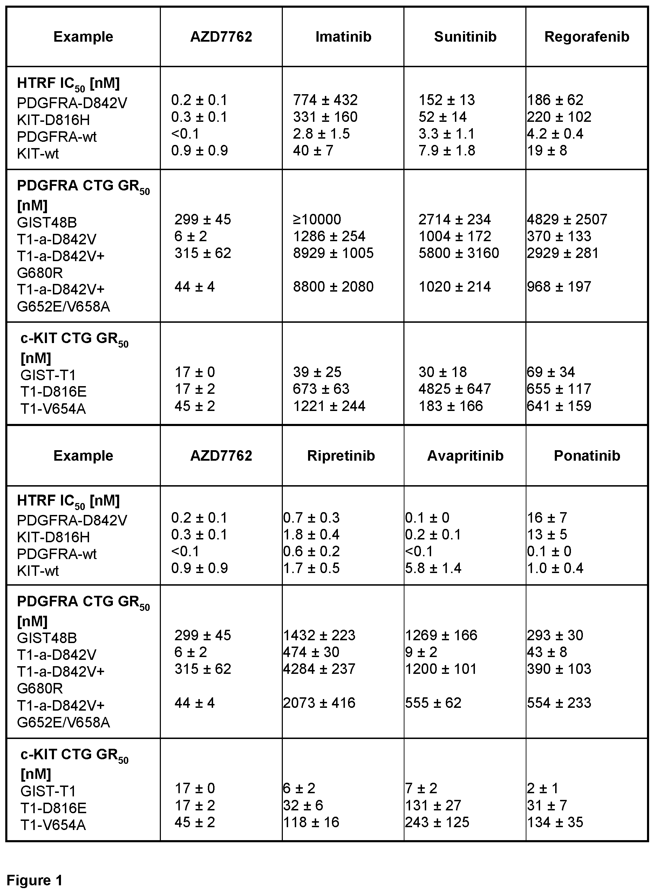

**Figure 1** shows results obtained with a compound according to chemical formula (I), (*S*)-5-(3-fluorophenyl)-N-(piperidin-3-yl)-3-ureidothiophene-2-carboxamide and other known RTK inhibitors, in mutated, drug-resistant KIT/PDGFRA GIST models. Said compound shows sub-nanomolar biochemical activity in biochemical inhibition assays.

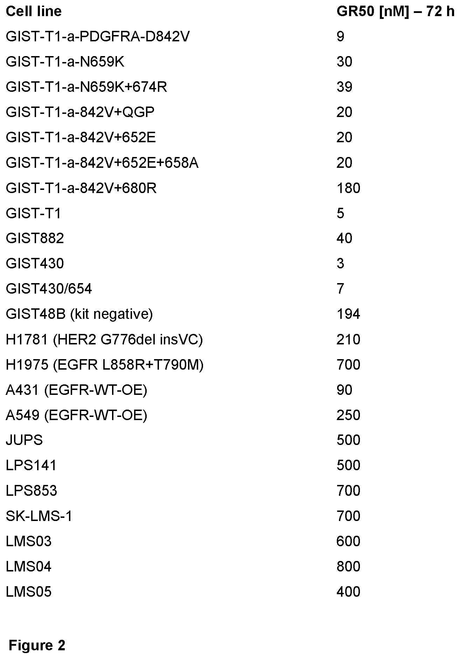

**Figure 2** shows cellular GR50 (concentration of a drug that reduces cell growth rate by half) in KIT/PDGFRA-mutated cell lines. The compound according to the invention, (*S*)-5-(3-fluorophenyl)-N-(piperidin-3-yl)-3-ureidothiophene-2-carboxamide, shows GR50 values in the range of 1-30 nM and GR50 > 180 nM in other tyrosine kinase (HER2/EGFR)-driven cell lines.

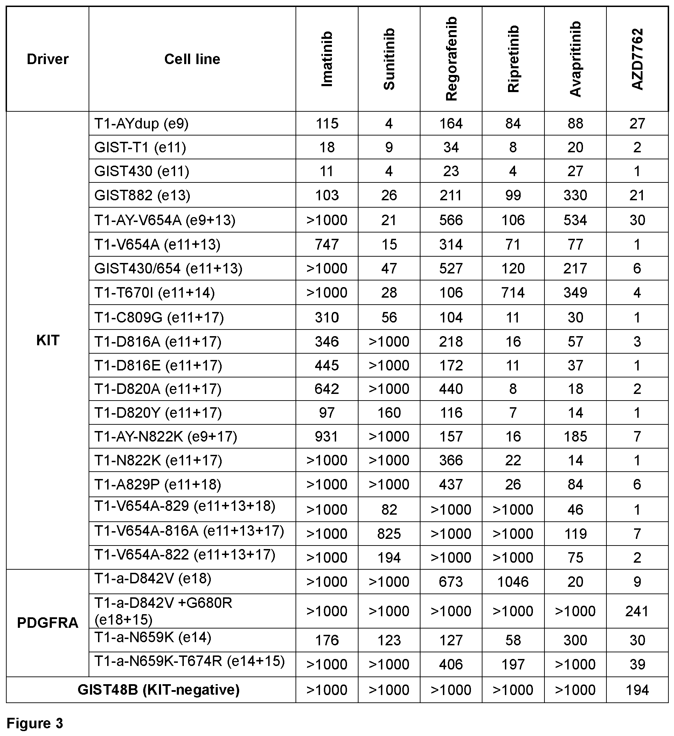

**Figure 3** shows a comparison between (*S*)-5-(3-fluorophenyl)-N-(piperidin-3-yl)-3-ureidothiophene-2-carboxamide, a compound according to the invention, and state-of-the-art TKI drugs approved for treatment of GIST in terms of GR50 values in a broad panel of KIT- and PDGFRA-mutated GIST cell lines, respectively, including newer types of multi-resistant, "triple-in-cis"-mutated cell lines (exon 11 + exon 13/14 + 17/18).

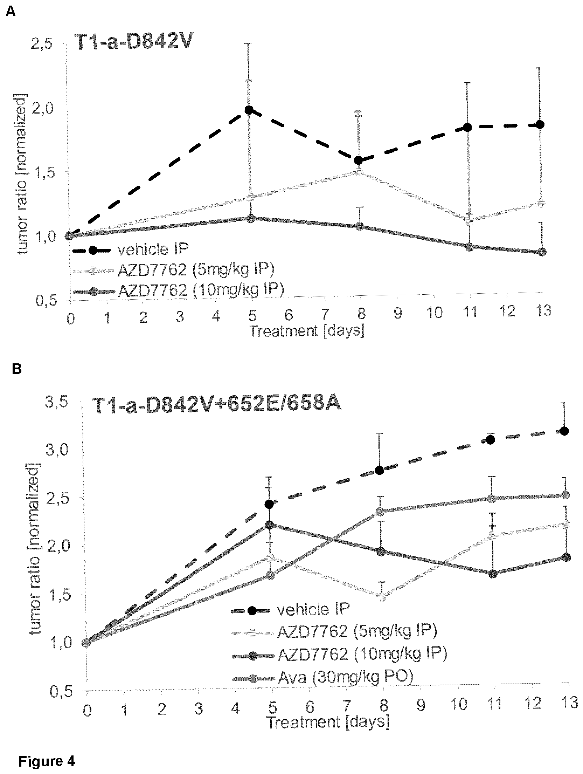

**Figure 4** shows tumor growth inhibition in xenograft models of PDGFRA-mutated, avapritinib-sensitive (A; comprising the PDGFRA D842V mutation) and -resistant (B; additionally comprising the G652E/V658A mutations) cell lines over 13 days with daily intraperitoneal administration of 5 or 10 mg/kg AZD7762 ((*S*)-5-(3-fluorophenyl)-N-(piperidin-3-yl)-3-ureidothiophene-2-carboxamide) or Ava (avapritinip; administered by oral gavage) relative to a vehicle control.

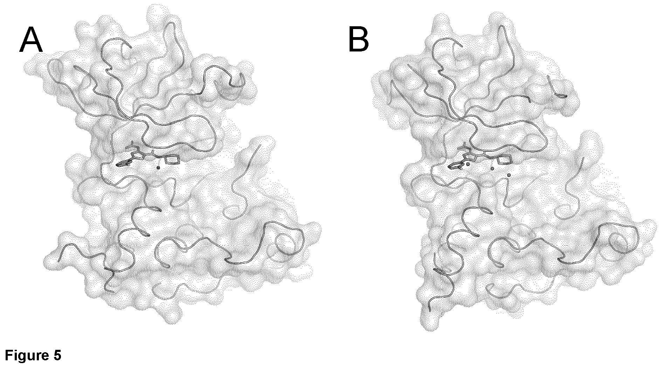

**Figure 5** depicts binding modes of a compound according to chemical formula (I), (S)-5-(3-fluorophenyl)-N-(piperidin-3-yl)-3-ureidothiophene-2-carboxamide, for KIT **(A)** and PDGFRA-T647I **(B),** respectively, as determined *via* X-ray crystallography.

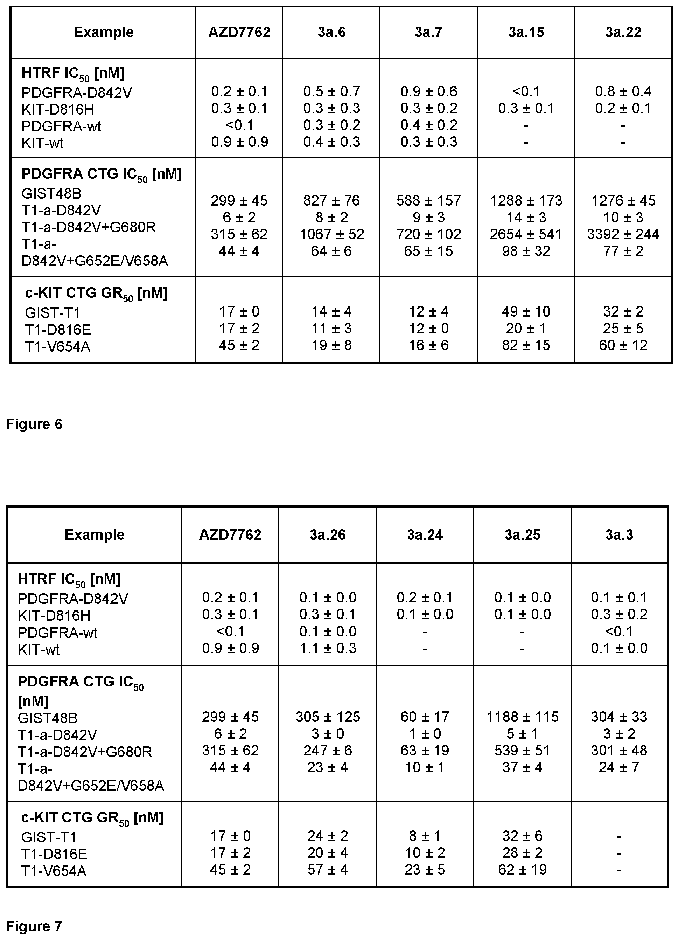

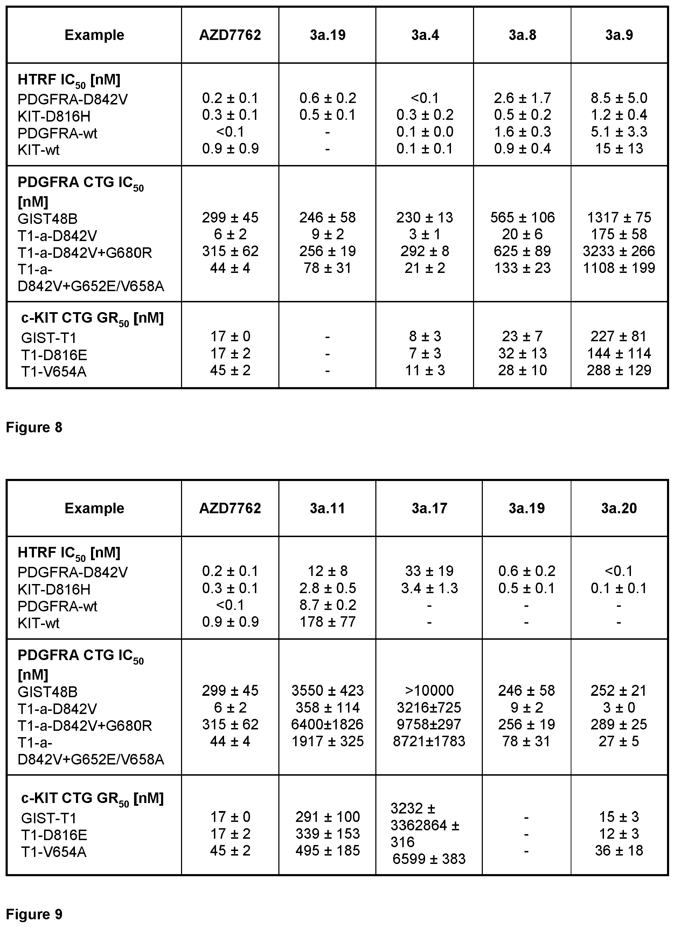

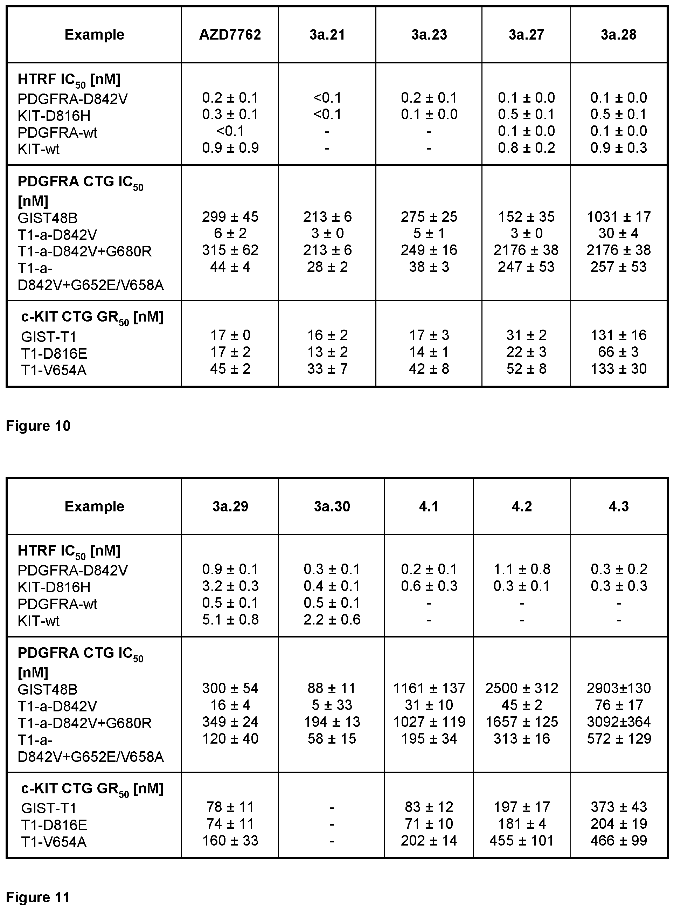

**Figures 6-11** show results obtained with various compounds according to chemical formula (I) in mutated, drug-resistant KIT/PDGFRA GIST models. Compounds are indicated by reference to preparation examples.

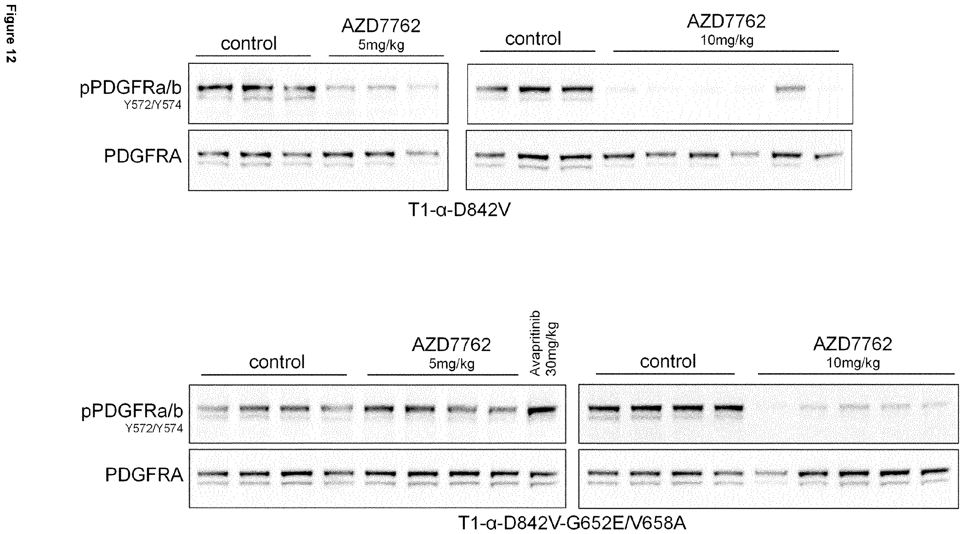

**Figure 12** shows changes in PDGFRA expression (PDGFRA) and phosphorylation (pPDGFRa/b) in tumor samples from xenograft models of PDGFRA-mutated, avapritinib-sensitive (T1-α-D842V) and -resistant (T1-α-D842V-G652E/658A) cell lines with or without intraperitoneal administration of 5 or 10 mg/kg AZD7762 ((*S*)-5-(3-fluorophenyl)-N-(piperidin-3-yl)-3-ureidothiophene-2-carboxamide), as determined by a Western Blot assay using antibodies against total PDGFRA and phosphorylated PDGFRA (Cell Signaling Technologies).

### Detailed description

Before describing in detail exemplary embodiments of the present invention, definitions which are important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±10 %, preferably ±5 %, more preferably ±2 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

It is to be understood that this invention is not limited to the particular methodology, protocols, reagents etc. described herein as these can vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As used herein the term "does not comprise" or "free of" means in the context that a composition of the present invention is free of a specific compound or group of compounds, which may be combined under a collective term, that the composition does not comprise said compound or group of compounds in an amount of more than 0.8 % by weight, based on the total weight of the composition. Furthermore, it is preferred that a composition according to the present invention does not comprise said compounds or group of compounds in an amount of more than 0.5 % by weight, preferably the composition does not comprise said compounds or group of compounds at all.

When referring to compositions and the weight percent of the therein comprised ingredients it is to be understood that according to the present invention the overall amount of ingredients does not exceed 100 % (± 1% due to rounding).

The definitions set forth in this section are intended to clarify terms used throughout this application. In this section, the definition applies to compounds of formula (I) and (II) and compounds of formula (Ia) unless otherwise stated. The term "herein" means the entire application.

Unless specified otherwise within this specification, the nomenclature used in this specification generally follows the examples and rules stated in Nomenclature of Organic Chemistry, Sections A, B, C, D, E, F, and H, Pergamon Press, Oxford, 1979, which is incorporated by references herein for its exemplary chemical structure names and rules on naming chemical structures.

The term "Cₘ₋ₙ" or "Cₘ₋ₙ group" used alone or as a prefix, refers to any group having m to n carbon atoms.

As used in this application, the term "optionally substituted" means that substitution is optional and therefore it is possible for the designated atom to be unsubstituted. In the event a substitution is desired then such substitution means that any number of hydrogens on the designated atom is replaced with a selection from the indicated group, provided that the normal valency of the designated atom is not exceeded, and that the substitution results in a stable compound. For example, when a substituent is oxo (i.e., =O), then 2 hydrogens on the atom are replaced. When a group is indicated to be "optionally substituted" or "substituted" unless otherwise expressly stated examples of suitable substituents include the following: halo, nitro, amino, cyano, trifluoromethyl, methyl, ethyl, alkyl, alkenyl, alkynyl, haloalkyl, alkoxy, hydroxy, alkylhydroxy, carbonyl, oxo, -CH(OH)CH₃, -CH₂NH-alkyl-OH, alkyl-(OH)CH₃, -Oalkyl, -OCOalkyl, -NHCHO, -N-(alkyl)-CHO, -NH-CO-amino, -N-(alkyl)-CO- amino, -NH-COalkyl, -N-(alkyl)-COalkyl, -carboxy, - amidino, -CO-amino, -CO-alkyl, - CO₂alkyl, mercapto, -Salkyl, -SO(alkyl), -SO₂(alkyl), -SO₂-amino, - alkylsulfonylamino, phenyl, cycloalkyl, heterocyclic and heteroaryl, -alkly-NH-cycloalkyl, -alkyl-NH-optionally substituted heterocyclyl, -alkyl-NH-alkyl-OH, -C(=O)OC(CH₃)₃, -N(CH₃)₂, -allcyl-NH-alkyl-optionally substituted heterocyclyl, alkyl-aryl, alkyl-polycyclyl, alkyl-amino, alkyl-hydroxy, -CH₂NH-alkyl-heterocyclyl, -CH₂NHCH₂CH(CH₃)₂. If the group to be substituted is a ring, the optional substituents could also be selected from: vicinal -O(alkyl)O-, vicinal -OC(haloalkyl)O-, vicinal -CH₂O(alkyl)O-, vicinal - S(alkyl)S- and - O(alkyl)S-. Each of these substituents can, themselves, be further substituted. Suitable examples of such further substitution include any of the foregoing suitable substituents.

The term "hydrocarbon" used alone or as a suffix or prefix, refers to any structure comprising only carbon and hydrogen atoms up to 14 carbon atoms.

The term "hydrocarbon radical" or "hydrocarbyl" used alone or as a suffix or prefix, refers to any structure as a result of removing one or more hydrogens from a hydrocarbon.

The term "alkyl" used alone or as a suffix or prefix, refers to monovalent straight or branched chain hydrocarbon radicals comprising 1 to about 12 carbon atoms that are saturated.

The term "alkenyl" used alone or as suffix or prefix, refers to a monovalent straight or branched chain hydrocarbon radical having at least one carbon-carbon double bond and comprising at least 2 up to about 12 carbon atoms.

The term "alkylene" used alone or as suffix or prefix, refers to divalent straight or branched chain hydrocarbon radicals comprising 1 to about 12 carbon atoms, which serves to links two structures together.

The term "alkynyl" used alone or as suffix or prefix, refers to a monovalent straight or branched chain hydrocarbon radical having at least one carbon-carbon triple bond and comprising at least 2 up to about 12 carbon atoms.

The term "cycloalkyl" used alone or as suffix or prefix, refers to a monovalent ring- containing hydrocarbon radical comprising at least 3 up to about 12 carbon atoms. When cycloalkyl contains more than one ring, the rings can be fused or unfused and include bicyclo radicals. Fused rings generally refer to at least two rings sharing two atoms therebetween.

The term "cycloalkenyl" used alone or as suffix or prefix, refers to a monovalent ring-containing hydrocarbon radical having at least one carbon-carbon double bond and comprising at least 3 up to about 12 carbon atoms. When cycloalkenyl contains more than one ring, the rings can be fused or unfused and include bicyclo radicals.

The term "aryl" used alone or as suffix or prefix, refers to a hydrocarbon radical having one or more polyunsaturated carbon rings having aromatic character, (*e.g.,* 4n + 2 delocalized electrons) and comprising 6 up to about 14 carbon atoms, wherein the radical is located on a carbon of the aromatic ring. Exemplary aryl includes phenyl, naphthyl, and indenyl.

The term "alkoxy" used alone or as a suffix or prefix, refers to radicals of the general formula -O-R, wherein -R is selected from a hydrocarbon radical. Exemplary alkoxy includes methoxy, ethoxy, propoxy, isopropoxy, butoxy, t-butoxy, isobutoxy, cyclopropylmethoxy, allyloxy, and propargyloxy.

The term "carbocyclyl" is intended to include both alicyclic and aromatic ring structures wherein the closed ring is made of carbon atoms. These can include fused or bridged polycyclic systems. Carbocyclyls can have from 3 to 10 carbon atoms in their ring structure, and often have 3, 4, 5, 6 and 7 carbon atoms in the ring structure. For example, "C₃₋₇ carbocyclyl" denotes such groups as cyclopropyl, cyclobutyl, cyclopentyl, cyclohexyl, cycloheptyl, cyclopentadiene or phenyl.

A "heterocyclyl" is a saturated, partially saturated or unsaturated, mono or bicyclic ring containing 4-12 atoms of which at least one atom is chosen from nitrogen, sulphur or oxygen, which can, unless otherwise specified, be carbon or nitrogen linked, wherein a -CH₂- group can optionally be replaced by a -C(O)- and a ring sulphur atom can be optionally oxidised to form the S-oxides. Heterocyclyl can contain more than one ring. When a heterocyclyl contains more than one ring, the rings can be fused or unfused. Fused rings generally refer to at least two rings sharing two atoms therebetween. Heterocyclyl can have aromatic character or can not have aromatic character. Examples of heterocyclyls include, but are not limited to, 1H-indazolyl, 2-pyrrolidonyl, 2H, 6H-1,5,2-dithiazinyl, 2H-pyrrolyl, 3H-indolyl, 4-piperidonyl, 4aH-carbazolyl, 4H-quinolizinyl, 6H-1,2,5-thiadiazinyl, acridinyl, azepanyl, azetidinyl, aziridinyl, azocinyl, benzimidazolyl, benzofiiranyl, benzofiiranyl, benzothiofiiranyl, benzothiophenyl, , benzodioxolyl, benzoxazolyl, benzthiophenyl, benzthiazolyl, benzotriazolyl, benzotetrazolyl, benzisoxazolyl, benzthiazole, benzisothiazolyl, benzimidazolyls, benzimidazalonyl, carbazolyl, 4aH-carbazolyl, b-carbolinyl, chromanyl, chromenyl, cinnolinyl, decahydroquinolinyl, 2H,6H-1,5,2-dithiazinyl, dioxolanyl, furyl, 2,3-dihydrofuranyl, 2,5-dihydrofuranyl, dihydrofuro[2,3-b]tefrahydrofuranyl, furanyl, furazanyl, homopiperidinyl, imidazolyl, imidazolidinyl, imidazolidinyl, imidazolinyl, imidazolyl, 1H-indazolyl, indolenyl, indolinyl, indolizinyl, indolyl, isobenzofuranyl, isochromanyl, isoindazolyl, isoindolinyl, isoindolyl, isoquinolinyl, isothiazolyl, isoxazolyl, morpholinyl, naphthyridinyl, octahydroisoquinolinyl, oxadiazolyl, 1,2,3-oxadiazolyl, 1,2,4- oxadiazolyl, 1,2,5-oxadiazolyl, 1,3,4-oxadiazolyl, oxazolidinyl, oxazolyl, oxiranyl, oxazolidinylperimidinyl, phenanthridinyl, phenanthrolinyl, phenarsazinyl, phenazinyl, phenothiazinyl, phenoxathiinyl, phenoxazinyl, phthalazinyl, piperazinyl, piperidinyl, pteridinyl, piperidonyl, 4-piperidonyl, purinyl, pyranyl, pyrrolidinyl, pyrrolinyl, pyrrolidinyl, pyrazinyl, pyrazolidinyl, pyrazolinyl, pyrazolyl, pyridazinyl, pyridooxazolyl, pyridoimidazolyl, pyridothiazolyl, pyridinyl, N-oxide-pyridinyl, pyridyl, pyrimidinyl, pyrrolidinyl, pyrrolinyl, pyrrolyl, pyridinyl, quinazolinyl, quinolinyl, 4H-quinolizinyl, quinoxalinyl, quinuclidinyl, carbolinyl, tetrahydrofuranyl, tetrahydroisoquinolinyl, thiophanyl, thiotetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, thiiranyl, friazinyl, 1,2,3-triazolyl, 1,2,4-triazolyl, 1,2,5-triazolyl, 1,3,4-triazolyl, and xanthenyl.

The terms "seven-membered" or "7-membered", "six-membered" or "6-membered", and "five-membered" or "5-membered", etc., used as prefix refer to groups having a ring that contains, respectively, seven, six, and five ring atoms.

The term "substituted" used as a suffix of a first structure, molecule or group, followed by one or more names of chemical groups refers to a second structure, molecule or group, which is a result of replacing one or more hydrogens of the first structure, molecule or group with the one or more named chemical groups. For example, a "phenyl substituted by nitro" refers to nitrophenyl.

The term "amine" or "amino" used alone or as a suffix or prefix, refers to radicals of the general formula - NRR', wherein R and R' are independently selected from hydrogen or a hydrocarbon radical.

The term "halo" includes fluorine, chlorine, bromine and iodine. "Halogenated" used as a prefix of a group, means one or more hydrogens on the group are replaced with one or more halogens.

As used herein, "RT" or "rt" means room temperature.

When any variable *(e.g.,* R¹, R⁴, etc.) occurs more than one time in any constituent or formula for a compound, its definition at each occurrence is independent of its definition at every other occurrence. Thus, for example, if a group is shown to be substituted with 0-3 R¹, then said group can optionally be substituted with 0, 1, 2 or 3 R¹ groups and R¹ at each occurrence is selected independently from the definition of R¹. Also, combinations of substituents and/or variables are permissible only if such combinations result in stable compounds.

A variety of compounds in the present invention can exist in particular geometric or stereoisomeric forms. The present invention takes into account all such compounds, including cis- and trans isomers, R- and S-enantiomers, diastereomers, (D)-isomers, (L)-isomers, the racemic mixtures thereof, and other mixtures thereof, as being covered within the scope of this invention. Additional asymmetric carbon atoms can be present in a substituent such as an alkyl group. All such isomers, as well as mixtures thereof, are intended to be included in this invention. The compounds herein described can have asymmetric centers. Compounds of the present invention containing an asymmetrically substituted atom can be isolated in optically active or racemic forms. It is well known in the art how to prepare optically active forms, such as by resolution of racemic forms or by synthesis from optically active starting materials. When required, separation of the racemic material can be achieved by methods known in the art. Many geometric isomers of olefins, C=N double bonds, and the like can also be present in the compounds described herein, and all such stable isomers are contemplated in the present invention. Cis and trans geometric isomers of the compounds of the present invention are described and can be isolated as a mixture of isomers or as separated isomeric forms. All chiral, diastereomeric, racemic forms and all geometric isomeric forms of a structure are intended, unless the specific stereochemistry or isomeric form is specifically indicated.

When a bond to a substituent is shown to cross a bond connecting two atoms in a ring, then such substituent can be bonded to any atom on the ring. When a substituent is listed without indicating the atom via which such substituent is bonded to the rest of the compound of a given formula, then such substituent can be bonded via any atom in such substituent. Combinations of substituents and/or variables are permissible only if such combinations result in stable compounds.

When a circle is shown within a ring structure, it indicates that the ring system is aromatic.

As used herein, the phrase "protecting group" means temporary substituents which protect a potentially reactive functional group from undesired chemical transformations. Examples of such protecting groups include esters of carboxylic acids, silyl ethers of alcohols, and acetals and ketals of aldehydes and ketones respectively. The field of protecting group chemistry has been reviewed (Greene, T.W.; Wuts, P.G.M. Protective Groups in Organic Synthesis, 3rd ed.; Wiley: New York, 1999).

As used herein, "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, inorganic or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids.

For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, maleic, tartaric, citric, ascorbic, palmitic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, the disclosure of which is hereby incorporated by reference.

The terms "stable compound" and "stable structure" are meant, in the context of the present invention, to indicate a compound that is sufficiently robust to survive isolation to a useful degree of purity from a reaction mixture, and formulation into an efficacious therapeutic agent.

In the context of the present invention, the term "individual," "patient," or "subject" are used interchangeably herein and include any animal, including mammals, including mice, rats, other rodents, rabbits, dogs, cats, swine, cattle, sheep, horses, or primates, and humans. The compounds described herein can be administered to a mammal, such as a human, but can also be administered to other mammals such as an animal in need of veterinary treatment, *e.g.,* domestic animals (*e.g.,* dogs, cats, and the like), farm animals (*e.g.,* cows, sheep, pigs, horses, and the like) and laboratory animals (*e.g.,* rats, mice, guinea pigs, and the like). The mammal treated in the methods described herein is desirably a mammal in which treatment of a disorder described herein is desired, such as a human.

As used herein, "treating" includes any effect, e.g., lessening, reducing, modulating, or eliminating, that results in the improvement of the condition, disease, disorder and the like or its symptoms.

In the context of the present invention, the term "therapeutically effective amount" includes the amount of the subject compound that will elicit the biological or medical response of a tissue, system, animal or human that is being sought by the researcher, veterinarian, medical doctor or other clinician. A compound described herein, *e.g.,* a compound according to formula (I) as defined herein, is administered in therapeutically effective amounts to treat a condition described herein, *e.g.,* gastrointestinal stromal tumors. Alternatively, a therapeutically effective amount of a compound is the quantity required to achieve a desired therapeutic and/or prophylactic effect, such as an amount which results in the prevention of or a decrease in the symptoms associated with the condition.

The present invention provides for a method of treating a patient suffering from a gastrointestinal stromal tumor, comprising administering to said patient a pharmaceutically effective amount of a compound of formula (I) or a pharmaceutically acceptable salt thereof, 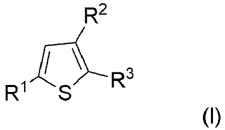 wherein
R¹ is selected from aryl and heterocyclyl, provided R¹ is not thienyl, and wherein R¹ can be optionally substituted on one or more carbon atoms by one or more R⁶; and further wherein if said heterocyclyl contains an -NH- moiety, the nitrogen of said moiety can be optionally substituted by a group selected from R7;
R² is -NHC(=O)NH₂;
R³ is -C(=O)NR⁴R⁵;
R⁴ is cycloalkyl or a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷;
R⁵ is H or taken together with R⁴ and the nitrogen atom to which it is attached form a heterocyclic ring, wherein said heterocyclic ring is selected from the group consisting of 4-, 5-, 6-, and 7-membered heterocyclic rings, wherein said heterocyclic ring can be optionally substituted on one or more carbon atoms by one or more R⁶, and wherein if said heterocyclyl contains a -NH- moiety, the nitrogen of said moiety can be optionally substituted by a group selected from R⁷;
each R⁶ and R⁷ is independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), - NHC(=O)OH, -N(C₁₋₆ alkyl)C(=O)(C₁₋₆ alkyl), -NHS0₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁸R⁹, mercapto, -S(C₁₋₆ alkyl), -SO(C₁₋₆ alkyl), -S0₂(C₁₋₆ alkyl), -SO₂-NR⁸R⁹, wherein R⁶ and R⁷ independently of each other can be optionally substituted on one or more carbon atoms by one or more R¹⁰ and on nitrogen of any moiety that contains an NH or NH₂ by R¹¹;
R⁸ and R⁹ are each independently selected from halo, nitro, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, - OC(=O)alkyl, -CHO, -C(=O)NR¹²R¹³, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR¹²R¹³, mercapto, -S(C₁₋₆ alkyl), -SO(C₁₋₆ alkyl), -S0₂(C₁₋₆ alkyl), -SO₂-NR¹²R¹³;
R¹⁰ and R¹¹ are independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), - NHC(=O)OH, -N(C₁₋₆ alkyl)C(=O)(C₁₋₆ alkyl), -NHS0₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁷R⁸, mercapto, -S(C₁₋₆ alkyl), -SO(C₁₋₆ alkyl), -S0₂(C₁₋₆ alkyl), -SO₂-NR⁸R⁹, R¹² and R¹³ are independently selected from H, C₁₋₆ alkyl, cycloalkyl, aryl, heterocyclyl; alternatively R¹² and R¹³ together with the nitrogen atom to which they are attached form a heterocyclic ring.

As surprisingly found by the inventors, the compounds of formula (I) are highly effective in biochemical, cellular and *in vivo* models of GIST **(****Figs. 1** **and** **6****-11).** Compounds of formula (I) act as ATP-competitive inhibitors of the protein kinases KIT and PDGFRA, by binding and thereby inhibiting them **(****Fig. 5****).** Particularly surprising is the extremely high efficacy the compounds of formula (I) have against multi-resistant KIT-triple mutations present on the same allele ("triple-in-cis"). The average growth inhibitory concentration (GR50) for almost all KIT mutations is in the low nanomolar range (except for PDGFRA-G680R solvent front mutation; triple-in-cis exon 11 T670/N822 subline) compared to cancer cell lines that do not possess a driving mutation in KIT or PFGFRA, which for other cell lines were found to be in the range of 200 to 800 nM **(****Fig. 3****).** Thus, the compounds of formula (I) can be used, alone or in combination with other active agents, in the treatment of drug resistant GIST (mutated KIT or mutated PDGFRA).

In the context of the present invention, the terms "KIT", "CD117" and "mast/stem cell growth factor receptor (SCFR)" can be used interchangeably. The receptor tyrosine kinase protein known as tyrosine-protein kinase KIT is encoded by the proto-oncogene c-KIT. The human KIT amino acid sequence is, for example, set forth in Uniprot database entry P10721.

The term "PDGFRA", as used herein, relates to platelet-derived growth factor receptor alpha. The human PDGFRA amino acid sequence is, for example, set forth in Uniprot database entry P16234.

In particular embodiments, the methods and uses of the present invention, as herein described and defined, induce cell death, apoptosis or prolonged cell stasis of GIST cells and/or induce the eradication of tumor cells to the limit of detection and/or reduce the volume of the tumor and/or inhibit the new growth of the tumor.

In the context of the present invention, the term "gastrointestinal stromal tumor (GIST)" as used herein refers to mesenchymal tumors located in the gastrointestinal tract (such as the stomach, the small intestine, and the large intestine) or in surrounding organs or tissues (such as the appendix, ampulla vater, rectum, omentum, anus, or the esophagus).

In various embodiments, in the above formula (I), R¹ is selected from aryl optionally substituted on one or more carbon atoms by one or more R⁶ In some such embodiments, R¹ is phenyl, optionally substituted on one or more carbon atoms by one or more R⁶, wherein each R⁶ is independently selected from halo, preferably fluoro; C₁₋₆ alkyl, preferably -CH₃; -O(C₁₋₆ alkyl), preferably -OCH₃; nitro; aryl, preferably phenyl; heterocyclyl, preferably imidazolyl, pyrazolyl, pyridinyl, or morpholino, wherein each R⁶ can be independently substituted on one or more carbon atoms by one or more R¹⁰ and on nitrogen of any moiety that contains an NH or NH₂ by R¹¹. In various embodiments, R¹ can be 3-fluorophenyl.

In some embodiments, R¹ is phenyl optionally substituted on one or more carbons atoms by one or more R⁶, wherein (each) R⁶ is preferably halo, more preferably fluoro. In some such embodiments, R¹ can be selected from 3-bromo phenyl; 3,4-difluoro phenyl; 2,4-difluoro phenyl; 2-fluoro phenyl; 3,5-difluoro phenyl; and 3-iodo phenyl. In some preferred embodiments, R¹ is 3-fluoro phenyl.

In various embodiments, R⁵ is H.

In some embodiments, R⁴ is a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷

In various embodiments, R⁵ is H and R⁴ is a cycloalkyl or a 5, 6, or 7-membered heterocyclyl ring containing at least one nitrogen atom, wherein R⁴ can be optionally substituted on one or more carbon atoms by one or more R⁶, wherein R⁶ is preferably selected from halo, preferably fluoro, and C₁₋₆ alkyl, preferably methyl and ethyl.

In various such embodiments, R⁴ is a 6-membered cycloalkyl or heterocyclyl ring containing at least one nitrogen atom, preferably selected from the group consisting of piperidinyl, such as 3-piperidinyl or 4-piperidinyl; pyridinyl, such as 4-pyridinyl or 2-pyridinyl; pyrazinyl, such as pyrazin-2-yl; and cyclohexyl. In some such embodiments, R⁴ is not substituted. In some such other embodiments, R⁴ is substituted on one or more carbon atoms by one or more R⁶, wherein R⁶ is preferably selected from halo, preferably fluoro, and C₁₋₆ alkyl, preferably methyl and ethyl.

In various embodiments, R⁴ is selected from pyridinyl, such as 2-pyridinyl, and piperidinyl, such as 3-piperidinyl, and substituted on one or more carbon atoms by one or more R⁶, wherein R⁶ is preferably selected from halo, preferably fluoro, and C₁₋₆ alkyl, preferably methyl and ethyl, more preferably selected from fluoro and methyl.

In various other embodiments, R⁵ is H and R⁴ is a 7-membered heterocyclyl ring containing at least one nitrogen atom, preferably selected from the group consisting of azepanyl, such as azepan-3-yl; and 2,6-diazaspiro[3.3]heptane-2-yl, optionally substituted on one or more carbon atoms by one or more R⁶. In some of these embodiments, R⁴ is not substituted.

In various other embodiments, R⁵ is H and R⁴ is a 5-membered heterocyclyl ring containing at least one nitrogen atom, preferably selected from the group consisting of pyrrolidine, such as 3-pyrrolidinyl, optionally substituted on one or more carbon atoms by one or more R⁶. In some of these embodiments, R⁴ is not substituted.

In some further embodiments, R⁵ is H and R¹ is selected from aryl optionally substituted on one or more carbon atoms by one or more R⁶

In some such embodiments, R¹ is phenyl, optionally substituted on one or more carbon atoms by one or more R⁶, wherein each R⁶ is independently selected from halo, preferably fluoro; C₁₋₆ alkyl, preferably -CH₃; -O(C₁₋₆ alkyl), preferably -OCH₃; nitro; aryl, preferably phenyl; heterocyclyl, preferably imidazolyl, pyrazolyl, pyridinyl, or morpholino, wherein each R⁶ can be independently substituted on one or more carbon atoms by one or more R¹⁰ and on nitrogen of any moiety that contains an NH or NH₂ by R¹¹. In various embodiments, R¹ can be 3-fluorophenyl.

In some such embodiments, R¹ is phenyl and R⁶ is preferably halo, more preferably fluoro, bromo or iodo. In some such embodiments, R¹ is selected from 3-bromo phenyl; 3,4-difluoro phenyl; 2,4-difluoro phenyl; 2-fluoro phenyl; 3,5-difluoro phenyl; and 3-iodo phenyl.

In some other embodiments, wherein R⁵ is H, R¹ is phenyl and R⁶ is nitro, for instance R¹ can be 2-nitrophenyl.

In some other such embodiments, R¹ is phenyl and each R⁶ is independently selected from halo and -O(C₁₋₆ alkyl), for example methoxy or ethoxy, more preferably methoxy. In some such embodiments, R¹ can be 5-fluoro-2-methoxy phenyl.

In various other embodiments, wherein R⁵ is H, R¹ is aryl, preferably phenyl, optionally substituted on one or more carbon atoms by one or more R⁶, wherein each R⁶ is independently selected from halo; C₁₋₆ alkyl, preferably -CH₃; -O(C₁₋₆ alkyl), preferably -OCH₃; nitro; aryl, preferably phenyl; heterocyclyl, preferably imidazolyl, pyrazolyl, pyridinyl, or morpholino, and wherein each R⁶ can be independently substituted on one or more carbon atoms by one or more R¹⁰ and on nitrogen of any moiety that contains an NH or NH₂ by R¹¹. In some such embodiments, R⁶ on the phenyl moiety can be aryl or heterocyclyl, for example in the meta position, both of which can be optionally substituted on one or more carbon atoms by one or more R¹⁰ and on nitrogen of any moiety that contains an NH or NH₂ by R¹¹.

In some embodiments, wherein R⁵ is H, R¹ is aryl, preferably phenyl, and R⁶ is phenyl optionally substituted by one or more R¹⁰, wherein R¹⁰ is selected from halo, preferably fluoro, and -NR⁸R⁹, wherein preferably both R⁸ and R⁹ are alkyl, more preferably CH₃. In some such embodiments, the R¹⁰ is in the para position.

In some other such embodiments, wherein R⁵ is H, R¹ is aryl, preferably phenyl, and R⁶ is heterocyclyl, for example pyrazolyl, such as 4-pyrazolyl, pyridinyl, such as 4-pyridinyl, or imidazolyl, such as 1-imidazolyl, both optionally substituted on one or more carbon atoms by one or more R¹⁰ and on NH nitrogen, if present, by R¹¹. In some such embodiments, one pyrazolyl nitrogen is substituted by R¹¹, with R¹¹ being C₁₋₆ alkyl, preferably -CH₃.

In some other embodiments, wherein R⁵ is H, R¹ is benzofuranyl, such as 2-benzofuranyl, optionally substituted on one or more carbon atoms by one or more R⁶ In some such embodiments, R¹ is not substituted.

In some other embodiments, wherein R⁵ is H, R¹ is pyridinyl, such as 3-pyridinyl, optionally substituted on one or more carbon atoms by one or more R⁶ In some such embodiments, R¹ is not substituted.

In some other embodiments, wherein R⁵ is H, R¹ is pyrazolyl, such as 4-pyrazolyl, optionally substituted on one or more carbon atoms by one or more R⁶ and optionally substituted on any nitrogen by a group selected from R⁷. In some such embodiments, each R⁶ on the pyrazolyl is indepdently selected from C₁₋₆ alkyl, preferably methyl or ethyl, and/or the R⁷ on the pyrazolyl is aryl, more preferably phenyl. In some such embodiments, R⁴ is 3-piperidinyl, R¹ is 4-pyrazolyl substituted by C₁₋₆ alkyl on one of the carbon atoms, such as the carbon in the 3-position, preferably methyl, and/or substituted on one nitrogen by R⁷, with R⁷ being aryl, preferably phenyl.

In some embodiments, R⁴ is a 6-membered heterocyclyl ring, preferably 3-piperidinyl, optionally substituted on one or more carbon atoms by one or more R⁶, and wherein the nitrogen of the -NH- moiety can be optionally substituted by a group selected from R⁷

In various embodiments, R⁵ is H and R⁴ is a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷; and R¹ is selected from aryl optionally substituted on one or more carbon atoms by one or more R⁶ In such embodiments, R⁴ can be a 6-membered heterocyclyl ring containing at least one nitrogen atom, preferably 3-piperidinyl, optionally substituted on one or more carbon atoms by one or more R⁶, and wherein the nitrogen of the -NH- moiety can be optionally substituted by a group selected from R⁷. In some such embodiments, R⁴ is unsubstituted 3-piperidinyl.

In various embodiments, the compound is selected from the following compounds: 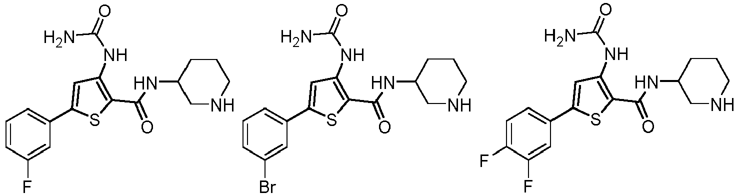 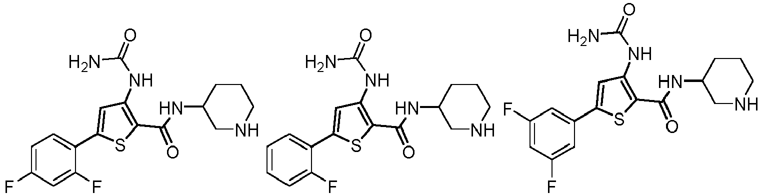 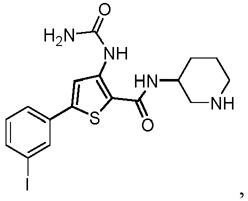 including pharmaceutically acceptable salts thereof.

In various embodiments, the compound is selected from the following compounds: 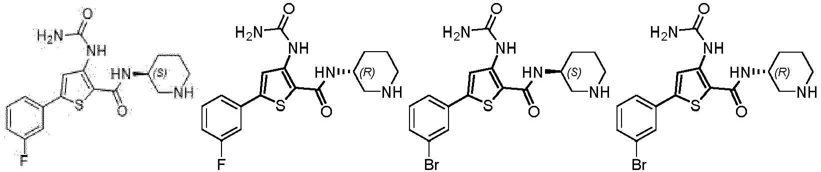 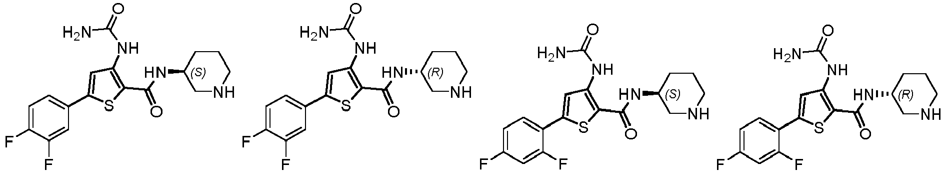 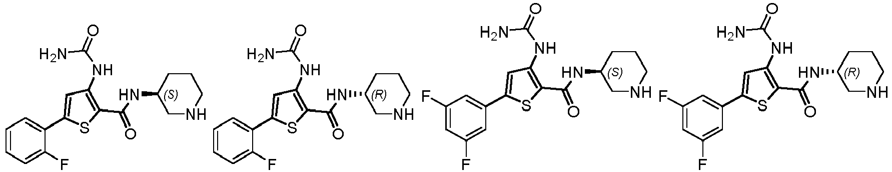 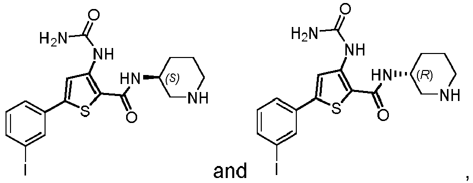 including pharmaceutically acceptable salts thereof.

In various embodiments, the compound is 5-(3-fluorophenyl)-N-(piperidin-3-yl)-3-ureidothiophene-2-carboxamide or a pharmaceutically acceptable salt thereof, preferably (S)-5-(3-fluorophenyl)-N-(piperidin-3-yl)-3-ureidothiophene-2-carboxamide or a pharmaceutically acceptable salt thereof, such as (S)-5-(3-fluorophenyl)-N-(piperidin-3-yl)-3-ureidothiophene-2-carboxamide hydrochloride.

In various embodiments, in which R⁵ is H and R⁴ is a 6-membered heterocyclyl ring containing at least one nitrogen atom, preferably 3-piperidinyl, optionally substituted on one or more carbon atoms by one or more R⁶, and wherein the nitrogen of the -NH- moiety can be optionally substituted by a group selected from R⁷, and R¹ is phenyl substituted on one or more carbon atoms by one or more R⁶, the R⁶ substituent on the phenyl moiety can be nitro.

In some embodiments, the compound is thus selected from: 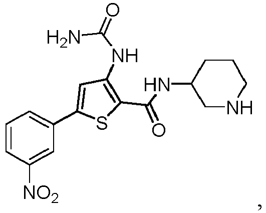 including pharmaceutically acceptable salts thereof.

In various embodiments, the compound is selected from the following compounds: 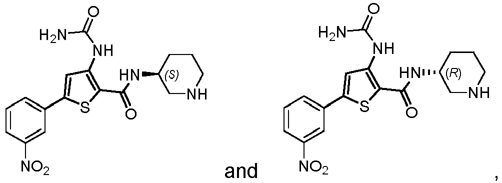 including pharmaceutically acceptable salts thereof.

In various embodiments, in which R⁵ is H and R⁴ is a 6-membered heterocyclyl ring containing at least one nitrogen atom, preferably 3-piperidinyl, optionally substituted on one or more carbon atoms by one or more R⁶, and wherein the nitrogen of the -NH- moiety can be optionally substituted by a group selected from R⁷, and R¹ is phenyl substituted on one or more carbon atoms by one or more R⁶, the phenyl moiety can be substituted by more than one R⁶ substituent, with at least being selected from halo and the other being selected from C₁₋₆ alkyl and -O(C₁₋₆ alkyl), for example methoxy. In some such embodiments, R¹ is 5-fluoro-2-methoxy phenyl. In some embodiments, the compound is therefore selected from: 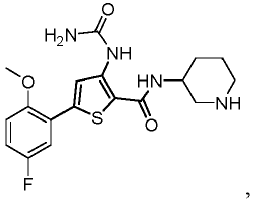 including pharmaceutically acceptable salts thereof.

In some embodiments, the compound is therefore selected from: 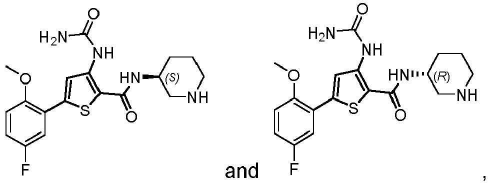 including pharmaceutically acceptable salts thereof.

In various embodiments, in which R⁵ is H and R⁴ is a 6-membered heterocyclyl ring, containing at least one nitrogen atom, preferably 3-piperidinyl, optionally substituted on one or more carbon atoms by one or more R⁶, and wherein the nitrogen of the -NH- moiety can be optionally substituted by a group selected from R⁷, and R¹ is phenyl, wherein R¹ is substituted on one or more carbon atoms by one or more R⁶, R⁶ on the phenyl moiety can be aryl or heterocyclyl, for example in the meta position, both of which can be optionally substituted on one or more carbon atoms by one or more R¹⁰ and on nitrogen of any moiety that contains an NH or NH₂ by R¹¹. In some embodiments, wherein R⁶ is aryl, such as phenyl, R⁶ is preferably substituted by one or more R¹⁰. In these embodiments, R¹⁰ can be selected from halo, preferably fluoro, and -NR⁸R⁹, wherein preferably both R⁸ and R⁹ are alkyl, more preferably CH₃. In these embodiments, the R¹⁰ substituent on the aryl, preferably phenyl, ring can be in the para position. In embodiments, wherein R⁶ is heterocyclyl, R⁶ can preferably be pyrazolyl, such as 4-pyrazolyl, and optionally such heterocyclyl can be substituted on one or more carbon atoms by one or more R¹⁰ and on nitrogen of any moiety that contains an NH or NH₂ by R¹¹. In some such embodiments, wherein R⁶ is pyrazolyl, the pyrazolyl nitrogen can be substituted by R¹¹. In such embodiments, R¹¹ can be C₁₋₆ alkyl, preferably -CH₃.

In various embodiments, the compound can thus be selected from the following compounds: 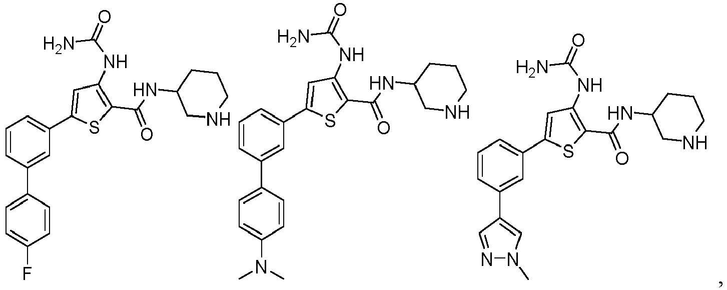 including pharmaceutically acceptable salts thereof.

In various embodiments, the compound can thus be selected from the following compounds: 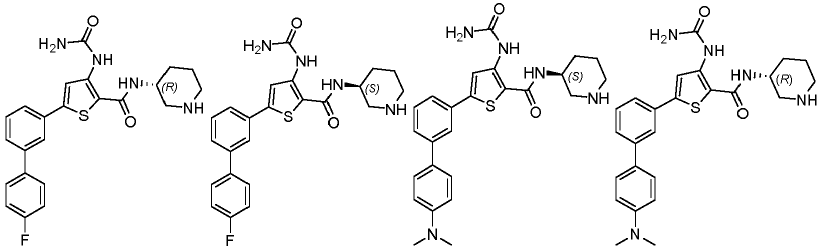 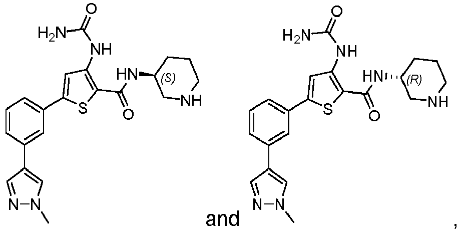 including pharmaceutically acceptable salts thereof.

In various embodiments, in which R⁵ is H and R⁴ is a 6-membered heterocyclyl ring containing at least one nitrogen atom, preferably 3-piperidinyl, optionally substituted on one or more carbon atoms by one or more R⁶, and wherein the nitrogen of the -NH- moiety can be optionally substituted by a group selected from R⁷, and R¹ is phenyl, wherein R¹ is substituted on one or more carbon atoms by one or more R⁶, R⁶ is heterocyclyl, preferably pyridinyl or morpholinyl, such as 4-pyridinyl or 4-morpholinyl.

In various embodiments, the compound can thus be selected from the following compounds: 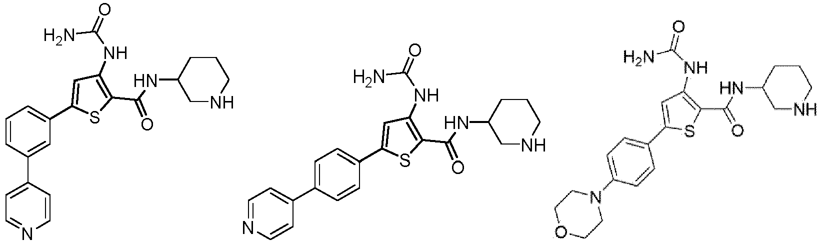 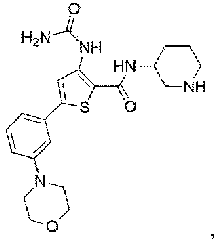 including pharmaceutically acceptable salts thereof.

In various embodiments, the compound can thus be selected from the following compounds: 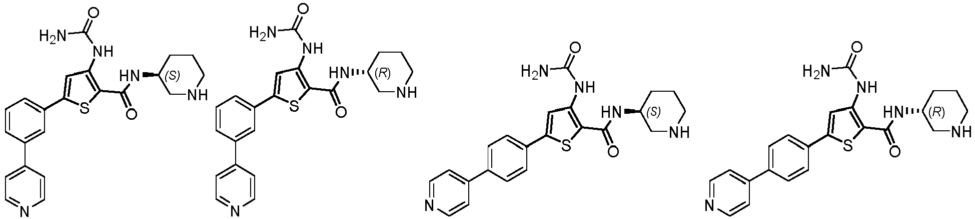 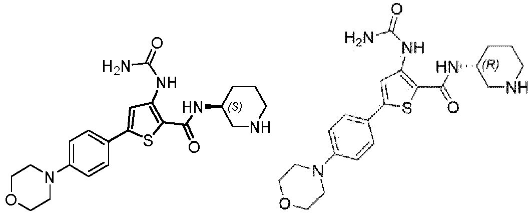 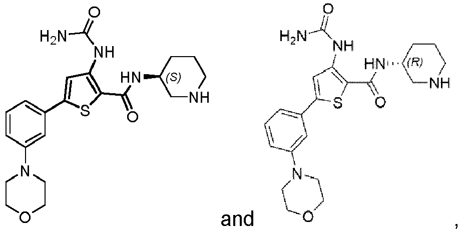 including pharmaceutically acceptable salts thereof.

In various embodiments, in which R⁵ is H and R⁴ is a 6-membered heterocyclyl ring containing at least one nitrogen atom, preferably 3-piperidinyl, optionally substituted on one or more carbon atoms by one or more R⁶, and wherein the nitrogen of the -NH- moiety can be optionally substituted by a group selected from R⁷, and R¹ is phenyl, wherein R¹ is substituted on one or more carbon atoms by one or more R⁶, R⁶ is imidazolyl.

In some embodiments, the compound can thus be selected from the following compounds: 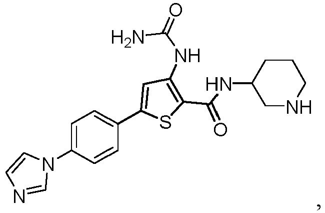 including pharmaceutically acceptable salts thereof.

In some embodiments, the compound can thus be selected from the following compounds: 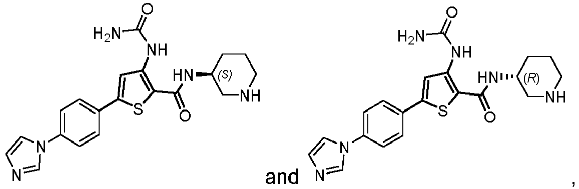 including pharmaceutically acceptable salts thereof.

In various other embodiments, in which R⁵ is H and R⁴ is a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷; R¹ is a heterocyclyl group, optionally substituted on one or more carbon atoms by one or more R⁶ and further if said heterocyclyl contains an -NH- moiety, the nitrogen of said moiety can be optionally substituted by a group selected from R⁷.

In some such embodiments, R⁴ is a 6-membered heterocyclyl ring containing at least one nitrogen atom, preferably 3-piperidinyl, optionally substituted on one or more carbon atoms by one or more R⁶, and wherein the nitrogen of the -NH- moiety can be optionally substituted by a group selected from R⁷, preferably unsubstituted 3-piperidinyl. In some of these embodiments, R¹ can be benzofuranyl, such as 2-benzofuranyl. In such embodiments, the compound can be selected from the following compounds: 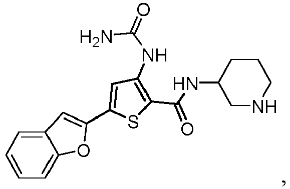 including pharmaceutically acceptable salts thereof.

In some embodiments, the compound can thus be selected from the following compounds: 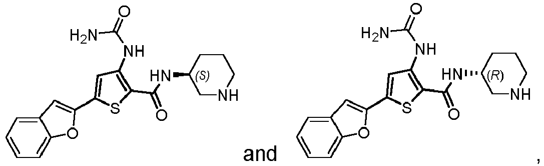 including pharmaceutically acceptable salts thereof.

In some other of these embodiments, R¹ can be pyridinyl, such as 3-pyridinyl. In such embodiments, the compound can be selected from the following compounds: 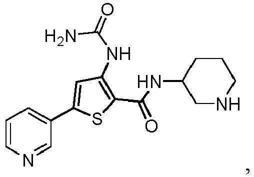 including pharmaceutically acceptable salts thereof.

In some embodiments, the compound can thus be selected from the following compounds:

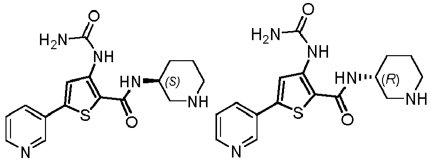

In various embodiments, in which R⁵ is H and R⁴ is a 6-membered heterocyclyl ring containing at least one nitrogen atom, preferably 3-piperidinyl, optionally substituted on one or more carbon atoms by one or more R⁶, and wherein the nitrogen of the -NH- moiety can be optionally substituted by a group selected from R⁷, R¹ is 4-pyrazolyl optionally substituted on one or more carbon atoms by one or more R⁶ and optionally substituted on any nitrogen by a group selected from R⁷. In some such embodiments, the R⁶ on the pyrazolyl can be C₁₋₆ alkyl, preferably methyl, and/or the R⁷ on the pyrazolyl can be aryl, more preferably phenyl. In some such embodiments, R⁴ is 3-piperidinyl, R¹ is 4-pyrazolyl substituted by C₁₋₆ alkyl on one of the carbon atoms, such as the carbon in the 3-position, preferably methyl, and/or the R⁷ is aryl, preferably phenyl. Examples for such compound can be selected from the following compounds: 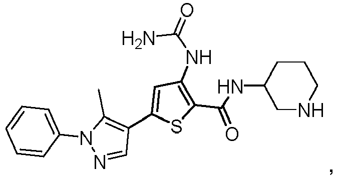 including pharmaceutically acceptable salts thereof.

In some embodiments, the compound can thus be selected from the following compounds: 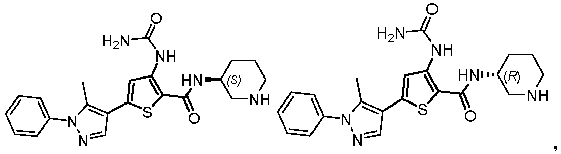 including pharmaceutically acceptable salts thereof.

In various embodiments, R⁵ is H and R⁴ is a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷; and R¹ is selected from aryl preferably substituted on one or more carbon atoms by one or more R⁶, R⁴ is a substituted heterocyclyl ring with each R⁶ and each R⁷ substituent on R¹ and R⁴ being independently selected from halo, -O(C₁₋₆ alkyl), nitro, aryl, and heterocyclyl. In some of these embodiments, R⁴ is a 6-membered heterocyclyl ring containing at least one nitrogen atom, preferably 3-piperidinyl, substituted on one or more carbon atoms by one or more R⁶, wherein each R⁶ on R⁴ is independently selected from halo, preferably fluoro; and wherein R¹ is phenyl, optionally substituted on one or more carbon atoms by one or more R⁶, wherein each R⁶ on R¹ is independently selected from halo; -O(C₁₋₆ alkyl); nitro; aryl; and heterocyclyl, preferably halo. Exemplary compound are the following: 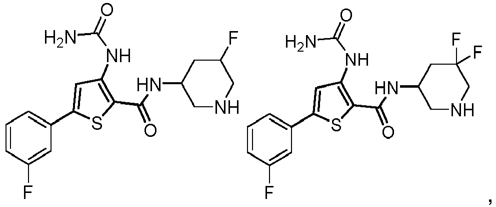 including pharmaceutically acceptable salts thereof.

In some embodiments, the compound can thus be selected from the following compounds: 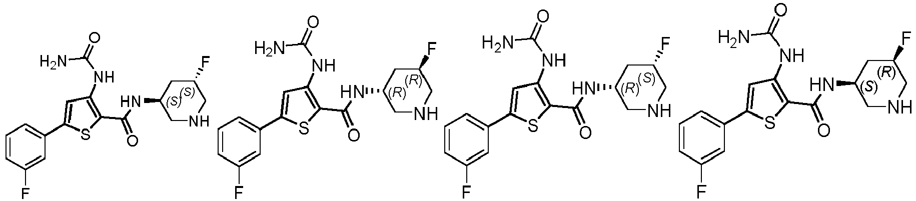 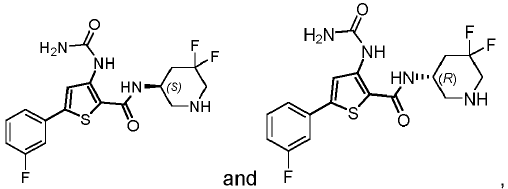 including pharmaceutically acceptable salts thereof.

In various embodiments, R⁵ is H and R⁴ is a 5-membered heterocyclyl ring containing at least one nitrogen atom, preferably 3-pyrrolidinyl, optionally substituted on one or more carbon atoms by one or more R⁶ and optionally substituted on the nitrogen of said moiety by a group selected from R⁷; with R¹ being phenyl, optionally substituted on one or more carbon atoms by one or more R⁶, wherein each R⁶ and each R⁷ being independently selected from halo, -O(C₁₋₆ alkyl), nitro, aryl, and heterocyclyl. In some embodiments, R⁴ is 3-pyrrolidinyl, R¹ is phenyl substituted on one or more carbon atoms by one or more R⁶, and R⁶ on R¹ is halo, preferably fluoro. Such compounds include, but are not limited to: 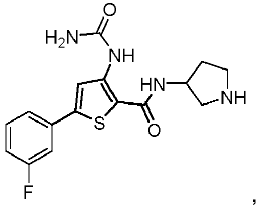 including pharmaceutically acceptable salts thereof.

In some embodiments, the compound can thus be selected from the following compounds: 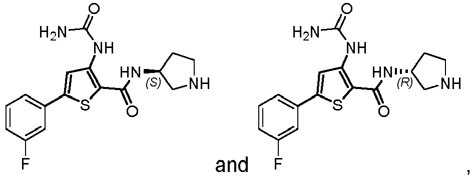 including pharmaceutically acceptable salts thereof.

In various embodiments, R⁵ is H and R⁴ can be a 7-membered heterocyclyl ring containing at least one nitrogen atom, preferably azepan-3-yl, optionally substituted on one or more carbon atoms by one or more R⁶ and optionally substituted on the nitrogen of said moiety by R⁷; with R¹ being phenyl, optionally substituted on one or more carbon atoms by one or more R⁶, wherein each R⁶ and each R⁷ is independently selected from halo, -O(C₁₋₆ alkyl), nitro, aryl, and heterocyclyl. In some of these embodiments, R⁴ is azepan-3-yl, R¹ is phenyl substituted on one or more carbon atoms by one or more R⁶, and R⁶ on R¹ is halo, preferably fluoro. Exemplary compound include the following compounds: 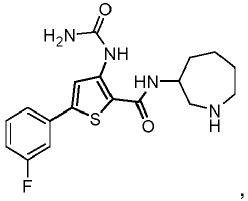 including pharmaceutically acceptable salts thereof.

In some embodiments, the compound can thus be selected from the following compounds: 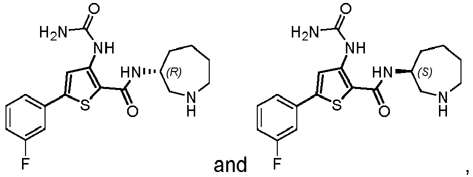 including pharmaceutically acceptable salts thereof.

In various embodiments, R⁵ is H and R⁴ is a 6-membered heterocyclyl ring containing at least one nitrogen atom, preferably 4-piperidinyl, optionally substituted on one or more carbon atoms by one or more R⁶ and optionally substituted on the nitrogen of said moiety by R⁷; with R¹ being phenyl, optionally substituted on one or more carbon atoms by one or more R⁶, wherein each R⁶ and each R⁷ is independently selected from halo, -O(C₁₋₆ alkyl), nitro, aryl, and heterocyclyl. In some of these embodiments, R⁶ on R¹ is halo, preferably fluoro. An exemplary compound, without limitation, is the following compound: 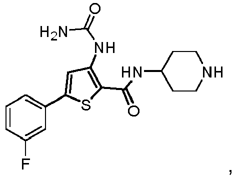 including pharmaceutically acceptable salts thereof.

In various embodiments, R⁵ is H and R⁴ is a 7-membered heterocyclyl ring containing at least one nitrogen atom, preferably 2,6-diazaspiro[3.3]heptane-2-yl, optionally substituted on one or more carbon atoms by one or more R⁶ and optionally substituted on the nitrogen of said moiety by a group selected from R⁷; with R¹ being phenyl, optionally substituted on one or more carbon atoms by one or more R⁶, wherein each R⁶ and each R⁷ is independently selected from halo, -O(C₁₋₆ alkyl), nitro, aryl, and heterocyclyl. In some embodiments, R⁴ is 2,6-diazaspiro[3.3]heptane-2-yl, R¹ is phenyl substituted on one or more carbon atoms by one or more R⁶, and R⁶ on R¹ is halo, preferably fluoro. In one embodiment, the compound is: 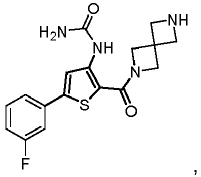 including pharmaceutically acceptable salts thereof.

In various embodiments, R⁵ is H and R⁴ is a 6-membered, optionally aromatic, heterocyclyl ring containing at least one nitrogen atom, preferably 4-pyridinyl, optionally substituted on one or more carbon atoms by one or more R⁶ and optionally substituted on the nitrogen of said moiety by a group selected from R⁷; and R¹ is phenyl, optionally substituted on one or more carbon atoms by one or more R⁶, wherein each R⁶ and each R⁷ is independently selected from halo, -O(C₁₋₆ alkyl), nitro, aryl; and heterocyclyl. In some embodiments, R⁴ is 4-pyridinyl, R¹ is phenyl substituted on one or more carbon atoms by one or more R⁶, and R⁶ on R¹ is halo, preferably fluoro. Exemplary compounds include, but are not limited to the following: 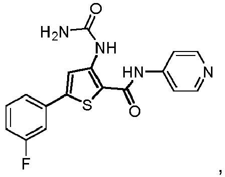 including pharmaceutically acceptable salts thereof.

In various embodiments, R⁵ is H and R⁴ is a 6-membered, optionally aromatic, heterocyclyl ring containing at least one nitrogen atom, preferably 5-methylpyridin-2-yl, optionally substituted on one or more carbon atoms by one or more R⁶ and optionally substituted on the nitrogen of said moiety by a group selected from R⁷; wherein R¹ is phenyl, optionally substituted on one or more carbon atoms by one or more R⁶, and wherein each R⁶ and each R⁷ is independently selected from halo, -O(C₁₋₆ alkyl), nitro, aryl; and heterocyclyl. In some of these embodiments, R⁴ is 5-methylpyridin-2-yl, R¹ is phenyl substituted on one or more carbon atoms by one or more R⁶, and R⁶ on R¹ is halo, preferably fluoro, for example the following compound: 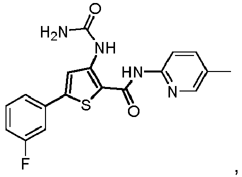 including pharmaceutically acceptable salts thereof.

In various embodiments, R⁵ is H and R⁴ is a 6-membered, optionally aromatic, heterocyclyl ring containing at least one nitrogen atom, preferably pyrazin-2-yl, optionally substituted on one or more carbon atoms by one or more R⁶ and optionally substituted on the nitrogen of said moiety by a group selected from R⁷; wherein R¹ is phenyl, optionally substituted on one or more carbon atoms by one or more R⁶, wherein each R⁶ and each R⁷ is independently selected from halo, -O(C₁₋₆ alkyl), nitro, aryl; and heterocyclyl. In some embodiments, R⁴ is pyrazin-2-yl, R¹ is phenyl substituted on one or more carbon atoms by one or more R⁶, and R⁶ on R¹ is halo, preferably fluoro, for example the following compound: 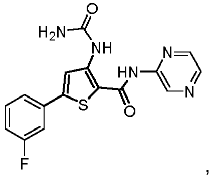 including pharmaceutically acceptable salts thereof.

In various other embodiments, R⁵ is H and R⁴ is cycloalkyl, preferably cyclohexyl, optionally substituted on one or more carbon atoms by one or more R⁶; R¹ is aryl, preferably phenyl, optionally substituted on one or more carbon atoms by one or more R⁶, wherein each R⁶ is independently selected from halo, -O(C₁₋₆ alkyl), nitro, aryl, and heterocyclyl. In some of these embodiments, R⁴ is cyclohexyl, R¹ is phenyl substituted on one or more carbon atoms by one or more R⁶, and each R⁶ on R¹ is independently selected from halo, preferably fluoro, for example the following compound: 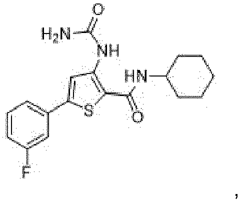 including pharmaceutically acceptable salts thereof.

In the context of the present invention, reference is made to mutations in KIT or PDGFRA, both of which are key factors in the development and progression of GIST. As used herein, a "mutation" refers to an addition (insertion), deletion, or substitution of a single nucleotide at a site in a nucleic acid molecule encoding for KIT or PDGFRA, for example resulting in the onset of the disease and/or conferring resistance to a particular therapy, such as treatment with imatinib. Thus, in particular aspects of the invention, an alteration in the sequence of KIT and/or PDGFRA results in a change that affects the activity, expression, or stability of a transcript or polypeptide encoded by the sequence such that certain cellular pathways are deregulated and/or at least some resistance to therapy with kinase inhibitor drugs, such as imatinib, occurs as a result.

KIT mutations commonly found in GIST are KIT exon 8 mutations (extracellular (EC) domain), KIT exon 9 mutations (EC domain), KIT exon 10 mutations, KIT exon 11 mutations (juxtamembrane (JM) domain), KIT exon 13 mutations (ATP binding site on TKD, first catalytic domain), KIT exon 14 mutations (ATP binding site on tyrosine kinase domain (TKD)), KIT exon 17 mutations (intracellular (IC) domain of activation loop, second catalytic domain), and KIT exon 18 mutations (activation loop (AL)).

PDGFRA mutations commonly encountered in GIST are PDGFRA exon 12 mutations (JMD), PDGFRA exon 13 mutations, PDGFRA exon 14 mutations (ATP binding site TKD), PDGFRA exon 15 mutations, and PDGFRA exon 18 mutations (activation loop of the IC domain).

Accordingly, in various embodiments, the GIST to be treated with a compound according to the present invention, comprises a KIT 8 exon mutation, a KIT exon 9 mutation, a KIT exon 10 mutation, a KIT exon 11 mutation, a KIT exon 13 mutation, a KIT exon 14 mutation, a KIT exon 17 mutation, a KIT exon 18 mutation, a PDGFRA exon 12 mutation, a PDGFRA exon 13 mutation, a PDGFRA exon 14 mutation, a PDGFRA exon 15 mutation, or a PDGFRA exon 18 mutation or any combination of two or more thereof, wherein the mutation can be a missense mutation, insertion mutation, a deletion mutation, or a combination thereof.

In some embodiments, the mutation in the GIST to be treated is a primary mutation. In the context of the present invention, the term "primary mutation" refers to a mutation in KIT or PDGFRA. Primary mutations in KIT (mostly e11 or e9) can be treated with imatinib . Once secondary mutations in KIT occur, imatinib becomes ineffective, and 2^{nd} line therapy sunitinib is typically prescribed.

For instance but without limitation, the primary mutation can be a KIT 8 exon mutation, a KIT exon 9 mutation, a KIT exon 10 mutation, a KIT exon 11 mutation, a KIT exon 13 mutation, a KIT exon 14 mutation, a KIT exon 17 mutation, a PDGFRA exon 12 mutation, a PDGFRA exon 13 mutation, a PDGFRA exon 14 mutation, a PDGFRA exon 15 mutation, or a PDGFRA exon 18 mutation.

In various embodiments, the primary mutation is a KIT exon 9 mutation, a KIT exon 11 mutation, a KIT exon 13 mutation or a KIT exon 17 mutation. While primary mutations in KIT exon 13 and KIT exon 17 are less prevalent, KIT exon 9 and KIT exon 11 are the most common types of primary KIT mutations. Accordingly, in various embodiments, the tumor has a primary mutation in KIT exon 9 or Kit exon 11 mutations.

In various other embodiments, the primary mutation is a PDGFRA exon 18 mutation or a PDGFRA exon 12 mutation. Thus, in various embodiments, the tumor has a primary mutation selected from PDGFRA exon 18 and PDGFRA exon 12 mutations.

In the following, if reference is made to specific mutations, the affected amino acids are indicated by their one letter codes. Further, the conventional nomenclature for substitutions, deletions and insertions is used. For example, "D842V" thus means that the aspartate (D) in position 842 is replaced by a valine (V). If two target amino acids are indicated, such as "N659K/T", this means that both mutations from N659 to K as well as to T are possible.

In various embodiments, the tumor has a KIT exon 9 mutation selected from the group consisting of p.A502_Y503dup, p.Y503_F504insAY, and p.S476I, with these mutations being on the protein level (as indicated by the "p.").

In various other embodiments, the tumor has a KIT exon 11 mutation affecting codons 550-570. In various such embodiments, the tumor has a KIT exon 11 mutation affecting codons 550-560, for instance affecting codons 557-560. In various such embodiments, the tumor has a KIT exon 11 mutation affecting codons 557-558. In various other embodiments, the tumor has a KIT exon 11 mutation affecting codons 568-570. In various embodiments, the tumor has a KIT exon 11 mutation selected from the group consisting of deletions involving or affecting codons 557/558, deletions upstream of codons 557/558, and deletions downstream of codons 557/558.

In various other embodiments, the tumor has a KIT exon 13 mutation leading to an amino acid substitution selected from K642E, V65A, and T670I.

In various other embodiments, the tumor has a KIT exon 17 mutation selected from mutations involving codons 809, 816, 820, 822 or 823.

In other embodiments, the tumor has a PDGFRA exon 12 mutation selected from the group consisting of p.W559_R560del and p.P581S.

In other embodiments, the tumor has a PDGFRA exon 18 mutation selected from the group consisting of p.D842_M84del, p.D842_I843delinsV, and p.D842V.

In various embodiments, the tumor has any one or more of the PDGFRA mutations: V598F, G605A, G652E, P653Q, V658A, N659K/T, T674R/I, E675G, Y676C, G680R/E, D842V, and I843_D846del.

In some embodiments, the tumor comprises (at least) the PDGFRA mutation D842V.

In some embodiments, the tumor comprises (at least) the PDGFRA mutation G680R.

In some embodiments, the tumor comprises (at least) the PDGFRA mutation G652E.

In some embodiments, the tumor comprises (at least) the PDGFRA mutation V658A.

In other embodiments, the tumor has any one or more of the KIT mutations: S476I, F522C, K642E, V654A, N655K, T670E/I, D816A/E/G/H/N/V, D820A/E/G/H/V/Y, N822I/K/Y, Y823C/D, and A829P.

In some embodiments, the tumor comprises (at least) the KIT mutation T670I.

In some embodiments, the tumor comprises (at least) the KIT mutation V654A.

In some embodiments, the tumor comprises (at least) the KIT mutation D816A.

In some embodiments, the tumor comprises (at least) the KIT mutation D820A.

In some embodiments, the tumor comprises (at least) the KIT mutation N822K.

In some embodiments, the tumor comprises (at least) the KIT mutation A829P.

In further embodiments, the tumor has at least one secondary mutation. In the context of the present invention, the term "secondary mutation" refers to mutation(s) that are typically not the initiating oncogenic event in the neoplastic disease or condition, *i.e.* in the GIST, but instead usually emerge at a later stage of GIST, particularly in the course of treatment with one or more drugs, such as, without limitation, one or more of imatinib, sunitinib, regorafenib, ripretinib, and avapritinib. In other words, a secondary mutation is a mutation the tumor has acquired at any stage after the primary mutation(s). Accordingly, in the context of the present invention, the tumor can have, in some embodiments, in addition to at least one primary mutation, as herein defined, at least one secondary mutation. The secondary mutation is essentially always a KIT or PDGFRA mutation and generally happens in the same gene as the primary mutation.

In some embodiments, the tumor has a drug resistant mutation. In the context of the present invention, the term "drug resistant mutation" refers to mutation(s) of a tumor that render treatment of the tumor with one or more drugs, non-effective, in other words, the tumor, originally and commonly susceptible to treatment with said drug, becomes unresponsive to treatment with said drug. In case of GIST, this means that drugs that are known to be effective, at least in a certain proportion of GIST cases, such as imatinib, sunitinib, regorafenib, ripretinib and avapritinib, become non-effective. Typically, tumor cells acquire such mutation during the course of a given treatment and thus escape the treatment regimen and can thus drive a relapse.

For instance, but without limitation, in some embodiments the tumor comprises a mutation that renders it imatinib resistant, sunitinib resistant, regorafenib resistant, ripretinib resistant and/or avapritinib resistant.

In the context of the present invention, the term "imatinib" refers to a compound having the following structure:

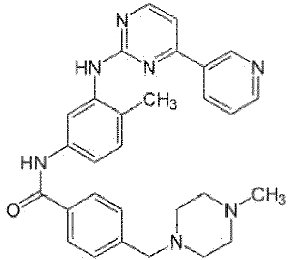

Imatinib is the typically first line drug for the treatment of GIST. In the context of the present invention, the term "imatinib resistant" refers to mutation(s) of a tumor that render treatment of the tumor with imatinib or a pharmaceutically acceptable salt thereof non-effective, in other words, the tumor is or becomes unresponsive to treatment with imatinib. This can mean that common doses used for the treatment do not have the desired effect.

In the context of the present invention, the term "sunitinib" refers to a compound having the following structure:

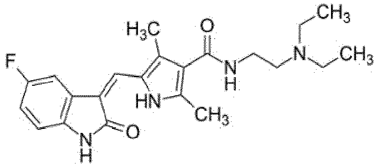

Sunitinib is the standard second-line treatment after imatinib failure, but in some instances can also be used as a third line treatment. In the context of the present invention, the term "sunitinib resistant" refers to mutation(s) of a tumor that render treatment of the tumor with sunitinib or a pharmaceutically acceptable salt thereof non-effective, in other words, the tumor is unresponsive to treatment with sunitinib or a pharmaceutically acceptable salt thereof. This can mean that common doses used for the treatment do not have the desired effect.

In the context of the present invention, the term "regorafenib" refers to a compound having the following structure:

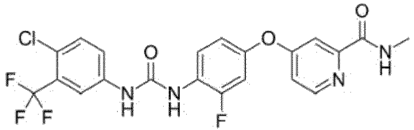

Regorafenib is active for some secondary mutations resistant to imatinib and sunitinib and is thus commonly used as a third-line treatment after imatinib and/or sunitinib failure. In the context of the present invention, the term "regorafenib resistant" refers to mutation(s) of a tumor that render treatment of the tumor with regorafenib or a pharmaceutically acceptable salt thereof non-effective, in other words, the tumor is unresponsive to treatment with regorafenib or a pharmaceutically acceptable salt thereof. This can mean that common doses used for the treatment do not have the desired effect.

In the context of the present invention, the term "ripretinib" refers to a compound having the following structure:

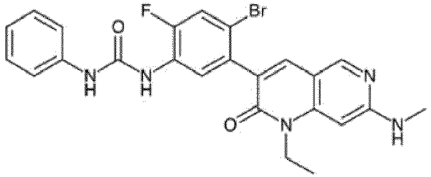

Ripretinib is commonly used as a fourth-line treatment after failure of imatinib, sunitinib and regorafenib. In the context of the present invention, the term "ripretinib resistant" refers to mutation(s) of a tumor that render treatment of the tumor with ripretinib or a pharmaceutically acceptable salt thereof non-effective, in other words, the tumor is unresponsive to treatment with ripretinib or a pharmaceutically acceptable salt thereof. This can mean that common doses used for the treatment do not have the desired effect.

In the context of the present invention, the term "avapritinib" refers to a compound having the following structure:

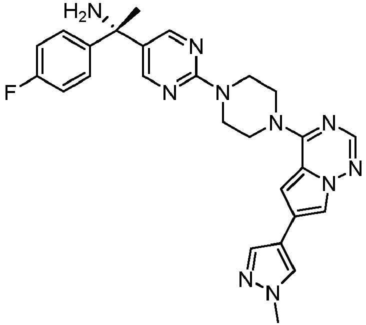

Avapritinib is currently used in the treatment of GIST with a confirmed PDGFRA D842V mutation, which is otherwise considered as refractory to treatment. In the context of the present invention, the term "avapritinib resistant" refers to mutation(s) of a tumor that render treatment of the tumor with avapritinib or a pharmaceutically acceptable salt thereof non-effective, in other words, the tumor is unresponsive to treatment with avapritinib or a pharmaceutically acceptable salt thereof. This can mean that common doses used for the treatment no longer have the desired effect.

In some embodiments, described herein is a method of treating a patient suffering from a GIST, comprising administering to said patient a pharmaceutically effective amount of a compound of formula (I) or a pharmaceutically acceptable salt thereof, as herein defined and described, wherein the patient's tumor has progressed from, or the patient was intolerant to, a previous administration of imatinib, a previous administration of sunitinib, a previous administration of regorafenib, a previous administration of ripretinib and/or a previous administration of avapritinib.

In some such embodiments, the patient's tumor has progressed from, or the patient was intolerant to, a previous administration of imatinib and a previous administration of sunitinib. In some other embodiments, the patient's tumor has progressed from, or the patient was intolerant to, a previous administration of imatinib and a previous administration of regorafenib. In some other embodiments, the patient's tumor has progressed from, or the patient was intolerant to, a previous administration of imatinib and a previous administration of ripretinib. In some other embodiments, the patient's tumor has progressed from, or the patient was intolerant to, a previous administration of imatinib and a previous administration of avapritinib.

In some other such embodiments, the patient's tumor has progressed from, or the patient was intolerant to, a previous administration of sunitinib and a previous administration of regorafenib. In some other embodiments, the patient's tumor has progressed from, or the patient was intolerant to, a previous administration of sunitinib and a previous administration of ripretinib. In some other embodiments, the patient's tumor has progressed from, or the patient was intolerant to, a previous administration of sunitinib and a previous administration of avapritinib.

In some other such embodiments, the patient's tumor has progressed from, or the patient was intolerant to, a previous administration of regorafenib and a previous administration of ripretinib. In some other embodiments, the patient's tumor has progressed from, or the patient was intolerant to, a previous administration of regorafenib and a previous administration of avapritinib.

In some other such embodiments, the patient's tumor has progressed from, or the patient was intolerant to, a previous administration of ripretinib and a previous administration of avapritinib.

In various embodiments, the compounds disclosed herein can thus be used as first line, second line, third line, fourth line or fifth line therapeutics, in particular as third, fourth or fifth line therapeutics once the standard first and second line treatments have failed. This particularly applies to GIST with KIT mutations that have escaped imatinib and/or sunitinib therapy and optionally also regorafenib and/or ripretinib treatment. In various embodiments, the compounds disclosed herein can also be used as a follow-up treatment to avapritinib treatment in GIST having the PDGFRA mutation D842V, for example as second line treatment in case first line treatment was with avapritinib.

In some embodiments, the patient's tumor has at least one primary KIT mutation, as herein defined above, and at least one secondary KIT mutation, as herein defined above. In particular secondary mutations in KIT exons 17 and 18 are known to render a tumor resistant to treatment with state-of-the-art KIT inhibitors, such as imatinib and sunitinib, those in exons 13, 14 to imatinib and ripretinib. Patents with primary exon 9 mutations frequently progress on ripretinib in the absence of secondary resistance mutations. Regorafenib shows some inhibitory activity against a broad panel of secondary resistance mutations but due to its broad activity against other kinases, KIT kinase inhibition is not effective at therapeutically feasible doses.

In some embodiments, the tumor has a primary KIT exon 9 mutation and a secondary KIT exon 13 mutation, a secondary KIT exon 14 mutation, a secondary KIT exon 17 mutation or a secondary KIT exon 18 mutation.

For instance, in some embodiments, the tumor has a primary KIT exon 9 mutation and a secondary KIT exon 13 mutation. In some embodiments, the tumor has a primary KIT exon 9 mutation and a secondary KIT exon 14 mutation. In some embodiments, the tumor has a primary KIT exon 9 mutation and a secondary KIT exon 17 mutation. In some embodiments, the tumor has a primary KIT exon 9 mutation and a secondary KIT exon 18 mutation.

In some embodiments, the tumor has a primary KIT exon 11 mutation and a secondary KIT exon 9 mutation, a secondary KIT exon 13 mutation, a secondary KIT exon 14 mutation, a secondary KIT exon 17 mutation or a secondary KIT exon 18 mutation.

For instance, in some embodiments, the tumor has a primary KIT exon 11 mutation and a secondary KIT exon 9 mutation. In some embodiments, the tumor has a primary KIT exon 11 mutation and a secondary KIT exon 13 mutation. In some embodiments, the tumor has a primary KIT exon 11 mutation and a secondary KIT exon 14 mutation. In some embodiments, the tumor has a primary KIT exon 11 mutation and a secondary KIT exon 17 mutation. In some embodiments, the tumor has a primary KIT exon 11 mutation and a secondary KIT exon 18 mutation.

In some embodiments, the tumor has a primary KIT exon 13 mutation and a secondary KIT exon 9 mutation, a secondary KIT exon 14 mutation, a secondary KIT exon 17 mutation or a secondary KIT exon 18 mutation.

For instance, in some embodiments, the tumor has a primary KIT exon 13 mutation and a secondary KIT exon 9 mutation. In some embodiments, the tumor has a primary KIT exon 13 mutation and a secondary KIT exon 14 mutation. In some embodiments, the tumor has a primary KIT exon 13 mutation and a secondary KIT exon 17 mutation. In some embodiments, the tumor has a primary KIT exon 13 mutation and a secondary KIT exon 18 mutation.

In some embodiments, the tumor has a primary KIT exon 17 mutation and a secondary KIT exon 9 mutation, a secondary KIT exon 13 mutation, a secondary KIT exon 14 mutation, or a secondary KIT exon 18 mutation.

For instance, in some embodiments, the tumor has a primary KIT exon 17 mutation and a secondary KIT exon 9 mutation. In some embodiments, the tumor has a primary KIT exon 17 mutation and a secondary KIT exon 13 mutation. In some embodiments, the tumor has a primary KIT exon 17 mutation and a secondary KIT exon 14 mutation. In some embodiments, the tumor has a primary KIT exon 17 mutation and a secondary KIT exon 18 mutation.

Alternatively, the patient's tumor can have at least one primary PDGFRA mutation, as herein defined above, and at least one secondary PDGFRA mutation, as herein defined above. Predominantly, secondary mutations in PDGFRA exons 13, 14, 15, and 18 render a tumor resistant to treatment with state-of-the-art PDGFRA inhibitors, such as imatinib, sunitinib, regorafenib, ripretinib and avapritinib, including pharmaceutically acceptable salts thereof. Primary mutations in exon 18 (particularly the D842V-mutations) render patients resistant to all state-of-the-art PDGFRA inhibitors such as imatinib, sunitinib, regorafenib, ripretinib with the exception of avapritinib. If the primary mutation is D842V and the secondary one of G652E, V658A, G652E+V658A, T674I, T674R, the tumor can be resistant to avapritinib.

In some such embodiments, the tumor has a primary PDGFRA exon 12 mutation and a secondary PDGFRA exon 13 mutation, a secondary PDGFRA exon 14 mutation, a secondary PDGFRA exon 15 mutation, or a secondary PDGFRA exon 18 mutation.

For instance, in some embodiments, the tumor has a primary PDGFRA exon 12 mutation and a secondary PDGFRA exon 13 mutation. In some embodiments, the tumor has a primary PDGFRA exon 12 mutation and a secondary PDGFRA exon 14 mutation. In some embodiments, the tumor has a primary PDGFRA exon 12 mutation and a secondary PDGFRA exon 15 mutation. In some embodiments, the tumor has a primary PDGFRA exon 12 mutation and a secondary PDGFRA exon 18 mutation.

In some embodiments, the tumor has a primary PDGFRA exon 18 mutation and a secondary PDGFRA exon 13 mutation, a secondary PDGFRA exon 14 mutation, or a secondary PDGFRA exon 15 mutation.

For instance, in some embodiments, the tumor has a primary PDGFRA exon 18 mutation and a secondary PDGFRA exon 13 mutation. In some embodiments, the tumor has a primary PDGFRA exon 18 mutation and a secondary PDGFRA exon 14 mutation. In some embodiments, the tumor has a primary PDGFRA exon 18 mutation and a secondary PDGFRA exon 15 mutation. In all embodiments disclosed herein, the PDGFRA exon 18 mutation can be D842V.

In some embodiments, the tumor additionally, i.e., in addition to at least one primary and at least one secondary mutation, as herein defined above, has at least one tertiary mutation on the same allele.

Accordingly, in some embodiments, the patient's tumor has at least one primary KIT mutation, as herein defined above, and at least one secondary KIT mutation, as herein defined above, and at least one tertiary KIT mutation.

In some such embodiments, the tumor has a primary KIT exon 9 mutation, a secondary KIT exon 13 mutation, and a tertiary KIT mutation selected from a tertiary KIT exon 17 mutation and a tertiary KIT exon 18 mutation.

In some embodiments, the tumor has a primary KIT exon 9 mutation, a secondary KIT exon 14 mutation, and a tertiary KIT mutation selected from a tertiary KIT exon 13 mutation, a tertiary KIT exon 17 mutation and a tertiary KIT exon 18 mutation.

In some embodiments, the tumor has a primary KIT exon 9 mutation, a secondary KIT exon 17 mutation, and a tertiary KIT mutation selected from a tertiary KIT exon 13 mutation, a tertiary KIT exon 14 mutation, and a tertiary KIT exon 18 mutation.

In some embodiments, the tumor has a primary KIT exon 9 mutation, a secondary KIT exon 18 mutation, and a tertiary KIT mutation selected from a tertiary KIT exon 13 mutation, a tertiary KIT exon 14 mutation, and a tertiary KIT exon 17 mutation.

In some embodiments, the tumor has a primary KIT exon 11 mutation, a secondary KIT exon 9 mutation, and a tertiary KIT mutation selected from a tertiary KIT exon 13 mutation, a tertiary KIT exon 14 mutation, a tertiary KIT exon 17 mutation, and a tertiary KIT exon 18 mutation.

In some embodiments, the tumor has a primary KIT exon 11 mutation, a secondary KIT exon 13 mutation, and a tertiary KIT mutation selected from a tertiary KIT exon 14 mutation, a tertiary KIT exon 17 mutation, and a tertiary KIT exon 18 mutation.

In some embodiments, the tumor has a primary KIT exon 11 mutation, a secondary KIT exon 14 mutation, and a tertiary KIT mutation selected a tertiary KIT exon 13 mutation, a tertiary KIT exon 17 mutation, and a tertiary KIT exon 18 mutation.

In some embodiments, the tumor has a primary KIT exon 11 mutation, a secondary KIT exon 17 mutation, and a tertiary KIT mutation selected from a tertiary KIT exon 13 mutation, a tertiary KIT exon 14 mutation, and a tertiary KIT exon 18 mutation.

In some embodiments, the tumor has a primary KIT exon 11 mutation, a secondary KIT exon 18 mutation, and a tertiary KIT mutation selected a tertiary KIT exon 13 mutation, a tertiary KIT exon 14 mutation, and a tertiary KIT exon 17 mutation.

Alternatively, in some embodiments, the tumor has a primary KIT exon 13 mutation, a secondary KIT exon 9 mutation, and a tertiary KIT mutation selected from a tertiary KIT exon 14 mutation, a tertiary KIT exon 17 mutation, and a tertiary KIT exon 18 mutation.

In some embodiments, the tumor has a primary KIT exon 13 mutation, a secondary KIT exon 14 mutation, and a tertiary KIT mutation selected from a tertiary KIT exon 17 mutation and a tertiary KIT exon 18 mutation.

In some embodiments, the tumor has a primary KIT exon 13 mutation, a secondary KIT exon 17 mutation, and a tertiary KIT mutation selected from a tertiary KIT exon 14 mutation and a tertiary KIT exon 18 mutation.

In some embodiments, the tumor has a primary KIT exon 13 mutation, a secondary KIT exon 18 mutation, and a tertiary KIT mutation selected from a tertiary KIT exon 14 mutation and a tertiary KIT exon 17 mutation.

In some embodiments, the tumor has a primary KIT exon 17 mutation, a secondary KIT exon 13 mutation, and a tertiary KIT mutation selected from a tertiary KIT exon 14 mutation and a tertiary KIT exon 18 mutation.

In some embodiments, the tumor has a primary KIT exon 17 mutation, a secondary KIT exon 13 mutation, and a tertiary KIT exon 11 mutation.

In some embodiments, the tumor has a primary KIT exon 17 mutation, a secondary KIT exon 14 mutation, and a tertiary KIT mutation selected from a tertiary KIT exon 13 mutation and a tertiary KIT exon 18 mutation.

In some embodiments, the tumor has a primary KIT exon 17 mutation, a secondary KIT exon 18 mutation, and a tertiary KIT mutation selected from a tertiary KIT exon 13 mutation and a tertiary KIT exon 14 mutation.

In some cases, a patient's tumor can develop a complex set of mutations, commonly referred to as complex compound mutations. In various embodiments, the tumor has complex compound mutations selected from double-in-cis mutations or triple-in-cis mutations. In the context of the present invention, the term "double-in-cis mutations" refers to double mutations in *cis* on the same allele, and the term "triple-in-cis mutations" refers to triple mutations in *cis* on the same allele.

In some embodiments, the tumor has a primary KIT exon 9 mutation and at least one acquired mutation selected from mutations in KIT exon 13, KIT exon 14, KIT exon 17, and KIT exon 18. In some embodiments, the tumor has a primary KIT exon 9 mutation and at least one acquired mutation in KIT exon 13 or KIT exon 14 and at least one acquired mutation in KIT exon 17 or KIT exon 18.

In some embodiments, the tumor has a primary KIT exon 11 mutation and at least one acquired mutation selected from mutations in KIT exon 13, KIT exon 14, KIT exon 17, and KIT exon 18. In some embodiments, the tumor has a primary KIT exon 11 mutation and at least one acquired mutation in KIT exon 13 or KIT exon 14 and at least one acquired mutation in KIT exon 17 or KIT exon 18.

In various embodiments, the tumor has an AP/AL genotype, *i.e.,* a genotype featuring at least one mutation in the ATP-binding pocket (AP) of KIT and at least one mutation in the activation loop (AL) of KIT. In some such embodiments, the AL mutation is a primary mutation and the AP mutation is a secondary mutation.

Specifically, in various embodiments, the tumor has a KIT exon 11 mutation and a KIT exon 17 mutation. In various such embodiments, the tumor further has an acquired KIT exon 13 mutation.

In various other specific embodiments, the tumor has a KIT exon 17 mutation and a KIT exon 13 mutation.

In another specific embodiment, the tumor has a PDGFRA D842V mutation.

In various other specific embodiments, the tumor has a PDGFRA exon 18 primary mutation (including but not limited to the D842V mutation) and secondary mutations in PDGFRA exons 13 or 14 or 15.

In various other specific embodiments, the tumor has a primary PDGFRA exon 12 mutation and secondary mutations in PDGFRA exons 13 or 14 or 15 or 18.

In various other specific embodiments, the tumor has a primary PDGFRA exon 14 mutation and secondary mutations in PDGFRA exons 13 or 15 or 18.

The compounds of the present invention, *e.g.,* compounds according to formula (I) as defined herein, can be advantageously used in the treatment of drug-resistant GISTs. Particularly, in some embodiments of the method of the present invention, the patient was previously treated with one or more kinase inhibitors selected from imatinib, sunitinib, regorafenib, lapatinib, gefinitib, erlotinib, vatlanib, crenolanib, ripretinib, nintedanib, ponartinib, nilotinib, axitinib, cabozantinib, pazopanib, pexidartinib, bezuclastinib, dasatinib, sorafenib, avapritinib, and pharmaceutically acceptable salts of any of the foregoing.

For instance, in some embodiments, the patient was previously treated with imatinib, sunitinib, regorafenib, ripretinib and/or avapritinib. In some such embodiments, the patient was previously treated with imatinib. In some embodiments, the patient was previously treated with sunitinib. In some embodiments, the patient was previous treated with regorafenib. In some embodiments, the patient was previously treated with ripretinib. In some embodiments, the patient was previously treated with avapritinib.

In some embodiments, the tumor is an advanced gastrointestinal stromal tumor that has progressed from, or the patient was intolerant to, administration of imatinib, administration of sunitinib, administration of regorafenib, administration of ripretinib and/or administration of avapritinib, or wherein the patient has a documented intolerance to one or more of imatinib, sunitinib, regorafenib, ripretinib and/or avapritinib.

In some embodiments, the tumor is an advanced gastrointestinal stromal tumor that has progressed from, or the patient was intolerant to, a first line administration of imatinib, a second line administration of sunitinib, and an optional third line administration of regorafenib or ripretinib, or wherein the patient has a documented intolerance to one or more of imatinib, sunitinib, regorafenib and/or ripretinib.

In some embodiments, described herein is a method of treating a patient suffering from a GIST, comprising administering to said patient a pharmaceutically effective amount of a compound of formula (I) or a pharmaceutically acceptable salt thereof, as herein defined and described, wherein the patient's tumor has progressed from, or the patient was intolerant to, a previous first line administration of imatinib, or wherein the patient has a documented intolerance to imatinib.

In some embodiments, the patient's tumor has progressed from, or the patient was intolerant to, a previous first line administration of imatinib and a previous second line administration of sunitinib, or wherein the patient has a documented intolerance to one or more of imatinib and/or sunitinib.

In some embodiments, the patient's tumor has progressed from, or the patient was intolerant to, a previous first line administration of imatinib, a previous second line administration of sunitinib, and a previous third line administration of regorafenib, or wherein the patient has a documented intolerance to one or more of imatinib, sunitinib and/or regorafenib.

In some embodiments, the patient's tumor has progressed from, or the patient was intolerant to, a previous first line administration of imatinib, a previous second line administration of sunitinib, and a previous third line administration of regorafenib, and a previous fourth line administration of ripretinib, or wherein the patient has a documented intolerance to one or more of imatinib, sunitinib, regorafenib, and/or ripretinib.

In some embodiments, the patient's tumor has progressed from, or the patient was intolerant to, a previous first line administration of imatinib, a previous second line administration of sunitinib, and a previous third line administration of regorafenib, and a previous fourth line administration of ripretinib, and a previous fifth line administration of avapritinib, or wherein the patient has a documented intolerance to one or more of imatinib, sunitinib, regorafenib, ripretinib and/or avapritinib.

In some embodiments, the tumor has a a KIT exon 9 mutation, a KIT exon 11 mutation, a KIT exon 13 mutation, a KIT exon 14 mutation, a KIT exon 17 mutation, or a KIT exon 18 mutation, preferably a KIT exon 9 mutation selected from the group consisting of p.A502_Y503dup, p.Y503_F504insAY, and p.S476l; a KIT exon 11 mutation selected from the group consisting of deletions involving codons 557/558, deletions upstream of codons 557/558, deletions downstream of codons 557/558, and deletions affecting codons 557/558;
a KIT exon 13 mutation selected from K642E, V654A, and T670I; or
a KIT exon 17 mutation selected from mutations involving codons 816, 820 or 823.

In other embodiments, the tumor is an advanced gastrointestinal stromal tumor that has progressed from, or the patient was intolerant to, administration of avapritinib, or wherein the patient has a documented intolerance to avapritinib.

In such embodiments, avapritinib can have been the first line therapy or can have been used as second, third, fourth, fifith or further line treatment. In such embodiments, the treatment disclosed herein can be used directly after the avapritinib treatment has been terminated/discontinued.

In some such embodiments, the tumor has a PDGFRA exon 12 mutation, a PDGFRA exon 13 mutation, a PDGFRA exon 14 mutation, a PDGFRA exon 15 mutation, or a PDGFRA exon 18 mutation, preferably a PDGFRA exon 12 mutation selected from the group consisting of p.W559_R560del and p.581S; or
a PDGFRA exon 18 mutation selected from the group consisting of p.D842_M84del, p.D842_I843delinsV, and p.D842V.

In various embodiments, particularly, the patient is a human patient.

A compound as described herein, *e.g.,* a compound according to formula (I) as defined herein, can be formulated as a pharmaceutical composition using a pharmaceutically acceptable carrier and administered by a variety of routes. Therefore, in various embodiments, the compound of formula (I), as herein defined, is comprised in a pharmaceutical composition, particularly together with at least one pharmaceutically acceptable carrier.

In the context of the present invention, the term "pharmaceutical composition" refers to a formulation of a compound according to formula (I) or a pharmaceutically acceptable salt thereof, as defined and described herein, and a generally accepted medium in the art for distributing the biologically active compound to mammals, for example, human beings. This includes all pharmaceutically acceptable carriers, diluents or excipients.

In some embodiments, such compositions according to the present invention are for oral administration. In some embodiments, compositions formulated for oral administration are provided as tablets. In some embodiments, such compositions are for parenteral (by injection) administration (*e.g.,* a composition formulated for local injection at the site of a tumor). In some embodiments, such compositions are for transdermal administration. In some embodiments, such compositions are for topical administration. In some embodiments, such compositions are for intravenous (IV) administration. In some embodiments, such compositions are for intramuscular (IM) administration. Such pharmaceutical compositions and processes for preparing them are well known in the art. See, *e.g.,* REMINGTON: THE SCIENCE AND PRACTICE OF PHARMACY (A. Gennaro, et al, eds., 19th ed. Mack Publishing Co., 1995).

Compounds of the present invention can be administered orally, sublingually, intramuscularly, subcutaneously, topically, intranasally, intraperitoneally, intrathoracially, intravenously, epidurally, intrathecally, intracerebroventricularly and by injection into a joint, or in the form of a parenteral, buccal, vaginal, or rectal administration, by inhalation, or by insufflation. Thus, in various embodiments, the compound of formula (I) is administered orally, parenterally, buccally, vaginally, rectally, by inhalation, by insufflation, sublingually, intramuscularly, subcutaneously, topically, intranasally, intraperitoneally, intrathoracially, intravenously, epidurally, intrathecally, intracerebroventricularly and by injection into the joints. In some aspects, the compound can be administered intravenously.

The dosage will depend on the route of administration, the severity of the disease, age and weight of the patient and other factors normally considered by the attending physician, when determining the individual regimen and dosage level as the most appropriate for a particular patient.

An effective amount of a compound of the present invention for use in therapy of a disease or condition is an amount sufficient to symptomatically relieve in a patient, particularly in a human, the symptoms of the disease or condition, to slow the progression of the disease or condition, or to reduce in patients with symptoms of the disease or condition the risk of getting worse. Particularly, in various embodiments, an effective amount is an amount that results in prolonged cell stasis of GIST cells and/or cytocidal cell elimination of GIST and/or apoptosis of GIST cells and/or eradication of GIST cells and/or regression of a GIST and/or reduction of the volume of the GIST tumor and/or inhibition of new GIST growth.

It is generally understood that an effective amount can vary depending on the mode of administration, specific location of the disease or disorder, and the subject's age, body weight and general health. The amount of compounds administered will depend on the degree, severity and type of the disease or condition, the amount of therapy desired and the release characteristics of the pharmaceutical formulation (s). It will also depend on the subject's health, size, weight, age, sex and tolerance to drugs. Typically, the compounds are administered for a period of time sufficient to achieve the desired therapeutic effect.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances, which can also act as diluents, flavoring agents, solubilizers, lubricants, suspending agents, binders, or tablet disintegrating agents; it can also be an encapsulating material.

In powders, the carrier is a finely divided solid, which is in a mixture with the finely divided active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture is then poured into convenient sized molds and allowed to cool and solidify.

Suitable carriers include magnesium carbonate, magnesium stearate, talc, lactose, sugar, pectin, dextrin, starch, tragacanth, methyl cellulose, sodium carboxymethyl cellulose, a low- melting wax, cocoa butter, and the like.

Some of the compounds of the present invention are capable of forming salts with various inorganic and organic acids and bases and such salts are also within the scope of this invention. Examples of such acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Base salts include ammonium salts, alkali metal salts such as sodium, lithium and potassium salts, alkaline earth metal salts such as aluminum, calcium and magnesium salts, salts with organic bases such as dicyclohexylamine salts, N-methyl-D-glucamine, and salts with amino acids such as arginine, lysine, ornithine, and so forth. Also, basic nitrogen-containing groups can be quaternized with such agents as: lower alkyl halides, such as methyl, ethyl, propyl, and butyl halides; dialkyl sulfates like dimethyl, diethyl, dibutyl; diamyl sulfates; long chain halides such as decyl, lauryl, myristyl and stearyl halides; aralkyl halides like benzyl bromide and others. Nontoxic physiologically-acceptable salts are preferred, although other salts are also useful, such as in isolating or purifying the product.

The salts can be formed by conventional means, such as by reacting the free base form of the product with one or more equivalents of the appropriate acid in a solvent or medium in which the salt is insoluble, or in a solvent such as water, which is removed in vacuo or by freeze drying or by exchanging the anions of an existing salt for another anion on a suitable ion-exchange resin.

In order to use a compound of the formula (I) or a pharmaceutically acceptable salt thereof for therapeutic treatment (including prophylactic treatment) of mammals including humans, it is normally formulated in accordance with standard pharmaceutical practice as a pharmaceutical composition. For instance, compositions according to the invention can be formulated by means known in the art into the form of, for example, tablets, capsules, aqueous or oily solutions, suspensions, emulsions, creams, ointments, gels, nasal sprays, suppositories, finely divided powders or aerosols or nebulisers for inhalation, and for parenteral use (including intravenous, intramuscular or infusion) sterile aqueous or oily solutions or suspensions or sterile emulsions. Liquid form compositions include solutions, suspensions, and emulsions. Sterile water or water-propylene glycol solutions of the active compounds can be mentioned as an example of liquid preparations suitable for parenteral administration.

Liquid compositions can also be formulated in solution, such as an aqueous polyethylene glycol solution. Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

The pharmaceutical compositions can be in unit dosage form. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms.

In addition to the compounds of the present invention, the pharmaceutical composition of this invention can also contain, or be co-administered (simultaneously or sequentially) with, one or more pharmacological agents of value in treating one or more disease conditions referred to herein.

Therefore, in some embodiments, the method of treating a patient suffering from a GIST as defined herein can comprise the sole application of a compound according to formula (I), as herein defined, or can, alternatively, comprise a combinatorial treatment, in other words, in a method according to the present invention, a compound of formula (I), as herein defined, can be administered as a sole therapy or, alternatively, the method according to the present invention can involve, in addition to administration of the compound according to formula (I), surgery and/or radiotherapy and/or additional chemotherapy. Accordingly, in various embodiments, the method of the present invention can be a combination therapy. In the context of the present invention, the term "combination therapy" refers to a treatment that includes the administration of two or more therapeutic agents, e.g., a compound according to formula (I), as herein defined and described, and a further drug, such as a further chemotherapeutically active agent, to a patient. The two or more therapeutic agents can be distributed at the same time, for example, in separate pharmaceutical compositions or in the same pharmaceutical composition, or they can be distributed at different times. For example, they can be distributed simultaneously or for concurrent periods of time, and/or a therapeutic agent can be distributed before or after the other therapeutic agent(s). Treatment with a combination of a compound according to formula (I) of the present invention and a further chemotherapeutically active agent optionally includes treatment with any single agent, preceded or followed by a period of simultaneous treatment with both agents. However, it is contemplated that, for some period, effective amounts of the two or more therapeutic agents are present within the patient.

In various embodiments, the combinatory treatment can be with at least one other known tyrosine kinase inhibitor, such as those disclosed above.

In a further aspect, the present invention also relates to a compound of formula (I) or a pharmaceutically acceptable salt thereof for use in the treatment of a gastrointestinal stromal tumor (GIST) in a patient, 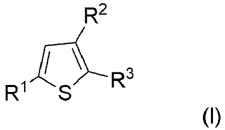 wherein
R¹ is selected from aryl and heterocyclyl, provided R¹ is not thienyl, and wherein R¹ can be optionally substituted on one or more carbon atoms by one or more R⁶; and further wherein if said heterocyclyl contains an -NH- moiety, the nitrogen of said moiety can be optionally substituted by a group selected from R⁷;
R² is -NHC(=O)NH₂;
R³ is -C(=O)NR⁴R⁵;
R⁴ is cycloalkyl or a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷;
R⁵ is H or taken together with R⁴ and the nitrogen atom to which it is attached form a heterocyclic ring, wherein said heterocyclic ring is selected from the group consisting of 4-, 5-, 6-, and 7-membered heterocyclic rings, wherein said heterocyclic ring can be optionally substituted on one or more carbon atoms by one or more R⁶, and wherein if said heterocyclyl contains a -NH- moiety, the nitrogen of said moiety can be optionally substituted by a group selected from R⁷;
each R⁶ and R⁷ is independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, - NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHS0₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁸R⁹, mercapto, -S(C₁₋₆ alkyl), -SO(C₁₋₆ alkyl), -S0₂(C₁₋₆ alkyl), -SO₂-NR⁸R⁹, wherein R⁶ and R⁷ independently of each other can be optionally substituted on one or more carbon atoms by one or more R¹⁰ and on nitrogen of any moiety that contains an NH or NH₂ by R¹¹;
R⁸ and R⁹ are each independently selected from halo, nitro, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -CHO, -C(=O)NR¹²R¹³, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR¹²R¹³, mercapto, -S(C₁₋₆ alkyl), -SO(C₁₋₆ alkyl), - SO₂(C₁₋₆ alkyl), -SO₂-NR¹²R¹³;
R¹⁰ and R¹¹ are independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, - NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHS0₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁷R⁸, mercapto, -S(C₁₋₆ alkyl), -SO(C₁₋₆ alkyl), -S0₂(C₁₋₆ alkyl), -SO₂-NR⁸R⁹,
R¹² and R¹³ are independently selected from H, C₁₋₆ alkyl, cycloalkyl, aryl, heterocyclyl; alternatively R¹² and R¹³ together with the nitrogen atom to which they are attached form a heterocyclic ring.

### Further embodiments

1. A method of treating a gastrointestinal stromal tumor (GIST) in a patient in need thereof, comprising administering to said patient a pharmaceutically effective amount of a compound of formula (I) or a pharmaceutically acceptable salt thereof, 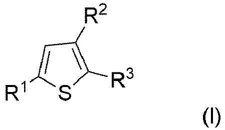 wherein
   R¹ is selected from aryl and heterocyclyl, provided R¹ is not thienyl, and wherein R¹ can be optionally substituted on one or more carbon atoms by one or more R⁶; and further wherein if said heterocyclyl contains an -NH- moiety, the nitrogen of said moiety can be optionally substituted by a group selected from R⁷;
   R² is -NHC(=O)NH₂;
   R³ is -C(=O)NR⁴R⁵;
   R⁴ is cycloalkyl or a 5, 6, or 7-membered heterocyclyl ring containing at least one nitrogen atom, wherein R⁴ may be optionally substituted on one or more carbon atoms by one or more R⁶, and wherein if said heterocyclyl contains a -NH- moiety, the nitrogen of said moiety may be optionally substituted by a group selected from R⁷;
   R⁵ is H or taken together with R⁴ and the nitrogen atom to which it is attached form a heterocyclic ring, wherein said heterocyclic ring is selected from the group consisting of 4-, 5-, 6-, and 7-membered heterocyclic rings, wherein said heterocyclic ring may be optionally substituted on one or more carbon atoms by one or more R⁶, and wherein if said heterocyclyl contains a -NH- moiety, the nitrogen of said moiety may be optionally substituted by a group selected from R⁷;
   each R⁶ and R⁷ is independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, - OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHS0₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁸R⁹, mercapto, -S(C₁₋₆ alkyl), -SO(C₁₋₆ alkyl), -S0₂(C₁₋₆ alkyl), -SO₂-NR⁸R⁹, wherein R⁶ and R⁷ independently of each other can be optionally substituted on one or more carbon atoms by one or more R¹⁰ and on nitrogen of any moiety that contains an NH or NH₂ by R¹¹;
   R⁸ and R⁹ are each independently selected from halo, nitro, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -CHO, -C(=O)NR¹²R¹³, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR¹²R¹³, mercapto, -S(C₁₋₆ alkyl), -SO(C₁₋₆ alkyl), -S0₂(C₁₋₆ alkyl), -SO₂-NR¹²R¹³;
   R¹⁰ and R¹¹ are independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHS0₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁷R⁸, mercapto, -S(C₁₋₆ alkyl), -SO(C₁₋₆ alkyl), -S0₂(C₁₋₆ alkyl), -SO₂-NR⁸R⁹,
   R¹² and R¹³ are independently selected from H, C₁₋₆ alkyl, cycloalkyl, aryl, heterocyclyl; alternatively
   R¹² and R¹³ together with the nitrogen atom to which they are attached form a heterocyclic ring.
2. The method of embodiment 1, wherein R¹ is selected from aryl optionally substituted on one or more carbon atoms by one or more R⁶.
3. The method of embodiment 2, wherein R¹ is phenyl optionally substituted on one or more carbons atoms by one or more R⁶, wherein R⁶ is preferably halo, more preferably fluoro.
4. The method of embodiment 3, wherein R¹ is 3-fluorophenyl.
5. The method of any one of embodiments 1 to 4, wherein R⁵ is H.
6. The method of any one of embodiments 1 to 5, wherein R⁴ is a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷.
7. The method of embodiment 6, wherein R⁴ is a 6-membered heterocyclyl ring, preferably 3-piperidinyl, optionally substituted on one or more carbon atoms by one or more R⁶, and wherein the nitrogen of the a -NH- moiety can be optionally substituted by a group selected from R⁷
8. The method of any one of embodiments 1 to 7, wherein the compound is 5-(3-fluorophenyl)-N-(piperidin-3-yl)-3-ureidothiophene-2-carboxamide or a pharmaceutically acceptable salt thereof, preferably (S)-5-(3-fluorophenyl)-N-(piperidin-3-yl)-3-ureidothiophene-2-carboxamide or a pharmaceutically acceptable salt thereof, optionally the hydrochloride salt thereof.
9. The method of any one of embodiments 1 to 8, wherein the tumor has a drug resistant mutation.
10. The method of embodiment 9, wherein the tumor has an imatinib resistant mutation, sunitinib resistant mutation, regorafenib resistant mutation, ripretinib resistant mutation, avapritinib resistant mutation, or any combination thereof.
11. The method of any one of embodiments 1 to 10, wherein the tumor has a KIT 8 exon mutation, a KIT exon 9 mutation, a KIT exon 10 mutation, a KIT exon 11 mutation, a KIT exon 13 mutation, a KIT exon 14 mutation, a KIT exon 17 mutation, a KIT exon 18 mutation, a PDGFRA exon 12 mutation, a PDGFRA exon 13 mutation, a PDGFRA exon 14 mutation, a PDGFRA exon 15 mutation, or a PDGFRA exon 18 mutation, wherein optionally the mutation is a missense mutation, insertion mutation, a deletion mutation, or a combination thereof.
12. The method of embodiment 11, wherein the mutation is a primary mutation.
13. The method of any one of embodiments 1 to 12, wherein the tumor has:
   - a KIT exon 9 mutation selected from the group consisting of p.A502_Y503dup, p.Y503_F504insAY, and p.S464L;
   - a KIT exon 11 mutation selected from the group consisting of deletions involving codons 557/558, deletions upstream of codons 557/558, deletions downstream of codons 557/558, and deletions affecting codons 557/558;
   - as a KIT exon 13 mutation selected from K642E, V65A, and T670I;
   - a KIT exon 17 mutation selected from mutations involving codons 809, 816, 820, 822, or 823;
   - a KIT exon 18 mutation selected from mutations involving codon 829,
   - a PDGFRA exon 12 mutation selected from the group consisting of p.W559_R560del and p.581S;
   - a PDGFRA exon 18 mutation selected from the group consisting of p.D842_M84del, p.D842_I843delinsV, and p.D842V.
14. The method of any one of embodiments 1 to 13, wherein the tumor has one or more PDGFRA mutations selected from the group consisting of V598F, G605A, G652E, P653Q, V658A, N659K/T, T674R/I, E675G, Y676C, G680R/E, D842V, and !843_D846de!.
15. The method of embodiment 14, wherein the tumor has the PDGFRA mutation D842V.
16. The method of embodiment 14, wherein the tumor has the PDGFRA mutation G680R.
17. The method of embodiment 14, wherein the tumor has the PDGFRA mutation G652E.
18. The method of embodiment 14, wherein the tumor has the PDGFRA mutation V658A.
19. The method of any one of embodiments 1 to 13, wherein the tumor has one or more KIT mutations selected from the group consisting of S476I, F522C, K642E, V654A, N655K, T670E/I, D816A/E/G/H/N/V, D820A/E/G/H/V/Y, N822I/K/Y, Y823C/D, and A829P.
20. The method of embodiment 19, wherein the tumor has the KIT mutation T670I.
21. The method of embodiment 19, wherein the tumor has the KIT mutation V654A.
22. The method of embodiment 19, wherein the tumor has the KIT mutation D816A.
23. The method of embodiment 19, wherein the tumor has the KIT mutation D820A.
24. The method of embodiment 19, wherein the tumor has the KIT mutation N822K.
25. The method of embodiment 19, wherein the tumor has the KIT mutation A829P.
26. The method of any one of embodiments 1 to 25, wherein the tumor has at least one primary and at least one secondary mutation.
27. The method of embodiment 26, wherein the tumor has a primary KIT exon 9 mutation and a secondary KIT exon 13 mutation, a secondary KIT exon 14 mutation, a secondary KIT exon 17 mutation or a secondary KIT exon 18 mutation.
28. The method of embodiment 26, wherein the tumor has a primary KIT exon 11 mutation and a secondary KIT exon 13 mutation, a secondary KIT exon 14 mutation, a secondary KIT exon 17 mutation or a secondary KIT exon 18 mutation.
29. The method of embodiment 26, wherein the tumor has a primary KIT exon 13 mutation and a, a secondary KIT exon 14 mutation, a secondary KIT exon 17 mutation or a secondary KIT exon 18 mutation.
30. The method of embodiment 26, wherein the tumor has a primary KIT exon 17 mutation and a secondary KIT exon 13 mutation, a secondary KIT exon 14 mutation, or a secondary KIT exon 18 mutation.
31. The method of embodiment 26, wherein the tumor has a primary PDGFRA exon 12 mutation and, a secondary PDGFRA exon 13 mutation, a secondary PDGFRA exon 14 mutation, a secondary PDGFRA exon 15 mutation, or a secondary PDGFRA exon 18 mutation.
32. The method of embodiment 26, wherein the tumor has a primary PDGFRA exon 18 mutation and a secondary PDGFRA exon 12 mutation, a secondary PDGFRA exon 13 mutation, a secondary PDGFRA exon 14 mutation, and a secondary PDGFRA exon 15 mutation.
33. The method of any one of embodiments 1 to 32, wherein the tumor has compound mutations selected from double-in-cis mutations or triple-in-cis mutations.
34. The method of embodiment 33, wherein the tumor has a primary KIT exon 9 mutation and at least one acquired mutation selected from mutations in KIT exon 13, KIT exon 14, KIT exon 17, and KIT exon 18.
35. The method of embodiment 34, wherein the tumor has a primary KIT exon 9 mutation and at least one acquired mutation in KIT exon 13 or KIT exon 14 and at least one acquired mutation in KIT exon 17 or KIT exon 18.
36. The method of embodiment 33, wherein the tumor has a primary KIT exon 11 mutation and at least one acquired mutation selected from mutations in KIT exon 13, KIT exon 14, KIT exon 17, and KIT exon 18.
37. The method of embodiment 36, wherein the tumor has a primary KIT exon 11 mutation and at least one acquired mutation in KIT exon 13 or KIT exon 14 and at least one acquired mutation in KIT exon 17 or KIT exon 18.
38. The method of any one of embodiments 1 to 37, wherein the patient was previously treated with one or more kinase inhibitors selected from imatinib, sunitinib, regorafenib, lapatinib, gefinitib, erlotinib, vatlanib, crenolanib, ripretinib, nintedanib, ponartinib, nilotinib, axitinib, cabozantinib, pazopanib, pexidartinib, bezuclastinib, dasatinib, sorafenib, and avapritinib, or a pharmaceutically acceptable salt thereof.
39. The method of embodiment 38, wherein the patient was previously treated with imatinib, sunitinib, regorafenib, ripretinib and/or avapritinib.
40. The method of embodiment 38 or 39, wherein the patient was previously treated with imatinib.
41. The method of any one of embodiments 38 to 40, wherein the patient was previously treated with sunitinib.
42. The method of any one of embodiments 38 to 41, wherein the patient was previous treated with regorafenib.
43. The method of any one of embodiments 38 to 42, wherein the patient was previously treated with ripretinib.
44. The method of any one of embodiments 38 to 43, wherein the patient was previously treated with avapritinib.
45. The method of any one of embodiments 1 to 44, wherein the tumor is an advanced gastrointestinal stromal tumor that has progressed from, or the patient was intolerant to, administration of imatinib, administration of sunitinib, administration of regorafenib, administration of ripretinib and/or administration of avapritinib, or wherein the patient has a documented intolerance to one or more of imatinib, sunitinib, regorafenib, ripretinib, and avapritinib.
46. The method of any one of embodiments 1 to 45, wherein the tumor is an advanced gastrointestinal stromal tumor that has progressed from, or the patient was intolerant to, a first line administration of imatinib, a second line administration of sunitinib, and an optional third line administration of regorafenib or ripretinib, or wherein the patient has a documented intolerance to one or more of imatinib, sunitinib, regorafenib and/or ripretinib.
47. The method of embodiment 46, wherein the tumor has a KIT 8 exon mutation, a KIT exon 9 mutation, a KIT exon 10 mutation, a KIT exon 11 mutation, a KIT exon 13 mutation, a KIT exon 14 mutation, a KIT exon 17 mutation, or a KIT exon 18 mutation, preferably a KIT exon 9 mutation selected from the group consisting of p.A502_Y503dup, p.Y503_F504insAY, and p.S464L;
   a KIT exon 11 mutation selected from the group consisting of deletions involving codons 557/558, deletions upstream of codons 557/558, deletions downstream of codons 557/558, and deletions affecting codons 557/558;
   a KIT exon 13 mutation selected from K642E, V65A, and T670I; or
   a KIT exon 17 mutation selected from mutations involving codons 816, 820 or 823.
48. The method of any one of embodiments 1 to 45, wherein the tumor is an advanced gastrointestinal stromal tumor that has progressed from, or the patient was intolerant to, administration of avapritinib, or wherein the patient has a documented intolerance to avapritinib.
49. The method of embodiment 48, wherein the tumor has a PDGFRA exon 12 mutation, a PDGFRA exon 13 mutation, a PDGFRA exon 14 mutation, a PDGFRA exon 15 mutation, or a PDGFRA exon 18 mutation, preferably a PDGFRA exon 12 mutation selected from the group consisting of p.W559_R560del and p.P581S; or
   a PDGFRA exon 18 mutation selected from the group consisting of p.D842_M84del, p.D842_I843delinsV, and p.D842V.
50. The method of any one of embodiments 1 to 49, wherein the patient is a human patient.
51. The method of any one of embodiments 1 to 50, wherein the compound of formula (I) is comprised in a pharmaceutical composition together with a pharmaceutically acceptable carrier.
52. The method of any one of embodiments 1 to 51, wherein the compound of formula (I) is administered orally, parenterally, buccally, vaginally, rectally, by inhalation, by insufflation, sublingually, intramuscularly, subcutaneously, topically, intranasally, intraperitoneally, intrathoracially, intravenously, epidurally, intrathecally, intracerebroventricularly, or by injection into a joint.
53. A compound of formula (I) or a pharmaceutically acceptable salt thereof for use in the treatment of a gastrointestinal stromal tumor (GIST) in a patient, 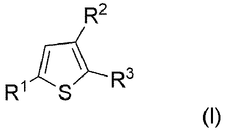
wherein
R¹ is selected from aryl and heterocyclyl, provided R¹ is not thienyl, and wherein R¹ can be optionally substituted on one or more carbon atoms by one or more R⁶; and further wherein if said heterocyclyl contains an -NH- moiety, the nitrogen of said moiety can be optionally substituted by a group selected from R⁷;
R² is -NHC(=O)NH₂;
R³ is -C(=O)NR⁴R⁵;
R⁴ is cycloalkyl or a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷;
R⁵ is H or taken together with R⁴ and the nitrogen atom to which it is attached form a heterocyclic ring, wherein said heterocyclic ring is selected from the group consisting of 4-, 5-, 6-, and 7-membered heterocyclic rings, wherein said heterocyclic ring can be optionally substituted on one or more carbon atoms by one or more R⁶, and wherein if said heterocyclyl contains a -NH- moiety, the nitrogen of said moiety can be optionally substituted by a group selected from R⁷;
each R⁶ and R⁷ is independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHSO₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁸R⁹, mercapto, -S(C₁₋₆ alkyl), -SO(C₁₋₆ alkyl), -SO₂(C₁₋₆ alkyl), -SO₂-NR⁸R⁹, wherein R⁶ and R⁷ independently of each other can be optionally substituted on one or more carbon atoms by one or more R¹⁰ and on nitrogen of any moiety that contains an NH or NH₂ by R¹¹;
R⁸ and R⁹ are each independently selected from halo, nitro, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -CHO, -C(=O)NR¹²R¹³, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR¹²R¹³, mercapto, -S(C₁₋₆ alkyl), -SO(C₁₋₆ alkyl), -SO₂(C₁₋₆ alkyl), -SO₂-NR¹²R¹³;
R¹⁰ and R¹¹ are independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHSO₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁷R⁸, mercapto, -S(C₁₋₆ alkyl), -SO(C₁₋₆ alkyl), -SO₂(C₁₋₆ alkyl), -SO₂-NR⁸R⁹,
R¹² and R¹³ are independently selected from H, C₁₋₆ alkyl, cycloalkyl, aryl, heterocyclyl; alternatively
R¹² and R¹³ together with the nitrogen atom to which they are attached form a heterocyclic ring.

All embodiments and features disclosed, described, and defined herein in the context of the method of treatment according to the present invention, i.e., a method of treating a patient suffering from a GIST, comprising administering to said patient a pharmaceutically effective amount of a compound of formula (I) or a pharmaceutically acceptable salt thereof, likewise apply to the compounds according to formula (I) or pharmaceutically acceptable salts thereof for use in the treatment of a GIST in a patient, and *vice versa.*

The compounds disclosed herein can be prepared in a number of ways well known to one skilled in the art of organic synthesis. The compounds of the present invention can, for example, be synthesized using the methods described below, together with synthetic methods known in the art of synthetic organic chemistry, or variations thereon as appreciated by those skilled in the art. Such methods include, but are not limited to, those described below.

All references cited herein are hereby incorporated in their entirety by reference. The novel compounds of this invention can be prepared using the reactions and techniques described herein. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and workup procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents, which are not compatible with the reaction conditions, will be readily apparent to one skilled in the art and alternate methods must then be used. The starting materials for the examples contained herein are either commercially available or are readily prepared by standard methods from known materials. For example, the following reactions are illustrations but not limitations of the preparation of some of the starting materials and examples used herein.

### Examples

### Example 1: Compound Synthesis

### Preparation Method 1: Synthesis of Thioacetates

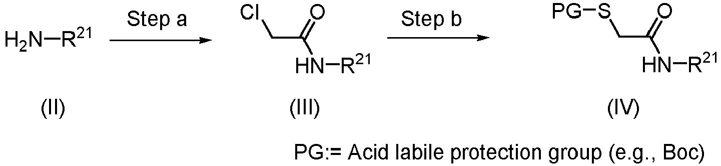

The reaction in above Step a is a process of introducing an amido group at variable positions of a given amine compound by allowing the amine compound represented by Chemical Formula (II) to react with chloroacetyl chloride (III).
Specifically, the reaction in Step a is performed at a temperature of 0 °C to 25 °C in the presence of an amine base. In this case, the amine base can be selected from mono-, di-, or tri-C₁₋₆ alkylamine or preferably from the heterocyclic amine pyridine. As a reaction solvent, a typical organic solvent can be used, and the Examples of the present invention specifically exemplify examples in which tetrahydrofuran (THF) is usually used, but the solvent of the present invention is not limited thereto.

The reaction in above Step b is a process of substituting a chloro group with a sulfide group by allowing the amide compound represented by Chemical Formula (III) to react with potassium thioacetate.
Specifically, the reaction in Step b is performed at room temperature without any organic or inorganic base. The reaction can be performed with a biphasic mixture of water and a typical organic solvent, like tetrahydrofuran. The mixed solvent can be used by appropriately mixing water and tetrahydrofuran within a volume ratio range of 1 : 3 to 3 : 1.

### [Preparation Examples of Method 1]

The compounds synthesized in the Examples of the present invention were subjected to structural analysis under the following HPLC conditions.
(1) HPLC Condition for Structural Analysis
   - Elution Solvent A: 0.1% trifluoroacetic acid (TFA)/water
   - Elution Solvent B: 0.1% trifluoroacetic acid (TFA)/CH₃CN
   - Column: NUCLEODUR^{™} C18 Gravity, 3 µm, 125 x 4 mm analytical column (Macherey- Nagel, Germany)
   - Elution Condition: Elution for 9.5 minutes while varying the concentration of Solvent B from 10 to 100 % at a moving rate of 1.0 mL/min.

### Preparation Example 1.1: Preparation of tert-butyl (S)-3-(2-(acetylthio)acetamido) piperidine-1-carboxylate

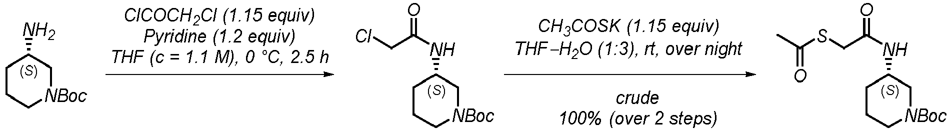

### Step 1. Preparation of tert-butyl (S)-3-(2-chloroacetamido)piperidine-1-carboxylate

In an oven-dried two-necked round bottom flask equipped with a dropping funnel *tert*-butyl (S)-3-aminopiperidine-1-carboxylate (C₁₀H₂₀N₂O₂, 200.28 g/mol, 5 g, 25 mmol, 1 equiv.) was dissolved in tetrahydrofuran (THF, C₄H₈O, c = 1.1 M, 22.5 mL) under an argon stream at 0 °C. After this, pyridine (C₅H₅N, 79.10 g/mol, 0.98 g/mL, 2.4 g, 2.4 mL, 30 mmol, 1.2 equiv.) and subsequently chloroacetyl chloride (C₂H₂Cl₂O, 112.94 g/mol, 1.42 g/mL, 3.2 g, 2.3 mL, 28.7 mmol, 1.15 equiv.) were added dropwise over a period of 20 minutes *via* dropping funnel, followed by a line wash of tetrahydrofuran (2 mL). After 2.5 h at ambient temperature, the reaction progress was monitored by TLC and 16 % (w/w) aqueous sodium chloride solution was added once all starting materials were fully consumed. This mixture was stirred for 30 min before separating off the aqueous phase. The organic phase was used directly for the next synthesis step without any further purification.

### Step 2. Preparation of tert-butyl (S)-3-(2-(acetylthio)acetamido) piperidine-1-carboxylate

In a one-necked round bottom flask was added a filtered solution of potassium thioacetate (C₂H₃KOS, 114.21 g/mol, 4.3 g, 37.4 mmol, 1.5 equiv.) in water (H₂O, c = 4.3 M, 8.6 mL) to the organic phase of Step 1. After stirring overnight at ambient temperature, the organic phase was checked for full conversion via thin layer chromatography (TLC). The aqueous layer was then separated with ethyl acetate (3x) and the combined organic phases were dried over Na₂SO₄ before evaporating the solvent under reduced pressure. The product was obtained as an orange yellow high viscous oil (C₁₄H₂₄N₂O₄S, 316.42 g/mol, 7.9 g, 25 mmol, 100 % (crude, over 2 steps)) and was used without further purification as a building block for Preparation Method 3 detailed below.

**High performance liquid chromatography-mass spectrometry (electrospray ionization mass spectrometry (HPLC-MS (ESI-MS)):** Calculated: 316.15 for C₁₄H₂₄N₂O₄S [M+H⁺]. Found: [M+H⁺+Na⁺] 339.2; [M+H⁺-Boc] 317.1. *t*_{R} = 5.7 min (HPLC Condition 1).

### Preparation Example 1.2: Preparation of tert-butyl (R)-3-(2-(acetylthio)acetamido) piperidine-1-carboxylate

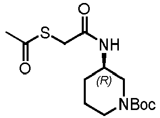

A target compound was obtained by repeating the same procedure as in Preparation Example 1.1, except that in Step 1 of Preparation Example 1.1, *tert*-butyl (*R*)-3-aminopiperidine-1-carboxylate (C₁₀H₂₀N₂O₂, 200.28 g/mol, 5000 mg, 25 mmol, 1 equiv.) was used instead of *tert*-butyl (*S*)-3-aminopiperidine-1-carboxylate. The product *tert*-butyl (*R*)-3-(2-(acetylthio)acetamido) piperidine-1-carboxylate (C₁₄H₂₄N₂O₄S, 316.42 g/mol, 1 equiv.) was obtained as an orange brown high viscous oil.

**HPLC-MS (ESI-MS):** Calculated: 316.15 for C₁₄H₂₄N₂O₄S [M+H⁺]. Found: [M+H⁺+Na⁺] 339.2. ***t*_{R}** = 5.5 min (HPLC Condition 1).

### Preparation Example 1.3: Preparation of tert-butyl (S)-3-(2-(acetylthio)acetamido)-pyrrolidine-1-carboxylate

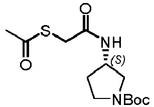

A target compound was obtained by repeating the same procedure as in Preparation Example 1.1, except that in Step 1 of Preparation Example 1.1, *tert*-butyl (*S*)-3-aminopyrrolidine-1-carboxylate (C₉H₁₈N₂O₂, 186.25 g/mol, 2000 mg, 10.7 mmol, 1 equiv.) was used instead of *tert*-butyl (*S*)-3-aminopiperidine-1-carboxylate. The product *tert*-butyl (*S*)-3-(2-(acetylthio)acetamido)-pyrrolidine-1-carboxylate (C₁₃H₂₂N₂O₄S, 302.39 g/mol, 1 equiv.) was obtained as a yellow oil.

**HPLC-MS (ESI-MS):** Calculated: 302.13 for C₁₃H₂₂N₂O₄S [M+H⁺]. Found: [M+H⁺+Na⁺] 325.1. ***t*_{R} =** 5.8 min (HPLC Condition 1).

### Preparation Example 1.4: Preparation of tert-butyl (R)-3-(2-(acetylthio)acetamido)-pyrrolidine-1-carboxylate

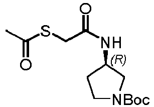

A target compound was obtained by repeating the same procedure as in Preparation Example 1.1, except that in Step 1 of Preparation Example 1.1, *tert*-butyl (*S*)-3-aminopyrrolidine-1-carboxylate (C₉H₁₈N₂O₂, 186.25 g/mol, 2000 mg, 10.7 mmol, 1 equiv.) was used instead of *tert*-butyl (*S*)-3-aminopiperidine-1-carboxylate. The product (C₁₃H₂₂N₂O₄S, 302.39 g/mol, 1 equiv.) was obtained as a dark yellow oil. **HPLC-MS (ESI-MS):** Calculated: 302.13 for C₁₃H₂₂N₂O₄S [M+H⁺]. Found: [M+H⁺+Na⁺] 325.1. *t*_{R} = 5.8 min (HPLC Condition 1).

### Preparation Example 1.5: Preparation of tert-butyl (S)-3-(2-(acetylthio)acetamido)-azepane-1-carboxylate

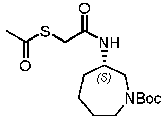

A target compound was obtained by repeating the same procedure as in Preparation Example 1.1, except that in Step 1 of Preparation Example 1.1, *tert*-butyl (*S*)-3-aminoazepane-1-carboxylate (C₁₁H₂₂N₂O₂, 214.31 g/mol, 1000 mg, 4.7 mmol, 1 equiv.) was used instead of *tert*-butyl (*S*)-3-aminopiperidine-1-carboxylate. The product *tert*-butyl (*S*)-3-(2-(acetylthio)acetamido)-azepane-1-carboxylate (C₁₅H₂₆N₂O₄S, 330.44 g/mol, 1 equiv.) was obtained as a red liquid.

**HPLC-MS (ESI-MS):** Calculated: 330.16 for C₁₅H₂₆N₂O₄S [M+H⁺]. Found: [M+H⁺-Boc] 231.1. ***t*_{R}** = 6.3 min (HPLC Condition 1).

### Preparation Example 1. 6: Preparation of tert-butyl (R)-3-(2-(acetylthio)acetamido)-azepane-1-carboxylate

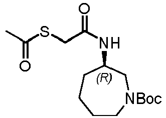

A target compound was obtained by repeating the same procedure as in Preparation Example 1.1, except that in Step 1 of Preparation Example 1.1, *tert*-butyl (*R*)-3-aminoazepane-1-carboxylate (C₁₁H₂₂N₂O₂, 214.31 g/mol, 1.02 g/mL, 600 mg, 0.6 mL, 2.8 mmol, 1 equiv.) was used instead of tert-butyl (S)-3-aminopiperidine-1-carboxylate. The product *tert*-butyl (*R*)-3-(2-(acetylthio)acetamido)-azepane-1-carboxylate (C₁₅H₂₆N₂O₄S, 330.44 g/mol, 1 equiv.) was obtained as a dark red liquid.

**HPLC-MS (ESI-MS):** Calculated: 330.16 for C₁₅H₂₆N₂O₄S [M+H⁺]. Found: [M+H⁺-Boc] 231.1. ***t*_{R}** = 6.5 min (HPLC Condition 1).

### Preparation Example 1.7: Preparation of S-(2-(cyclohexylamino)-2-oxoethyl) ethane-thioate

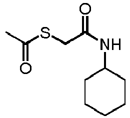

A target compound was obtained by repeating the same procedure as in Preparation Example 1.1, except that in Step 1 of Preparation Example 1.1, cyclohexylamine (C₆H₁₃N, 99.18 g/mol, 0.86 g/mL, 3500 mg, 4.1 mL, 35.3 mmol, 1 equiv.) was used instead of *tert*-butyl (S)-3-aminopiperidine-1-carboxylate. The product *S*-(2-(cyclohexylamino)-2-oxoethyl) ethane-thioate (C₁₀H₁₇NO₂S, 215.31 g/mol, 1 equiv.) was obtained as a crude mixture as a yellow liquid.

**HPLC-MS (ESI-MS):** Calculated: 215.10 for C₁₀H₁₇NO₂S [M+H⁺], Found: 216.0. ***t*_{R}** = 8.1 min (HPLC Condition 1).

### Preparation Example 1.8: Preparation of S-(2-oxo-2-(pyridin-4-ylamino)ethyl) ethane-thioate

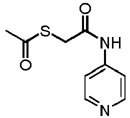

A target compound was obtained by repeating the same procedure as in Preparation Example 1.1, except that in Step 1 of Preparation Example 1.1, fampridin (C₅H₆N₂, 94.12 g/mol, 3500 mg, 37.2 mmol, 1 equiv.) was used instead of *tert*-butyl (*S*)-3-aminopiperidine-1-carboxylate. The product *S*-(2-oxo-2-(pyridin-4-ylamino)ethyl) ethane-thioate (C₉H₁₀N₂O₂S, 210.25 g/mol, 1 equiv.) was obtained as a crude mixture as a black solid.

**HPLC-MS (ESI-MS):** Calculated: 210.05 for C₉H₁₀N₂O₂S [M+H⁺], Found: [M+Na⁺] 232.1. ***t*_{R}** = 2.1 min (HPLC Condition 1).

### Preparation Example 1.9: Preparation of tert-butyl 4-(2-(acetylthio)acetamido)piperidine-1-carboxylate

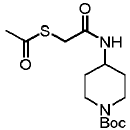

A target compound was obtained by repeating the same procedure as in Preparation Example 1.1, except that in Step 1 of Preparation Example 1.1, *tert*-butyl 4-aminopiperidine-1-carboxylate (C₁₀H₂₀N₂O₂, 200.28 g/mol, 1500 mg, 7.5 mmol, 1 equiv.) was used instead of *tert*-butyl (*S*)-3-aminopiperidine-1-carboxylate. The product *tert*-butyl 4-(2-(acetylthio)acetamido)piperidine-1-carboxylate (C₁₄H₂₄N₂O₄S, 316.42 g/mol, 1 equiv.) was obtained as a crude mixture as an orange-brown solid.

**HPLC-MS (ESI-MS):** Calculated: 316.15 for C₁₄H₂₄N₂O₄S [M+H⁺]. Found: [M+H⁺+Na⁺] 339.2; [M+H⁺-Boc] 219. ***t*_{R}** = 5.5 min (HPLC Condition 1).

### Preparation Example 1.10: Preparation of tert-butyl 6-(2-(acetylthio)acetyl)-2,6-diazaspiro[3.3]heptane-2-carboxylate

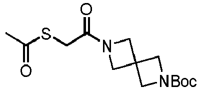

A target compound was obtained by repeating the same procedure as in Preparation Example 1.1, except that in Step 1 of Preparation Example 1.1, *tert*-butyl 2,6-diazaspiro[3.3]heptane-2-carboxylate oxalate (C₂₂H₃₈N₄O₈, 486.57 g/mol, 1000 mg, 2.1 mmol, 1 equiv.) was used instead of *tert*-butyl (S)-3-aminopiperidine-1-carboxylate and an additional equivalent of Pyridine was added to neutralize the oxalate salt. The product *tert*-butyl 6-(2-(acetylthio)acetyl)-2,6-diazaspiro[3.3]heptane-2-carboxylate (C₁₄H₂₂N₂O₄S, 314.40 g/mol, 1 equiv.) was obtained as a crude mixture as a red liquid.

**HPLC-MS (ESI-MS):** Calculated: 314.13 for C₁₄H₂₂N₂O₄S [M+H⁺]. Found: [M+H⁺+Na⁺] 337.2; [M+H⁺-Boc] 215.2. ***t*_{R}** = 5.2 min (HPLC Condition 1).

### Preparation Example 1.11: Preparation of S-(2-((5-methylpyridin-2-yl)amino)-2-oxoethyl) ethanethioate

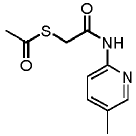

A target compound was obtained by repeating the same procedure as in Preparation Example 1.1, except that in Step 1 of Preparation Example 1.1, 5-methylpyridin-2-amine (C₆H₈N₂, 108.14 g/mol, 605.6 mg, 5.6 mmol, 1 equiv.) was used instead of *tert*-butyl (S)-3-aminopiperidine-1-carboxylate. The product *S*-(2-((5-methylpyridin-2-yl)amino)-2-oxoethyl) ethanethioate (C₁₀H₁₂N₂O₂S, 224.28 g/mol, 1 equiv.) was obtained as a crude mixture as a black liquid.
**HPLC-MS (ESI-MS):** Calculated: 224.06 for C₁₀H₁₂N₂O₂S [M+H⁺], Found: [M+Na'] 246.1. ***t*_{R}** = 1.9 min (HPLC Condition 1)

### Preparation Example 1.12: Preparation of S-(2-oxo-2-(pyrazin-2-ylamino)ethyl) ethanethioate

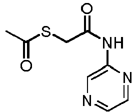

A target compound was obtained by repeating the same procedure as in Preparation Example 1.1, except that in Step 1 of Preparation Example 1.1, pyrazin-2-amine (C₄H₅N₃, 95.10 g/mol, 532.6 mg, 5.6 mmol, 1 equiv.) was used instead of *tert*-butyl (*S*)-3-aminopiperidine-1-carboxylate. The product *S*-(2-oxo-2-(pyrazin-2-ylamino)ethyl) ethanethioate (C₈H₉N₃O₂S, 211.24 g/mol, 1 equiv.) was obtained as a crude mixture as a brown-orange solid.

**HPLC-MS (ESI-MS):** Calculated: 211.04 for C₈H₉N₃O₂S [M+H⁺]. Found: 212.0. ***t*_{R}** = 0.8 min (HPLC Condition 1).

### Preparation Example 1.13: Preparation of tert-butyl (3S,5S)-3-(2-(acetylthio)acetamido)-5-fluoropiperidine-1-carboxylate

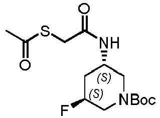

A target compound was obtained by repeating the same procedure as in Preparation Example 1.1, except that in Step 1 of Preparation Example 1.1, *tert*-butyl (3*S*,5*S*)-3-amino-5-fluoropiperidine-1-carboxylate (C₁₀H₁₉FN₂O₂, 218.27 g/mol, 804.9 mg, 3.7 mmol, 1 equiv.) was used instead of *tert*-butyl (S)-3-aminopiperidine-1-carboxylate. The product *tert*-butyl (3*S*,5*S*)-3-(2-(acetylthio)acetamido)-5-fluoropiperidine-1-carboxylate (C₁₄H₂₃FN₂O₄S, 334.41 g/mol, 1 equiv.) was obtained as a crude mixture as a high viscous orange oil.

**HPLC-MS (ESI-MS):** Calculated: 334.14 for C₁₄H₂₃FN₂O₄S [M+H⁺]. Found: [M+H⁺+Na⁺] 357.1; [M+H⁺-Boc] 219. ***t*_{R}** = 5.1 min (HPLC Condition 1).

### Preparation Example 1.14: Preparation of tert-butyl (S)-5-(2-(acetylthio)acetamido)-3,3-difluoropiperidine-1-carboxylate

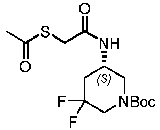

A target compound was obtained by repeating the same procedure as in Preparation Example 1.1, except that in Step 1 of Preparation Example 1.1, *tert*-butyl (S)-5-amino-3,3-difluoropiperidine-1-carboxylate (C₁₀H₁₈F₂N₂O₂, 236.26 g/mol, 871.2 mg, 3.7 mmol, 1 equiv.) was used instead of *tert*-butyl (*S*)-3-aminopiperidine-1-carboxylate. The product *tert*-butyl (*S*)-5-(2-(acetylthio)acetamido)-3,3-difluoropiperidine-1-carboxylate (C₁₄H₂₂F₂N₂O₄S, 352.40 g/mol, 1 equiv.) was obtained as a crude mixture as a highly viscous yellow oil.

**HPLC-MS (ESI-MS):** Calculated: 352.13 for C₁₄H₂₂F₂N₂O₄S [M+H⁺]. Found: [M+H⁺+Na⁺] 375.2. ***t*_{R}** = 5.5 min (HPLC Condition 1).

### Preparation Method 2: Synthesis of β-Chlorocinnamonitriles

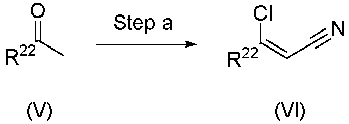

The reaction in above Step a is a process of introducing a β-chloromalononitrile group of a given keto compound by allowing the ketone compound represented by Chemical Formula (V) to react with phosphoryl chloride in presence of dimethylformamide and subsequentially adding hydroxylamine hydrochloride.

Specifically, the first step of the reaction in above Step a is performed at a temperature of 39 °C to 41 °C in the presence of a chlorinating reagent such as phosphoryl chloride, oxalyl chloride or thionyl chloride. As a reaction solvent dimethylformamide was used.

Specifically, the second step of the reaction was carried out at temperatures between 39 °C to 45 °C while adding a solution of hydroxylamine hydrochloride in dimethylformamide. After completion of this step the solution was cooled to 15 °C to 20 °C before adding water.

### [Preparation Examples of Method 2]

The compounds synthesized in the Examples of the present invention were purified or subjected to structural analysis under the following conditions.
(1) HPLC Condition for Structural Analysis - Elution Solvent A: 0.1 % trifluoroacetic acid (TFA)/water
   - Elution Solvent B: 0.1 % trifluoroacetic acid (TFA)/CH₃CN
   - Column: NUCLEODUR^{™} C18 Gravity, 3 µm, 125 x 4 mm analytical column (Macherey- Nagel, Germany)
   - Elution Condition: Elution for 9.5 minutes while varying the concentration of Solvent B from 10 to 100% at a moving rate of 1.0 mL/min.
(2) Condition 1 for Automated Flash Column Chromatography with Teledyne ISCO COMBIFLASH^{™} NextGen 300+ (Teledyne ISCO, Lincoln, NE)
   - Elution Solvent A: petroleum ether 40-60 °C (PE)
   - Elution Solvent B: ethylacetate (EtOAc)
   - Column: FLASHPURE^{™} EXOFLEX^{™} Silica, 24 g, 40 µm irregular, 60 Å pore size, preparative column (Büchi, Switzerland)
   - Elution Condition: Elution for 16 Column Volumes (CV) at a flow rate of 32 mL/min while varying the concentration of Solvent B in the following pattern: 0 % (3 CV) → 25 % (5 CV) → 25 % (5 CV) → 100 % (1 CV) → 100 % (2 CV)
(3) Condition 2 for Automated Flash Column Chromatography with COMBIFLASH^{™} NextGen 300+ (Teledyne ISCO, Lincoln, NE)
   - Elution Solvent A: n-pentane
   - Elution Solvent B: diethylether (Et₂O)
   - Column: FLASHPURE^{™} EXOFLEX^{™} Silica, 24 g, 40 µm irregular, 60 Å pore size, preparative column (Büchi, Switzerland))
   - Elution Condition: Elution for 16 Column Volumes (CV) while varying the concentration of Solvent B in the following pattern: 0 % (3 CV) → 50 % (5 CV) → 50 % (5 CV) → 100 % (1 CV) → 100 % (2 CV)

### Preparation Example 2.1: Preparation of 3-chloro-3-(3-fluorophenyl)acrylonitrile

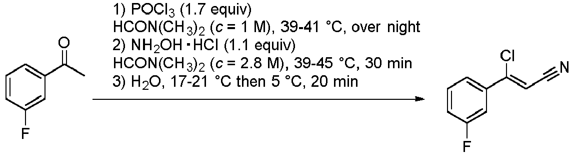

### Step 1. Preparation of 3-chloro-3-(3-fluorophenyl)acrylonitrile

In an oven-dried three-necked round bottom flask equipped with a dropping funnel, a FINDENSER^{™} (Heidolph, Germany), and a thermometer to measure the internal reaction temperature, 3'-fluoroacetophenone (C₈H₇FO, 138.14 g/mol, 1.13 g/mL, 8 g, 7.1 mL, 57.9 mmol, 1 equiv.) was dissolved in dimethylformamide (DMF, C₃H₇NO, c = 1 M, 57.9 mL) under an argon stream at 40 °C. To the resulting solution, phosphoryl chloride (POCl₃, 153.33 g/mol, 1.65 g/mL, 15.1 g, 9.2 mL 1.7 equiv.) was added dropwise, maintaining the internal temperature at 39 to 41 °C during the addition. After this, the reaction mixture was stirred at 40 °C overnight.

When TLC indicated full consumption of starting materials a solution of hydroxylamine hydrochloride (NH₄OCl, 69.49 g/mol, 4.4 g, 63.7 mmol, 1.1 equiv.) in dimethylformamide (DMF, C₃H₇NO, c = 2.8 M, 24 mL) was added dropwise, maintaining the temperature at 39-45 °C during the addition. The solution was then stirred at 40 °C for 30 min before cooling to 15-20 °C.

Water (H₂O, 7 mL) was added dropwise, keeping the internal temperature between 17-21 °C, before cooling the resulting suspension to 5 °C and stirring for another 20 min. The precipitate was filtered off, washed with two separate portions of water (2 x 2 mL) and dried under reduced pressure to afford 3-chloro-3-(3-fluorophenyl)acrylonitrile (C₉H₅ClFN, 181.60 g/mol, 7 g, 38.8 mmol, 67 %) as a yellow solid in moderate yield and sufficient purity for the next steps.

**HPLC-MS (ESI-MS):** Calculated: 181.01 for C₉H₅ClFN [M+H⁺]. Found: 182.0. ***t*_{R}** = 7.3 min (HPLC Condition 1).

### Preparation Example 2.2: Preparation of 3-(3-bromophenyl)-3-chloroacrylonitrile

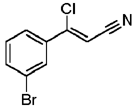

A target compound was obtained by repeating the same procedure as in Preparation Example 2.1, except that in Step 1 of Preparation Example 2.1, 3'-bromoacetophenone (C₃H₇BrO, 199.05 g/mol, 1.49 g/mL, 10.0 g, 6.7 mL, 50.2 mmol, 1 equiv.) was used instead of 3'-fluoroacetophenone. The product 3-(3-bromophenyl)-3-chloroacrylonitrile (C₉H₅ClBrN, 242.50 g/mol, 8.6 g, 35.6 mmol, 71 %) was obtained as a brown solid in moderate yield and sufficient purity for the next steps.

**HPLC-MS (ESI-MS):** Calculated: 240.93 for C₉H₅ClBrN [M+H⁺]. Found: 241.9. ***t*_{R}** = 7.1 min (HPLC Condition 1).

### Preparation Example 2.3: Preparation of 3-chloro-3-(3,4-difluorophenyl)acrylo nitrile

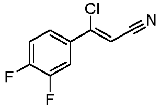

A target compound was obtained by repeating the same procedure as in Preparation Example 2.1, except that in Step 1 of Preparation Example 2.1, 3',4'-difluoroacetophenone (C₈H₆F₂O, 145.13 g/mol, 1.25 g/mL, 4000 mg, 3.2 mL, 25.6 mmol, 1 equiv.) was used instead of 3'-fluoroacetophenone. The product 3-chloro-3-(3,4-difluorophenyl)acrylo nitrile (C₉H₄ClF₂N, 199.58 g/mol, 3477.1, 17.4 mmol, 68 %) was obtained as brown yellow solid in moderate yield and sufficient purity for the next steps.

**HPLC-MS (ESI-MS):** Calculated: 199.00 for C₉H₄ClF₂N [M+H⁺]. Found: 200.0. ***t*_{R}** = 7.4 min (HPLC Condition 1).

### Preparation Example 2.4: Preparation of 3-chloro-3-(2,4-difluorophenyl)acrylonitrile

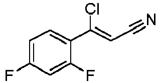

A target compound was obtained by repeating the same procedure as in Preparation Example 2.1, except that in Step 1 of Preparation Example 2.1, 2',4'-difluoroacetophenone (C₈H₆F₂O, 145.13 g/mol, 1.23 g/mL, 4000 mg, 3.2 mL, 25.6 mmol, 1 equiv.) was used instead of 3'-fluoroacetophenone. The product 3-chloro-3-(2,4-difluorophenyl)acrylonitrile (C₉H₄ClF₂N, 199.58 g/mol, 2454.4 mg, 12.3 mmol, 48 %) was obtained as crystalline brown powder in moderate yield and sufficient purity for the next steps.

**HPLC-MS (ESI-MS):** Calculated: 199.00 for C₉H₄ClF₂N [M+H⁺]. Found: 200.0. ***t*_{R}** = 7.2 min (HPLC Condition 1).

### Preparation Example 2.5: Preparation of 3-chloro-3-(2-fluorophenyl)acrylonitrile

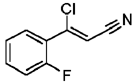

A target compound was obtained by repeating the same procedure as in Preparation Example 2.1, except that in Step 1 of Preparation Example 2.1, 2'-fluoroacetophenone (C₈H₇FO, 138.14 g/mol, 1.14 g/mL, 4000 mg, 3.5 mL, 29.0 mmol, 1 equiv.) was used instead of 3'-fluoroacetophenone. The product (C₉H₅ClFN, 181.59 g/mol, 2944.6 mg, 16.2 mmol, 56 %) was obtained as a yellow oil in moderate yield and sufficient purity for the next steps.

**HPLC-MS (ESI-MS):** Calculated: 181.01 for C₉H₅ClFN [M+H⁺]. Found: 182.0. ***t*_{R}** = 7.3 min (HPLC Condition 1).

### Preparation Example 2.6: Preparation of 3-chloro-3-(3,5-difluorophenyl)acrylonitrile

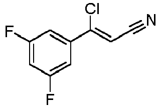

A target compound was obtained by repeating the same procedure as in Preparation Example 2.1, except that in Step 1 of Preparation Example 2.1, 3',5'-fluoroacetophenone (C₃H₆F₂O, 145.13 g/mol, 4000 mg, 25.6 mmol, 1 equiv.) was used instead of 3'-fluoroacetophenone. The product 3-chloro-3-(3,5-difluorophenyl)acrylonitrile (C₉H₄ClF₂N, 199.58 g/mol, 2913.4 mg, 14.6 mmol, 57 %) was obtained as a white solid in moderate yield and sufficient purity for the next steps.

**HPLC-MS (ESI-MS):** Calculated: 199.00 for C₉H₄ClF₂N [M+H⁺]. Found: 200.0. ***t*_{R}** = 7.6 min (HPLC Condition 1).

### Preparation Example 2.7: Preparation of 3-chloro-3-(pyridin-3-yl)acrylonitrile

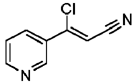

A target compound was obtained by repeating the same procedure as in Preparation Example 2.1, except that in Step 1 of Preparation Example 2.1, 3-acetylpyridine (C₇H₇NO, 121.14 g/mol, 1.10 g/mL, 2422.8 mg, 2.2 mL, 20 mmol, 1 equiv.) was used instead of 3'-fluoroacetophenone. The product 3-chloro-3-(pyridin-3-yl)acrylonitrile (C₈H₅ClN₂, 164.59 g/mol, 395 mg, 2.4 mmol, 12 %) was obtained as grey solid.

**HPLC-MS (ESI-MS):** Calculated: 164.01 for C₈H₅ClN₂ [M+H⁺], Found: [M+Na⁺] 188.1. ***t_{R}*** = 5.8 min (HPLC Condition 1).

### Preparation Example 2.8: Preparation of 3-chloro-3-(3-nitrophenyl)acrylonitrile

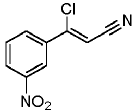

A target compound was obtained by repeating the same procedure as in Preparation Example 2.1, except that in Step 1 of Preparation Example 2.1, 3'-nitroacetophenone (C₈H₇NO₃, 165,15 g/mol, 1.19 g/mL, 4145.2 mg, 3.5 mL, 25.1 mmol, 1 equiv.) was used instead of 3'-fluoroacetophenone. The product 3-chloro-3-(3-nitrophenyl)acrylonitrile (C₉H₅ClN₂O₂, 208.60 g/mol, 3246.3 mg, 15.6 mmol, 62 %) was obtained as a yellow solid in moderate yield and sufficient purity for the next steps.

**HPLC-MS (ESI-MS):** Calculated: 208.00 for C₉H₅ClN₂O₂ [M+H⁺]. Found: 209.0. ***t*_{R}** = 7.9 min (HPLC Condition 1).

### Preparation Example 2.9: Preparation of 3-chloro-3-(3-iodophenyl)acrylonitrile

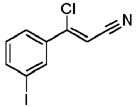

A target compound was obtained by repeating the same procedure as in Preparation Example 2.1, except that in Step 1 of Preparation Example 2.1, 3'-iodoacetophenone (C₃H₇IO, 246.04 g/mol, 1.19 g/mL, 5000 mg, 4.2 mL, 20.3 mmol, 1 equiv.) was used instead of 3'-fluoroacetophenone. The product 3-chloro-3-(3-iodophenyl)acrylonitrile (C₉H₅IFN, 289.50 g/mol, 4588.8 mg, 15.8 mmol, 78 %) was obtained as yellow crystalline powder in good yield and sufficient purity for the next steps.

**HPLC-MS (ESI-MS):** Calculated: 288.92 for C₉H₅IFN [M+H⁺]. Found: 289.9. ***t*_{R}** = 8.2 min (HPLC Condition 1).

### Preparation Example 2.10: Preparation of 3-chloro-3-(5-fluoro-2-methoxyphenyl)-acrylonitrile

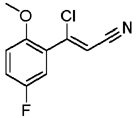

A target compound was obtained by repeating the same procedure as in Preparation Example 2.1, except that in Step 1 of Preparation Example 2.1, 5'-fluoro-2'-methoxyacetophenone (C₉H₉FO₂, 168.17 g/mol, 1.12 g/mL, 3413.8 mg, 3.1 mL, 20.3 mmol, 1 equiv.) was used instead of 3'-fluoroacetophenone. The product 3-chloro-3-(5-fluoro-2-methoxyphenyl)-acrylonitrile (C₁₀H₇ClFNO, 211.62 g/mol, 3007.1 mg, 14.2 mmol, 70 %) was obtained as red brown powder in moderate yield and sufficient purity for the next steps. **HPLC-MS (ESI-MS):** Calculated: 211.02 for C₁₀H₇ClFNO [M+H⁺]. Found: 212.0. ***t*_{R}** = 7.1 min (HPLC Condition 1).

### Preparation Example 2.11: Preparation of 3-(benzofuran-2-yl)-3-chloroacrylonitrile

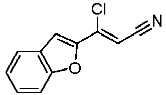

A target compound was obtained by repeating the same procedure as in Preparation Example 2.1, except that in Step 1 of Preparation Example 2.1, 1-(benzofuran-2-yl)ethan-1-one (C10H8O2, 160.17 g/mol, 4004.3 mg, 25 mmol, 1 equiv.) was used instead of 3'-fluoroacetophenone. The product 3-(benzofuran-2-yl)-3-chloroacrylonitrile (C₁₁H₆ClNO, 203.62 g/mol, 1934.5 mg, 9.5 mmol, 38 %) was obtained as an orange brown solid in moderate yield and sufficient purity for the next steps.

**HPLC-MS (ESI-MS):** Calculated: 203.01 for C₁₁H₆ClNO [M+H⁺]. Found: 204.0. ***t*_{R}** = 8.1 min (HPLC Condition 1).

### Preparation Example 2.12: Preparation of 3-chloro-3-(4-morpholinophenyl)acrylonitrile

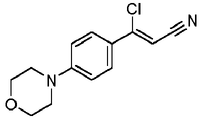

A target compound was obtained by repeating the same procedure as in Preparation Example 2.1, except that in Step 1 of Preparation Example 2.1, 1-(4-morpholinophenyl)ethan-1-one (C₁₂H₁₅NO₂, 205.26 g/mol, 4105.1 mg, 20 mmol, 1 equiv.) was used instead of 3'-fluoroacetophenone. The product 3-chloro-3-(4-morpholinophenyl)acrylonitrile (C₁₃H₁₃ClN₂O, 248.71 g/mol, 397.9 mg, 1.6 mmol, 8 %) was obtained as dark green solid in low yield and sufficient purity for the next steps.

**HPLC-MS (ESI-MS):** Calculated: 248.07 for C₁₃H₁₃ClN₂O [M+H⁺]. Found: 249.0. ***t*_{R}** = 7.1 min (HPLC Condition 1).

### Preparation Example 2.13: Preparation of 3-chloro-3-(5-methyl-1-phenyl-1H-pyrazol-4-yl)acrylonitrile

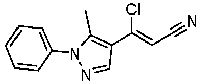

A target compound was obtained by repeating the same procedure as in Preparation Example 2.1, except that in Step 1 of Preparation Example 2.1, 1-(5-methyl-1-phenyl-1*H*-pyrazol-4-yl)ethan-1-one (C₁₂H₁₂N₂O, 200.24 g/mol, 1000 mg, 5 mmol, 1 equiv.) was used instead of 3'-fluoroacetophenone. The product 3-chloro-3-(5-methyl-1-phenyl-1H-pyrazol-4-yl)acrylonitrile (C₁₃H₁₀ClN₃, 243.69 g/mol, 499 mg, 2.1 mmol, 41 %) was obtained as brown solid in moderate yield and sufficient purity for the next steps.

**HPLC-MS (ESI-MS):** Calculated: 243.06 for C₁₃H₁₀ClN₃ [M+H⁺]. Found: 244.0. ***t*_{R}** = 6.4 min (HPLC Condition 1).

### Preparation Example 2.14: Preparation of 3-chloro-3-(4-morpholinophenyl)acrylonitrile

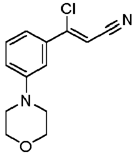

A target compound was obtained by repeating the same procedure as in Preparation Example 2.1, except that in Step 1 of Preparation Example 2.1, 1-(3-morpholinophenyl)ethan-1-one (C₁₂H₁₅NO₂, 205.26 g/mol, 1000 mg, 4.9 mmol, 1 equiv.) was used instead of 3'-fluoroacetophenone. The product 3-chloro-3-(4-morpholinophenyl)acrylonitrile (C₁₃H₁₃ClN₂O, 248.71 g/mol, 230.2 mg, 0.9 mmol, 19 %) was obtained as a yellow high viscous oil in low yield and sufficient purity for the next steps.

**HPLC-MS (ESI-MS):** Calculated: 248.07 for C₁₃H₁₃ClN₂O [M+H⁺]. Found: 249. ***t*_{R}** = 7.0 min (HPLC Condition 1).

### Preparation Example 2.15: Preparation of 3-(4-(1H-imidazol-1-yl)phenyl)-3-chloro-acrylonitrile

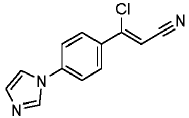

A target compound was obtained by repeating the same procedure as in Preparation Example 2.1, except that in Step 1 of Preparation Example 2.1, 1-(4-(1*H*-imidazol-1-yl)phenyl)ethan-1-one (C₁₁H₁₀N₂O, 186.21 g/mol, 2383.5 mg, 12.8 mmol, 1 equiv.) was used instead of 3'-fluoroacetophenone. The product 3-(4-(1*H-*imidazol-1-yl)phenyl)-3-chloro-acrylonitrile (C₁₂H₈ClN₃, 229.61 g/mol, 382.2 mg, 1.7 mmol, 13 %) was obtained as yellow orange solid in low yield and sufficient purity for the next steps.

**HPLC-MS (ESI-MS):** Calculated: 229.04 for C₁₂H₈ClN₃ [M+H⁺]. Found: 230. ***t*_{R}** = 4.5 min (HPLC Condition 1).

### Preparation Example 2.16: Preparation of 3-chloro-3-(3-(pyridin-4-yl)phenyl)acrylonitrile

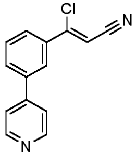

A target compound was obtained by repeating the same procedure as in Preparation Example 2.1, except that in Step 1 of Preparation Example 2.1, 1-(3-(pyridin-4-yl)phenyl)ethan-1-one (C₁₃H₁₁NO, 197.24 g/mol, 0.99 g/mL, 986.2 mg, 1 mL, 5 mmol, 1 equiv.) was used instead of 3'-fluoroacetophenone. The product 3-chloro-3-(3-(pyridin-4-yl)phenyl)acrylonitrile (C₁₄H₉ClN₂, 240.69 g/mol, 132.4 mg, 0.6 mmol, 11 %) was obtained as a purple solid in low yield and sufficient purity for the next steps.

**HPLC-MS (ESI-MS):** Calculated: 240.05 for C₁₄H₉ClN₂ [M+H⁺]. Found: 241.0. ***t*_{R}** = 4.5 min (HPLC Condition 1).

### Preparation Example 2.17: Preparation of 3-chloro-3-(4-(pyridin-4-yl)phenyl)acrylonitrile

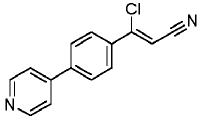

A target compound was obtained by repeating the same procedure as in Preparation Example 2.1, except that in Step 1 of Preparation Example 2.1, 1-(4-(pyridin-4-yl)phenyl)ethan-1-one (C₁₃H₁₁NO, 197.24 g/mol, 0.99 g/mL, 986.2 mg, 1 mL, 5 mmol, 1 equiv.) was used instead of 3'-fluoroacetophenone. The product 3-chloro-3-(4-(pyridin-4-yl)phenyl)acrylonitrile (C₁₄H₉ClN₂, 240.69 g/mol, 156.5 mg, 0.7 mmol, 13 %) was obtained as grey solid in low yield and sufficient purity for the next steps.

**HPLC-MS (ESI-MS):** Calculated: 240.05 for C₁₄H₉ClN₂ [M+H⁺]. Found: 241.0. ***t*_{R}** = 4.3 min (HPLC Condition 1).

### Preparation Method 3a: Synthesis of 2,3-Thiophenecarboxamideureas

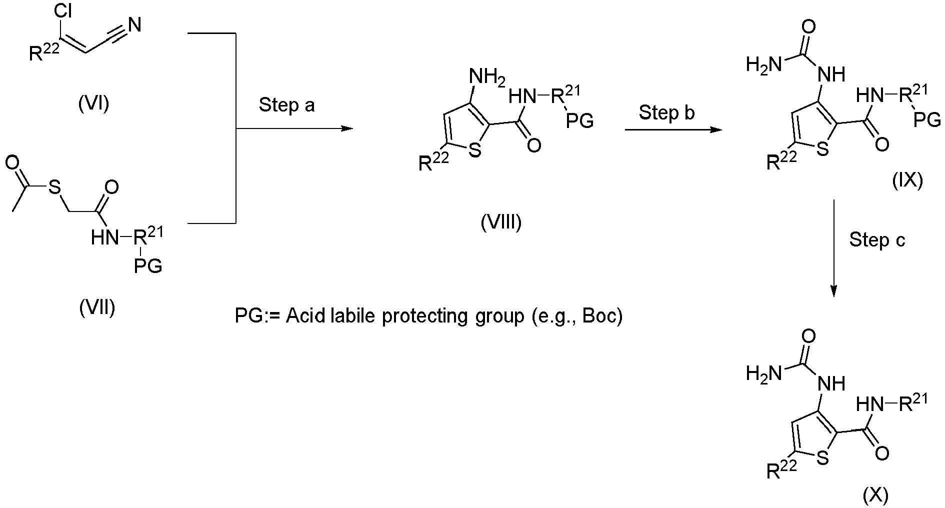

The reaction in above Step a is a process of forming a 2,3,5-substituted amino thiophene core by allowing a β-chlorocinnamonitrile compound represented by Chemical Formula (VI) to react with a thioacetate compound represented by Chemical Formula (VII).

Specifically, the reaction in above Step a was performed at a temperature of 21 °C to 24 °C in the presence of a strong alkoxide base. In this case, the base was selected from mono-, di-, or tri-C₁₋₆ alkoxide derivatives. As a reaction solvent, a typical organic solvent was used, and the Examples of the present invention specifically exemplify examples in which tetrahydrofuran (THF) was usually used, but the solvent of the present invention is not limited thereto.

The reaction in above Step b is a process of interconverting a primary amine group into a urea group at the C3 position of the thiophene core by allowing the 2,3,5-substituted amino thiophene compound represented by Chemical Formula (VIII) to react with trichloroacetyl isocyanate and subsequentially adding methanolic ammonia solution.

Specifically, the first step of the reaction in above Step b was performed at a temperature of 21 °C to 24 °C in the presence of an isocyanate compound like trichloroacetyl isocyanate. As a reaction solvent, a typical organic solvent was used, and the Examples of the present invention specifically exemplify examples in which tetrahydrofuran (THF) was usually used, but the solvent of the present invention is not limited thereto.

Specifically, the second step of the reaction in above Step b was performed at a temperature of 0 °C to 5 °C in the presence of a methanolic ammonia solution, which also serves as a solvent. The reaction in above Step c is a process of introducing a secondary amine by cleaving a *tert-*butyloxycarbonyl group.

Specifically, the reaction in above Step c was performed at a temperature of 0 °C to 25 °C in the presence of strong acid such as hydrogen chloride in an organic solvent such as 1 ,4-dioxane. As a reaction solvent, a typical anhydrous organic solvent was used, and the Examples of the present invention specifically exemplify examples in which methanol (CH₃OH) was usually used, but the solvent of the present invention is not limited thereto.

### [Preparation Examples of Method 3a]

The compounds synthesized in the Examples of the present invention were purified or subjected to structural analysis under the following conditions.
(1) HPLC Condition for Structural Analysis
   - Elution Solvent A: 0.1 % trifluoroacetic acid (TFA)/water
   - Elution Solvent B: 0.1 % trifluoroacetic acid (TFA)/CH₃CN
   - Column: NUCLEODUR^{™} C18 Gravity, 3 µm, 125 x 4 mm analytical column (Macherey- Nagel, Germany)
   - Elution Condition: Elution for 9.5 minutes while varying the concentration of Solvent B from 10 to 100 % at a moving rate of 1.0 mL/min.
(2) Condition 1a for Automated Flash Column Chromatography on COMBIFLASH^{™} NextGen 300+ (Teledyne ISCO, Lincoln, NE)
   - Elution Solvent A: petroleum Ether 40-60 °C (PE)
   - Elution Solvent B: ethylacetate (EtOAc)
   - Column: FLASHPURE^{™} EXOFLEX^{™} Silica, 24 g, 40 µm irregular, 60 Å pore size, preparative column (Büchi, Switzerland)
   - Elution Condition: Elution for 16 Column Volumes (CV) at a flow rate of 32 mL/min while varying the concentration of Solvent B in the following pattern: 0 % (3 CV) → 60 % (5 CV) → 60 % (5 CV) → 100 % (1 CV) → 100 % (2 CV)
(3) Condition 1b for Automated Flash Column Chromatography on COMBIFLASH^{™} NextGen 300+ (Teledyne ISCO, Lincoln, NE)
   - Elution Solvent A: petroleum ether 40-60 °C (PE)
   - Elution Solvent B: ethylacetate (EtOAc)
   - Column: FLASHPURE^{™} EXOFLEX^{™} Silica, 24 g, 40 µm irregular, 60 Å pore size, preparative column (Büchi, Switzerland)
   - Elution Condition: Elution for 16 Column Volumes (CV) at a flow rate of 32 mL/min while varying the concentration of Solvent B in the following pattern: 0 % (3 CV) → 80 % (5 CV) → 80 % (5 CV) → 100 % (1 CV) →f 100 % (2 CV)
(4) Condition 2a for Automated Flash Column Chromatography on COMBIFLASH^{™} NextGen 300+ (Teledyne ISCO, Lincoln, NE)
   - Elution Solvent A: dichloromethane (DCM)
   - Elution Solvent B: ethylacetate (EtOAc)
   - Column: FLASHPURE^{™} EXOFLEX^{™} Silica, 40 g, 40 µm irregular, 60 Å pore size, preparative column (Büchi, Switzerland)
   - Elution Condition: Elution for 16 Column Volumes (CV) at a flow rate of 40 mL/min while varying the concentration of Solvent B in the following pattern: 0 % (3 CV) → 30 % (3 CV) → 30 % (5 CV) → 100 % (3 CV) → 100 % (2 CV)
(5) Condition 2b for Automated Flash Column Chromatography on COMBIFLASH^{™} NextGen 300+ (Teledyne ISCO, Lincoln, NE)
   - Elution Solvent A: dichloromethane (DCM)
   - Elution Solvent B: ethylacetate (EtOAc)
   - Column: FLASHPURE^{™} EXOFLEX^{™} Silica, 40 g, 40 µm irregular, 60 Å pore size, preparative column (Büchi, Switzerland)
   - Elution Condition: Elution for 18 Column Volumes (CV) at a flow rate of 40 mL/min while varying the concentration of Solvent B in the following pattern: 0 % (3 CV) → 50 % (5 CV) → 50 % (6 CV) → 100 % (4 CV) → 100 % (2 CV)
(6) Condition 2c for Automated Flash Column Chromatography on COMBIFLASH^{™} NextGen 300+ (Teledyne ISCO, Lincoln, NE)
   - Elution Solvent A: 1 % triethylamine (TEA)/dichloromethane (DCM)
   - Elution Solvent B: 1 % triethylamine (TEA)ethylacetate (EtOAc)
   - Column: FLASHPURE^{™} EXOFLEX^{™} Silica, 24 g, 40 µm irregular, 60 Å pore size, preparative column (Büchi, Switzerland)
   - Elution Condition: Elution for 15 Column Volumes (CV) at a flow rate of 32 mL/min while varying the concentration of Solvent B in the following pattern: 0 % (3 CV) → 40 % (5 CV) → 40 % (3 CV) → 100 % (2 CV) → 100 % (2 CV)
(7) Condition 3 for Automated Flash Column Chromatography on COMBIFLASH^{™} NextGen 300+ (Teledyne ISCO, Lincoln, NE)
   - Elution Solvent A: n-pentane (Pen)
   - Elution Solvent B: diethylether (Et₂O)
   - Column: FLASHPURE^{™} EXOFLEX^{™} Silica, 24 g, 40 µm irregular, 60 Å pore size, preparative column (Büchi, Switzerland)
   - Elution Condition: Elution for 16 Column Volumes (CV) while varying the concentration of Solvent B in the following pattern: 0 % (3 CV) → 50 % (5 CV) → 50 % (5 CV) → 100 % (1 CV) → 100 % (2 CV)
(8) Condition 4a for Automated Flash Column Chromatography on COMBIFLASH^{™} NextGen 300+ (Teledyne ISCO, Lincoln, NE)
   - Elution Solvent A: dichloromethane (DCM)
   - Elution Solvent B: methanol (CH₃OH)
   - Column: FLASHPURE^{™} EXOFLEX^{™} Silica, 24 g, 40 µm irregular, 60 Å pore size, preparative column (Büchi, Switzerland)
   - Elution Condition: Elution for 15 Column Volumes (CV) at a flow rate of 32 mL/min while varying the concentration of Solvent B in the following pattern: 0 % (3 CV) → 20 % (5 CV) → 20 % (1 CV) → 100 % (3 CV) → 100 % (3 CV)
(9) Condition 4b for Automated Flash Column Chromatography on COMBIFLASH^{™} NextGen 300+ (Teledyne ISCO, Lincoln, NE)
   - Elution Solvent A: dichloromethane (DCM)
   - Elution Solvent B: methanol (CH₃OH)
   - Column: FLASHPURE^{™} EXOFLEX^{™} Silica, 24 g, 40 µm irregular, 60 Å pore size, preparative column (Büchi, Switzerland)
   - Elution Condition: Elution for 15 Column Volumes (CV) at a flow rate of 32 mL/min while varying the concentration of Solvent B in the following pattern: 0 % (3 CV) → 10 % (5 CV) → 10 % (2 CV) → 100 % (2 CV) → 100 % (3 CV)
(10)Condition 5 for Automated Flash Column Chromatography on COMBIFLASH^{™} NextGen 300+ (Teledyne ISCO, Lincoln, NE)
   - Elution Solvent A: 0.1 % trifluoroacetic acid (TFA)/water
   - Elution Solvent B: 0.1 % trifluoroacetic acid (TFA)/CH₃CN
   - Column: FLASHPURE^{™} EXOFLEX^{™} C18, 20 g, 50 µm spherical, 100 Å pore diameter, preparative column (Büchi, Switzerland)
   - Elution Condition: Elution for 13 Column Volumes (CV) at a flow rate of 40 mL/min while varying the concentration of Solvent B in the following pattern: 10 % (3 CV) → 55 % (3 CV) → 30 % (4 CV) → 100 % (1 CV) → 100 % (2 CV)

### Preparation Example 3a.1: Preparation of (S)-5-(3-fluorophenyl)-N-(piperidin-3-yl)-3-ureidothiophene-2-carboxamide

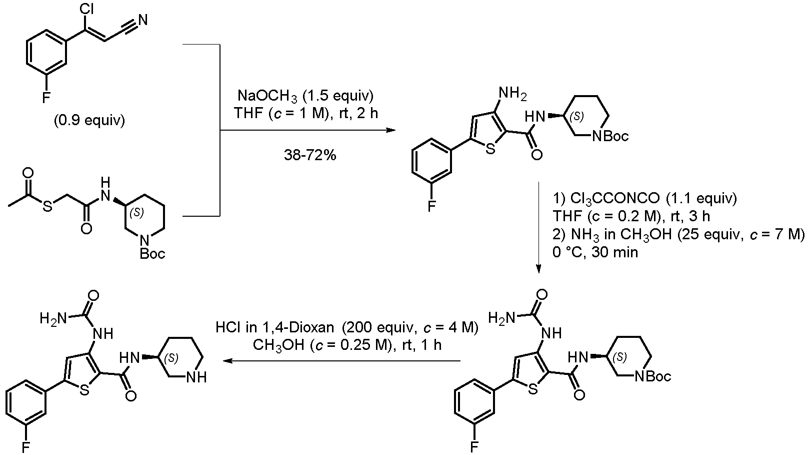

### Step 1. Preparation of tert-butyl (S)-3-(3-amino-5-(3-fluorophenyl)thiophene-2-carboxamido)piperidine-1-carboxylate

To an oven-dried one-necked round bottom flask a solution of (S)-3-(2-(acetylthio)acetamido) piperidine-1-carboxylate (C₁₄H₂₄N₂O₄S, 316.42 g/mol, 800 mg, 2.5 mmol, 1 equiv.) in tetrahydrofuran (THF, C₄H₈O, *c* = 1 M, 2.5 mL) was added under an argon stream at room temperature. After this, solid 3-chloro-3-(3-fluorophenyl)acrylonitrile (C₉H₅ClFN, 181.60 g/mol, 413.2 mg, 2.3 mmol, 0.9 equiv.) was added in one portion before dropwise addition of a sodium methoxide solution (NaOCH₃, 54.02 g/mol, w = 30% in CH₃OH, 0.97 g/mL, 682.9 mg, 0.7 mL, 3.8 mmol, 1.5 equiv.). Upon completion of addition the reaction mixture turned into a dark red solution, which was stirred at rt for 2 h. When TLC indicated full consumption of all starting materials, the solvent was removed under reduced pressure and the residue was purified by Condition 2a for Automated Flash Column Chromatography using the COMBIFLASH^{™} NextGen 300+ (Teledyne ISCO, Lincoln, NE). The obtained product *tert*-butyl (*S*)-3-(3-amino-5-(3-fluorophenyl)thiophene-2-carboxamido)piperidine-1-carboxylate (C₂₁H₂₆FN₃O₃S, 419.52 g/mol, 668 mg, 1.6 mmol, 63 %) was isolated as a brown solid with good yield and sufficient purity for the next step.

**HPLC-MS (ESI-MS):** Calculated: 419.17 for C₂₁H₂₆FN₃O₃S [M+H⁺]. Found: 420.1. ***t*_{R}** = 7.9 min (HPLC Condition 1).

### Step 2. Preparation of tert-butyl (S)-3-(5-(3-fluorophenyl)-3-ureidothiophene-2-carboxamido)piperidine-1-carboxylate

To an oven-dried one-necked round bottom flask a solution of (*S*)-3-(3-amino-5-(3-fluorophenyl)thiophene-2-carboxamido)piperidine-1-carboxylate (C₂₁H₂₆FN₃O₃S, 419.52 g/mol, 465 mg, 1.1 mmol, 1 equiv.) in tetrahydrofuran (THF, C₄H₈O, c = 0.2 M, 5.5 mL) was added under an argon stream at room temperature. After this, trichloroacetyl isocyanate (TCANCO, C₃Cl₃NO₂, 188.40 g/mol, 1.2 g/mL, 229.7 mg, 146 µL, 1.2 mmol, 1.1 equiv.) was added dropwise, maintaining the temperature between 20 °C to 30 °C. The solution was then stirred at rt for 3 hours, whereas after 20 minutes the solution turned into a suspension, and a white precipitate formed. After complete consumption of the starting material, indicated by TLC, the solvent was removed under reduced pressure and the resulting precipitate was cooled to 0 °C before being taken into a methanolic ammonia solution (NH₃, 17.03 g/mol, c = 7 M in CH₃OH, 469.2 mg, 5.5 mL, 27.6 mmol, 25 equiv.). The resulting solution was stirred for 30 minutes, and the solvent was removed under reduced pressure. After purification of the crude product by Condition 2b for Automated Flash Column Chromatography using the COMBIFLASH^{™} NextGen 300+ (Teledyne ISCO, Lincoln, NE) (S)-3-(5-(3-fluorophenyl)-3-ureidothiophene-2-carboxamido)piperidine-1-carboxylate (C₂₂H₂₇FN₄O₄S, 462.54 g/mol, 498.2 mg, 1 mmol, 98 %) could be obtained in high yield over two steps.

**HPLC-MS (ESI-MS):** Calculated: 462.17 for C₂₂H₂₇FN₄O₄S [M+H⁺], Found: 463.2. ***t*_{R}** = 6.2 min (HPLC Condition 1).

### Step 3. Preparation of (S)-5-(3-fluorophenyl)-N-(piperidin-3-yl)-3-ureidothiophene-2-carboxamide

To an oven-dried one-necked round bottom flask a solution of (*S*)-3-(5-(3-fluorophenyl)-3-ureidothiophene-2-carboxamido)piperidine-1-carboxylate (C₂₂H₂₇FN₄O₄S, 462.54 g/mol, 320 mg, 0.7 mmol, 1 equiv.) in anhydrous methanol (CH₃OH, c = 0.25 M, 2.8 mL) was added under an argon stream at 0 °C. After this, a solution of hydrochloric acid in 1,4-dioxane (HCl, 36.46 g/mol, 1.05 g/mL, c = 4 M, 5 g, 4.8 mL, 138.4 mmol, 200 equiv.) was added dropwise, maintaining the temperature between 0 °C to 5 °C. The solution was then stirred at rt for 1 h, until completion of conversion (TLC control). The solvent was then removed under reduced pressure and the remaining solid was purified by Condition 5 for Automated Flash Column Chromatography using the COMBIFLASH^{™} NextGen 300+ (Teledyne ISCO, Lincoln, NE) to obtain (S)-5-(3-fluorophenyl)-*N*-(piperidin-3-yl)-3-ureidothiophene-2-carboxamide (C₁₇H₁₉FN₄O₂S, 362.42 g/mol, 250 mg, 0.7 mmol, 100 %) as a crystalline white-yellow powder in quantitative yield and high purity.

**¹H NMR** (600 MHz, DMSO-d₆) δ = 10.00 (br. s., 1H), 8.28 (s, 1H), 7.95 (d, *J* = 7.77 Hz, 1H), 7.41 - 7.54 (m, 3H), 7.24 (appt, *J* = 8.20 Hz, 1H), 6.66 (br. s., 2H), 3.88 - 4.00 (m, 1H), 3.06 (dd, J = 2.88, 11.65 Hz, 1H), 2.93 (appd, *J* = 12.44 Hz, 1H), 2.62 (t, *J* = 10.81 Hz, 1H), 2.56 (appt, *J* = 10.33 Hz, 1H), 1.84 (appd, *J* = 9.81 Hz, 1H), 1.71 (dd, *J* = 3.56, 9.47 Hz, 1H), 1.46 - 1.61 (m, 2H).

**HPLC-MS (ESI-MS):** Calculated: 362.12 for C₁₇H₁₉FN₄O₂S [M+H⁺], Found: 363.2. ***t*_{R}** = 4.7 min (HPLC Condition 1).

### Preparation Example 3a.2: Preparation of (R)-5-(3-fluorophenyl)-N-(piperidin-3-yl)-3-ureidothiophene-2-carboxamide

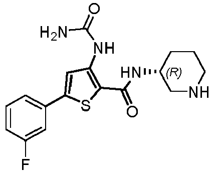

A target compound was obtained by repeating the same procedure as in Preparation Method 3a, except that in Step 1 of Preparation Example 3a.1, *tert*-butyl (*R*)-3-(2-(acetylthio)acetamido) piperidine-1-carboxylate (C₁₄H₂₄N₂O₄S, 316.42 g/mol, 120 mg, 0.38 mmol, 1 equiv.) was used instead of *tert*-butyl (*S*)-3-(2-(acetylthio)acetamido) piperidine-1-carboxylat. The intermediate *tert*-butyl (*R*)-3-(3-amino-5-(3-fluorophenyl)thiophene-2-carboxamido)piperidine-1-carboxylate (C₂₁H₂₆FN₃O₃S, 419.52 g/mol, 45 mg, 0.11 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*R*)-3-(5-(3-fluorophenyl)-3-ureidothiophene-2-carboxamido)piperidine-1-carboxylate (C₂₂H₂₇FN₄O₄S, 462.54 g/mol, 30 mg, 0.06 mmol, 1 equiv.) of Step 2 was purified by Condition 2b for Automated Flash Column Chromatography on COMBIFLASH^{™} NextGen 300+. The final product (R)-5-(3-fluorophenyl)-*N*-(piperidin-3-yl)-3-ureidothiophene-2-carboxamide (C₁₇H₁₉FN₄O₂S, 362.42 g/mol, 20.3 mg, 0.06 mmol, 15 % (over 3 steps)) was obtained as a yellow, flaky powder with high purity and low yield over 3 steps after purification by Condition 5 for Automated Flash Column Chromatography on COMBIFLASH^{™} NextGen 300+.

**¹H NMR** (600 MHz, DMSO-d₆) δ = 10.00 (br. s., 1H), 8.28 (s, 1H), 7.95 (d, *J* = 7.77 Hz, 1H), 7.41 - 7.54 (m, 3H), 7.24 (appt, *J* = 8.20 Hz, 1H), 6.66 (br. s., 2H), 3.88 - 4.00 (m, 1H), 3.06 (dd, *J* = 2.88, 11.65 Hz, 1H), 2.93 (appd, *J* = 12.44 Hz, 1H), 2.62 (t, *J* = 10.81 Hz, 1H), 2.56 (appt, *J* = 10.33 Hz, 1H), 1.84 (appd, *J* = 9.81 Hz, 1H), 1.71 (dd, *J* = 3.56, 9.47 Hz, 1H), 1.46 - 1.61 (m, 2H).

**HPLC-MS (ESI-MS):** Calculated: 362.12 for C₁₇H₁₉FN₄O₂S [M+H⁺], Found: 363.2. ***t*_{R}** = 4.8 min (HPLC Condition 1).

### Preparation Example 3a.3: Preparation of (S)-5-(3-bromophenyl)-N-(piperidin-3-yl)-3-ureidothiophene-2-carboxamide

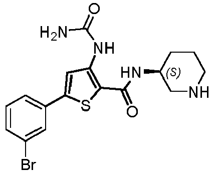

A target compound was obtained by repeating the same procedure as in Preparation Method 3a, except that in Step 1 of Preparation Example 3a.1, 3-(3-bromophenyl)-3-chloroacrylonitrile (C₉H₅BrClN, 242.50 g/mol, 4467.9 mg, 18.42 mmol, 0.9 equiv.) was used instead of 3-(3-fluorophenyl)-3-chloroacrylonitrile. The intermediate *tert*-butyl (*S*)-3-(3-amino-5-(3-bromophenyl)thiophene-2-carboxamido)piperidine-1-carboxylate (C₂₁H₂₆BrN₃O₃S, 480.42 g/mol, 1500 mg, 3.12 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(3-bromophenyl)-3-ureidothiophene-2-carboxamido)piperidine-1-carboxylate (C₂₂H₂₇BrN₄O₄S, 480.42 g/mol, 1170.6 mg, 2.43 mmol, 1 equiv.) of Step 2 was purified by Condition 2b for Automated Flash Column Chromatography on COMBIFLASH^{™} NextGen 300+. The final product (*S*)-5-(3-bromophenyl)-*N*-(piperidin-3-yl)-3-ureidothiophene-2-carboxamide (C₁₇H₁₉BrN₄O₂S, 423.33 g/mol, 724.10 mg, 1.71 mmol, 8 % (over 3 steps)) was obtained as a white solid with high purity and low yield over 3 steps after purification by Condition 5 for Automated Flash Column Chromatography on COMBIFLASH^{™} NextGen 300+.

**¹H NMR** (500 MHz, DMSO-d₆) δ = 10.03 (s, 1H), 8.28 (s, 1H), 7.77 - 7.83 (m, 2H), 7.64 (td, *J* = 0.80, 7.80 Hz, 1H), 7.59 (ddd, *J* = 0.76, 1.83, 8.10 Hz, 1H), 7.43 (appt, *J* = 7.90 Hz, 1H), 6.67 (br. s., 2H), 3.80 (apptq, *J* = 4.40, 8.40 Hz, 1H), 2.94 (dd, *J* = 3.20, 11.60 Hz, 1H), 2.79 (td, *J* = 3.20, 12.05 Hz, 1H), 2.43 (dquin, J = 1.50, 11.00 Hz, 2H), 1.81 (td, *J* = 3.20, 8.85 Hz, 1H), 1.62 (td, *J* = 4.10, 12.80 Hz, 1H), 1.50 (dq, *J* = 4.10, 11.00 Hz, 1H), 1.41 (tt, *J* = 4.00, 11.10 Hz, 1H).

**HPLC-MS (ESI-MS):** Calculated: 422.04 for C₁₇H₁₉BrN₄O₂S [M+H⁺], Found: [⁸¹Br-M+H⁺] 425.1; [⁷⁹Br-M+H⁺] 423.1. ***t*_{R}** = 0.8 min (HPLC Condition 1).

### Preparation Example 3a.4: Preparation of (S)-5-(3,4-difluorophenyl)-N-(piperidin-3-yl)-3-ureidothiophene-2-carboxamide

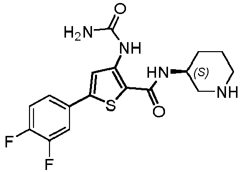

A target compound was obtained by repeating the same procedure as in Preparation Method 3a, except that in Step 1 of Preparation Example 3a.1, 3-chloro-3-(3,4-difluorophenyl)-acrylonitrile (C₉H₅ClF₂N, 199.59 g/mol, 473.1 mg, 2.37 mmol, 1 equiv.) was used instead of 3-(3-fluorophenyl)-3-chloroacrylonitrile. The intermediate *tert*-butyl (*S*)-3-(3-amino-5-(3,4-difluorophenyl)thiophene-2-carboxamido)piperidine-1-carboxylate (C₂₁H₂₅F₂N₃O₃S, 437.51 g/mol, 800 mg, 1.69 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(3,4-difluorophenyl)-3-ureidothiophene-2-carboxamido)piperidine-1-carboxylate (C₂₂H₂₆F₂N₄O₄S, 480.54 g/mol, 664.7 mg, 1.38 mmol, 1 equiv.) of Step 2 was purified by Condition 2b for Automated Flash Column Chromatography on COMBIFLASH^{™} NextGen 300+. The final product (*S*)-5-(3,4-difluorophenyl)-*N*-(piperidin-3-yl)-3-ureidothiophene-2-carboxamide (C₁₇H₁₈F₂N₄O₂S, 380.41 g/mol, 524.2 mg, 1.38 mmol, 58 % (over 3 steps)) was obtained as a yellow solid with high purity and moderate yield over 3 steps after purification by Condition 5 for Automated Flash Column Chromatography on COMBIFLASH^{™} NextGen 300+.

**¹H NMR** (400 MHz, DMSO-d₆) δ = 10.01 (s, 1H), 8.23 (s, 1H), 7.79 (d, *J* = 7.95 Hz, 1H), 7.71 (appddd, *J* = 2.14, 7.64, 11.62 Hz, 1H), 7.44 - 7.58 (m, 2H), 6.64 (br. s., 2H), 3.80 (apptq, *J* = 4.40, 8.40 Hz, 1H), 2.94 (dd, *J* = 3.06, 11.62 Hz, 1H), 2.79 (td, *J* = 4.00, 12.23 Hz, 1H), 2.38 - 2.48 (m, 2H), 1.81 (td, *J* = 3.20, 8.90 Hz, 1H), 1.63 (td, *J* = 4.10, 12.80 Hz, 1H), 1.51 (dq, *J* = 4.10, 11.00 Hz, 1H), 1.35 - 1.46 (m, 1H).

**HPLC-MS (ESI-MS):** Calculated: 380.11 for C₁₇H₁₈F₂N₄O₂S [M+H⁺], Found: 381.1. ***t*_{R}** = 4.9 min (HPLC Condition 1).

### Preparation Example 3a.5 Preparation of (R)-5-(3, 4-difluorophenyl)-N-(piperidin-3-yl)-3-ureidothiophene-2-carboxamide

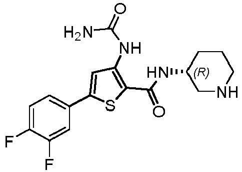

A target compound was obtained by repeating the same procedure as in Preparation Method 3a, except that in Step 1 of Preparation Example 3a.1, 3-chloro-3-(3,4-difluorophenyl)acrylonitrile (C₉H₅ClF₂N, 199.59 g/mol, 473.1 mg, 2.37 mmol, 1 equiv.) and *tert*-butyl (*R*)-3-(2-(acetylthio)acetamido) piperidine-1-carboxylate (C₁₄H₂₄N₂O₄S, 316.42 g/mol, 750 mg, 2.37 mmol, 1 equiv.) was used instead of 3-(3-fluorophenyl)-3-chloroacrylonitrile and *tert*-butyl (*S*)-3-(2-(acetylthio)acetamido) piperidine-1-carboxylate. The intermediate *tert*-butyl (*R*)-3-(3-amino-5-(3,4-difluorophenyl)thiophene-2-carboxamido)piperidine-1-carboxylate (C₂₁H₂₅F₂N₃O₃S, 437.51 g/mol, 500 mg, 1.14 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*R*)-3-(5-(3,4-difluorophenyl)-3-ureidothiophene-2-carboxamido)piperidine-1-carboxylate (C₂₂H₂₆F₂N₄O₄S, 480.54 g/mol, 660.8 mg, 1.37 mmol, 1 equiv.) of Step 2 was purified by Condition 2b for Automated Flash Column Chromatography on COMBIFLASH^{™} NextGen 300+. The final product (*R*)-5-(3,4-difluorophenyl)-*N*-(piperidin-3-yl)-3-ureidothiophene-2-carboxamide (C₁₇H₁₈F₂N₄O₂S, 380.41 g/mol, 520.8 mg, 1.37 mmol, 58 % (over 3 steps)) was obtained as a yellow solid with high purity and moderate yield over 3 steps after purification by Condition 5 for Automated Flash Column Chromatography on COMBIFLASH^{™} NextGen 300+.

**¹H NMR** (400 MHz, DMSO-d₆) δ = 10.01 (s, 1H), 8.23 (s, 1H), 7.79 (d, *J* = 7.95 Hz, 1H), 7.71 (appddd, *J* = 2.14, 7.64, 11.62 Hz, 1H), 7.44 - 7.58 (m, 2H), 6.64 (br. s., 2H), 3.80 (apptq, *J* = 4.40, 8.40 Hz, 1H), 2.94 (dd, *J* = 3.06, 11.62 Hz, 1H), 2.79 (td, *J* = 4.00, 12.23 Hz, 1H), 2.38 - 2.48 (m, 2H), 1.81 (td, *J* = 3.20, 8.90 Hz, 1H), 1.63 (td, *J* = 4.10, 12.80 Hz, 1H), 1.51 (dq, *J* = 4.10, 11.00 Hz, 1H), 1.35 - 1.46 (m, 1H).

**HPLC-MS (ESI-MS):** Calculated: 380.11 for C₁₇H₁₈F₂N₄O₂S [M+H⁺], Found: 381.1. ***t*_{R}** = 4.9 min (HPLC Condition 1).

### Preparation Example 3a.6: Preparation of (S)-5-(2,4-difluorophenyl)-N-(piperidin-3-yl)-3-ureidothiophene-2-carboxamide

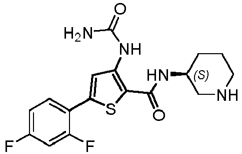

A target compound was obtained by repeating the same procedure as in Preparation Method 3a, except that in Step 1 of Preparation Example 3a.1, 3-chloro-3-(2,4-difluorophenyl)-acrylonitrile (C₉H₅ClF₂N, 199.59 g/mol, 473.1 mg, 2.37 mmol, 1 equiv.) was used instead of 3-(3-fluorophenyl)-3-chloroacrylonitrile. The intermediate *tert*-butyl (*S*)-3-(3-amino-5-(2,4-difluorophenyl)thiophene-2-carboxamido)piperidine-1-carboxylate (C₂₁H₂₅F₂N₃O₃S, 437.51 g/mol, 740 mg, 1.69 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(2,4-difluorophenyl)-3-ureidothiophene-2-carboxamido)piperidine-1-carboxylate (C₂₂H₂₆F₂N₄O₄S, 480.54 g/mol, 415.2 mg, 0.86 mmol, 1 equiv.) of Step 2 was purified by Condition 2b for Automated Flash Column Chromatography on COMBIFLASH^{™} NextGen 300+. The final product (*S*)-5-(2,4-difluorophenyl)-*N*-(piperidin-3-yl)-3-ureidothiophene-2-carboxamide (C₁₇H₁₈F₂N₄O₂S, 380.41 g/mol, 323 mg, 0.85 mmol, 36 % (over 3 steps)) was obtained as a crystalline yellow powder with high purity and moderate yield over 3 steps after purification by Condition 5 for Automated Flash Column Chromatography on COMBIFLASH^{™} NextGen 300+.

**¹H NMR** (400 MHz, DMSO-d₆) δ = 10.04 (s, 1H), 8.31 (s, 1H), 7.74 - 7.87 (m, 2H), 7.45 (appddd, *J* = 2.69, 9.14, 11.77 Hz, 1H), 7.22 (appddt, *J* = 0.70, 2.70, 8.50 Hz, 1H), 6.63 (br. s., 2H), 3.80 (apptq, *J* = 4.40, 8.90 Hz, 1H), 2.94 (dd, *J* = 3.24, 11.68 Hz, 1H), 2.79 (td, *J* = 4.00, 12.20 Hz, 1H), 2.43 (dquin, *J* = 1.90, 11.00 Hz, 2H), 1.81 (td, *J* = 3.20, 8.90 Hz, 1H), 1.61 (td, *J* = 4.10, 12.80 Hz, 1H), 1.51 (dq, *J* = 4.10, 11.00 Hz, 1H), 1.40 (tt, *J* = 4.00, 11.10 Hz, 1H).

**HPLC-MS (ESI-MS):** Calculated: 380.11 for C₁₇H₁₈F₂N₄O₂S [M+H⁺], Found: 381.2. ***t*_{R}** = 0.8 min (HPLC Condition 1).

### Preparation Example 3a.7: Preparation of (S)-5-(2-fluorophenyl)-N-(piperidin-3-yl)-3-ureidothiophene-2-carboxamide

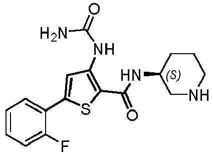

A target compound was obtained by repeating the same procedure as in Preparation Method 3a, except that in Step 1 of Preparation Example 3a.1, 3-chloro-3-(2-fluorophenyl)acrylonitrile (C₉H₅ClFN, 181.60 g/mol, 430.4 mg, 2.37 mmol, 1 equiv.) was used instead of 3-(3-fluorophenyl)-3-chloroacrylonitrile. The intermediate *tert*-butyl (*S*)-3-(3-amino-5-(2,4-difluorophenyl)thiophene-2-carboxamido)piperidine-1-carboxylate (C₂₁H₂₆FN₃O₃S, 419.52 g/mol, 700 mg, 1.67 mmol, 1 equiv.) from Step 1 was purified using Condition 1a for Automated Flash Column Chromatography on COMBIFLASH^{™} NextGen 300+ (Teledyne ISCO, Lincoln, NE), while the intermediate *tert*-butyl (*S*)-3-(5-(2,4-difluorophenyl)-3-ureidothiophene-2-carboxamido)piperidine-1-carboxylate (C₂₂H₂₇FN₄O₄S, 462.54 g/mol, 399.6 mg, 0.86 mmol, 1 equiv.) of Step 2 was purified by Condition 2b for Automated Flash Column Chromatography on COMBIFLASH^{™} NextGen 300+. The final product (*S*)-5-(2-fluorophenyl)-*N*-(piperidin-3-yl)-3-ureidothiophene-2-carboxamide (C₁₇H₁₉FN₄O₂S, 362.42 g/mol, 310.9 mg, 0.86 mmol, 36 % (over 3 steps)) was obtained as a fine crystalline yellow powder with high purity and moderate yield over 3 steps after purification by Condition 5 for Automated Flash Column Chromatography on COMBIFLASH^{™} NextGen 300+.

**¹H NMR** (400 MHz, DMSO-d₆) δ = 9.98 (s, 1H), 8.29 (s, 1H), 7.74 (appd, *J* = 7.95 Hz, 1H), 7.66 (dt, *J* = 1.65, 7.92 Hz, 1H), 7.22 - 7.42 (m, 3H), 6.55 (br. s., 2H), 3.72 (apptq, *J* = 4.40, 8.40 Hz, 1H), 2.86 (dd, *J* = 3.24, 11.68 Hz, 1H), 2.71 (td, *J* = 4.00, 12.20 Hz, 1H), 2.35 (dquin, *J* = 2.20, 11.00 Hz, 2H), 1.74 (td, *J* = 3.20, 8.90 Hz, 1H), 1.54 (td, *J* = 3.50, 12.80 Hz, 1H), 1.44 (dq, *J* = 4.10, 11.00 Hz, 1H), 1.33 (tt, *J* = 4.10, 11.10 Hz, 1H).

**HPLC-MS (ESI-MS):** Calculated: 362.12 for C₁₇H₁₉FN₄O₂S [M+H⁺], Found: 363.1. ***t*_{R}** = 4.6 min (HPLC Condition 1).

### Preparation Example 3a.8: Preparation of (S)-5-(3,5-difluorophenyl)-N-(piperidin-3-yl)-3-ureidothiophene-2-carboxamide

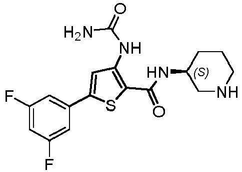

A target compound was obtained by repeating the same procedure as in Preparation Method 3a, except that in Step 1 of Preparation Example 3a.1, 3-chloro-3-(3,5-difluorophenyl)-acrylonitrile (C₉H₅ClF₂N, 199.59 g/mol, 100 mg, 0.50 mmol, 1 equiv.) was used instead of 3-(3-fluorophenyl)-3-chloroacrylonitrile. The intermediate *tert*-butyl (*S*)-3-(3-amino-5-(3,5-difluorophenyl)thiophene-2-carboxamido)piperidine-1-carboxylate (C₂₁H₂₅F₂N₃O₃S, 437.51 g/mol, 160 mg, 0.37 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(3,5-difluorophenyl)-3-ureidothiophene-2-carboxamido)piperidine-1-carboxylate (C₂₂H₂₆F₂N₄O₄S, 480.54 g/mol, 415.2 mg, 0.86 mmol, 1 equiv.) of Step 2 was purified by Condition 2b for Automated Flash Column Chromatography on COMBIFLASH^{™} NextGen 300+. The final product (*S*)-5-(3,5-difluorophenyl)-*N*-(piperidin-3-yl)-3-ureidothiophene-2-carboxamide (C₁₇H₁₈F₂N₄O₂S, 380.41 g/mol, 58.7 mg, 0.15 mmol, 49 % (over 3 steps)) was obtained as a fine crystalline yellow powder with high purity and moderate yield over 3 steps after purification by Condition 5 for Automated Flash Column Chromatography on COMBIFLASH^{™} NextGen 300+.

**¹H NMR** (500 MHz, DMSO-d₆) δ = 9.99 (s, 1H), 8.31 (s, 1H), 7.89 (d, *J* = 7.78 Hz, 1H), 7.27 - 7.41 (m, 3H), 6.63 (br. s., 2H), 3.83 (apptq, *J* = 4.40, 8.40 Hz, 1H), 2.97 (dd, *J* = 3.20, 11.60 Hz, 1H), 2.83 (appd, *J =* 12.05 Hz, 1H), 2.38 - 2.48 (m, 2H), 1.82 (appd, *J* = 10.50 Hz, 1H), 1.65 (td, J= 3.40, 12.21 Hz, 1H), 1.51 (dq, *J=* 4.10, 11.70 Hz, 1H), 1.43 (appt, *J* = 11.60 Hz, 1H).

**HPLC-MS (ESI-MS):** Calculated: 380.11 for C₁₇H₁₈F₂N₄O₂S [M+H⁺], Found: 381.1. ***t*_{R}** = 0.8 min (HPLC Condition 1).

### Preparation Example 3a.9: Preparation of (S)-5-(3-fluorophenyl)-N-(pyrrolidin-3-yl)-3-ureidothiophene-2-carboxamide

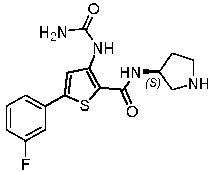

A target compound was obtained by repeating the same procedure as in Preparation Method 3a, except that in Step 1 of Preparation Example 3a.1, *tert*-butyl (*S*)-3-(2-(acetylthio)acetamido)-pyrrolidine-1-carboxylate (C₁₃H₂₂N₂O₄S, 302.39 g/mol, 750 mg, 2.48 mmol, 1 equiv.) was used instead of *tert*-butyl (S)-3-(2-(acetylthio)acetamido) piperidine-1-carboxylate. The intermediate *tert*-butyl (*S*)-3-(3-amino-5-(3-fluorophenyl)thiophene-2-carboxamido)pyrrolidine-1-carboxylate (C₂₆H₂₄FN₃O₃S, 405.49 g/mol, 800 mg, 1.97 mmol, 1 equiv) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(3-fluorophenyl)-3-ureidothiophene-2-carboxamido)pyrrolidine-1-carboxylate (C₂₁H₂₅FN₄O₄S, 448.52 g/mol, 387.5 mg, 0.86 mmol, 1 equiv.) of Step 2 was purified by Condition 2b for Automated Flash Column Chromatography on COMBIFLASH^{™} NextGen 300+. The final product (S)-5-(3-fluorophenyl)-*N*-(pyrrolidin-3-yl)-3-ureidothiophene-2-carboxamide (C₁₆H₁₇FN₄O₂S, 348.40 g/mol, 293.1 mg, 0.84 mmol, 34 % (over 3 steps)) was obtained as a yellow dumpy solid with high purity and moderate yield over 3 steps after purification by Condition 5 for Automated Flash Column Chromatography on COMBIFLASH^{™} NextGen 300+.

**¹H NMR** (600 MHz, DMSO-d₆) δ = 10.00 (d, *J* = 13.85 Hz, 1H), 8.24 - 8.34 (m, 2H), 7.51 (appq, *J* = 7.20 Hz, 1H), 7.45 - 7.48 (m, 1H), 7.43 (appd, *J* = 9.95 Hz, 1H), 7.24 (dt, *J* = 2.23, 8.20 Hz, 1H), 6.67 (br. s., 2H), 4.42 - 4.48 (m, 1H), 4.37 (br. s., 1H), 3.14 - 3.25 (m, 2H), 2.96 - 3.08 (m, 2H), 2.10 (appsxt, *J* = 7.40 Hz, 1H), 1.85 - 1.93 (m, 1H).

**HPLC-MS (ESI-MS):** Calculated: 348.11 for C₁₆H₁₇FN₄O₂S [M+H⁺], Found: 349.1; [M+Na⁺] 371.0. ***t*_{R}** = 4.6 min (HPLC Condition 1).

### Preparation Example 3a.10: Preparation of (R)-5-(3-fluorophenyl)-N-(pyrrolidin-3-yl)-3-ureidothiophene-2-carboxamide

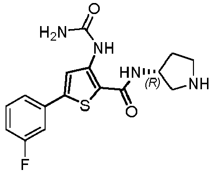

A target compound was obtained by repeating the same procedure as in Preparation Method 3a, except that in Step 1 of Preparation Example 3a.1, *tert*-butyl (*R*)-3-(2-(acetylthio)acetamido)-pyrrolidine-1-carboxylate (C₁₃H₂₂N₂O₄S, 302.39 g/mol, 750 mg, 2.48 mmol, 1 equiv.) was used instead of *tert*-butyl (*S*)-3-(2-(acetylthio)acetamido) piperidine-1-carboxylate. The intermediate *tert*-butyl (*R*)-3-(3-amino-5-(3-fluorophenyl)thiophene-2-carboxamido)pyrrolidine-1-carboxylate (C₂₆H₂₄FN₃O₃S, 405.49 g/mol, 789.1 mg, 1.95 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*R*)-3-(5-(3-fluorophenyl)-3-ureidothiophene-2-carboxamido)pyrrolidine-1-carboxylate (C₂₁H₂₅FN₄O₄S, 448.52 g/mol, 377.9 mg, 0.84 mmol, 1 equiv.) of Step 2 was purified by Condition 2b for Automated Flash Column Chromatography on COMBIFLASH^{™} NextGen 300+. The final product (*R*)-5-(3-fluorophenyl)-*N*-(pyrrolidin-3-yl)-3-ureidothiophene-2-carboxamide (C₁₆H₁₇FN₄O₂S, 348.40 g/mol, 290.8 mg, 0.83 mmol, 34 % (over 3 steps)) was obtained as a yellow dumpy solid with high purity and moderate yield over 3 steps after purification by Condition 5 for Automated Flash Column Chromatography on COMBIFLASH^{™} NextGen 300+.

**¹H NMR** (600 MHz, DMSO-d₆) δ = 10.00 (d, *J* = 13.85 Hz, 1H), 8.24 - 8.34 (m, 2H), 7.51 (appq, *J* = 7.20 Hz, 1H), 7.45 - 7.48 (m, 1H), 7.43 (appd, *J* = 9.95 Hz, 1H), 7.24 (dt, *J* = 2.23, 8.20 Hz, 1H), 6.67 (br. s., 2H), 4.42 - 4.48 (m, 1H), 4.37 (br. s., 1H), 3.14 - 3.25 (m, 2H), 2.96 - 3.08 (m, 2H), 2.10 (appsxt, *J* = 7.40 Hz, 1H), 1.85 - 1.93 (m, 1H).

**HPLC-MS (ESI-MS):** Calculated: 348.11 for C₁₆H₁₇FN₄O₂S [M+H⁺], Found: 349.1; [M+Na⁺] 371.0. ***t*_{R}** = 4.6 min (HPLC Condition 1).

### Preparation Example 3a.11: Preparation of (R)-N-(azepan-3-yl)-5-(3-fluorophenyl)-3-ureidothiophene-2-carboxamide

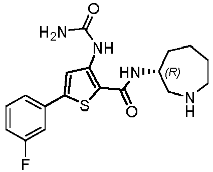

A target compound was obtained by repeating the same procedure as in Preparation Method 3a, except that in Step 1 of Preparation Example 3a.1 *tert*-butyl (*R*)-3-(2-(acetylthio)acetamido)-azepane-1-carboxylate (C₁₅H₂₆N₂O₄S, 330.45 g/mol, 750 mg, 2.27 mmol, 1 equiv.) was used instead of tert-butyl (S)-3-(2-(acetylthio)acetamido) piperidine-1-carboxylate. The intermediate *tert*-butyl (*R*)-3-(3-amino-5-(3-fluorophenyl)thiophene-2-carboxamido)azepane-1-carboxylate (C₂₂H₂₈FN₃O₃S, 433.55 g/mol, 600 mg, 1.38 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*R*)-3-(5-(3-fluorophenyl)-3-ureidothiophene-2-carboxamido)azepane-1-carboxylate (C₂₃H₂₉FN₄O₄S, 476.57 g/mol, 411.7 mg, 0.86 mmol, 1 equiv.) of Step 2 was purified by Condition 2b for Automated Flash Column Chromatography on COMBIFLASH^{™} NextGen 300+. The final product (R)-N-(azepan-3-yl)-5-(3-fluorophenyl)-3-ureidothiophene-2-carboxamide (C₁₈H₂₁FN₄O₂S, 376.45 g/mol, 323.5 mg, 0.86 mmol, 38 % (over 3 steps)) was obtained as a white-yellow clumpy solid with high purity and moderate yield over 3 steps after purification by Condition 5 for Automated Flash Column Chromatography on COMBIFLASH^{™} NextGen 300+.

**¹H NMR** (400 MHz, DMSO-d₆) δ = 10.03 (br. s., 1H), 8.28 (s, 1H), 7.78 (d, *J* = 7.58 Hz, 1H), 7.38 - 7.59 (m, 3H), 7.23 (ddt, *J* = 1.50, 2.10, 8.10 Hz, 1H), 6.64 (br. s., 2H), 3.85 - 4.09 (m, 1H), 2.92 (dd, *J* = 4.28, 13.57 Hz, 1H), 2.62 - 2.83 (m, 3H), 1.74 - 1.85 (m, 1H), 1.42 - 1.73 (m, 5H).

**HPLC-MS (ESI-MS):** Calculated: 376.14 for C₁₈H₂₁FN₄O₂S [M+H⁺]. Found: 377.1. ***t*_{R}** = 4.9 min (HPLC Condition 1).

### Preparation Example 3a:12: Preparation of (R)-5-(3,5-difluorophenyl)-N-(piperidin-3-yl)-3-ureidothiophene-2-carboxamide

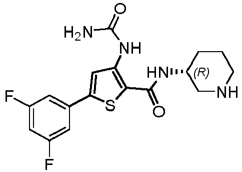

A target compound was obtained by repeating the same procedure as in Preparation Method 3a, except that in Step 1 of Preparation Example 3a.1, 3-chloro-3-(3,5-difluorophenyl)-acrylonitrile (C₉H₅ClF₂N, 199.59 g/mol, 94 mg, 0.47 mmol, 0.9 equiv.) and *tert*-butyl (*R*)-3-(2-(acetylthio)acetamido) piperidine-1-carboxylate (C₁₄H₂₄N₂O₄S, 316.42 g/mol, 165.6 mg, 0.52 mmol, 1 equiv.) was used instead of 3-(3-fluorophenyl)-3-chloroacrylonitrile and tert-butyl (*S*)-3-(2-(acetylthio)acetamido) piperidine-1-carboxylate. The intermediate *tert*-butyl (*R*)-3-(3-amino-5-(3,5-difluorophenyl)thiophene-2-carboxamido)piperidine-1-carboxylate (C₂₁H₂₅F₂N₃O₃S, 437.51 g/mol, 174 mg, 0.40 mmol, 1 equiv) from Step 1 was purified using Condition 2a for Automated Flash Column Chromatography on COMBIFLASH^{™} NextGen 300+ (Teledyne ISCO, Lincoln, NE), while the intermediate *tert*-butyl (*R*)-3-(5-(3,5-difluorophenyl)-3-ureidothiophene-2-carboxamido)piperidine-1-carboxylate (C₂₂H₂₆F₂N₄O₄S, 480.54 g/mol, 105.6 mg, 0.22 mmol, 1 equiv.) of Step 2 was purified by Condition 2b for Automated Flash Column Chromatography on COMBIFLASH^{™} NextGen 300+. The final product (*R*)-5-(3,5-difluorophenyl)-*N*-(piperidin-3-yl)-3-ureidothiophene-2-carboxamide (C₁₇H₁₈F₂N₄O₂S, 380.41 g/mol, 82.7 mg, 0.22 mmol, 42 % (over 3 steps)) was obtained as a white-yellow solid with high purity and moderate yield over 3 steps after purification by Condition 5 for Automated Flash Column Chromatography on COMBIFLASH^{™} NextGen 300+.

**¹H NMR** (500 MHz, DMSO-d₆) δ = 9.99 (s, 1H), 8.31 (s, 1H), 7.89 (d, *J* = 7.78 Hz, 1H), 7.27 - 7.41 (m, 3H), 6.63 (br. s., 2H), 3.83 (apptq, *J* = 4.40, 8.40 Hz, 1H), 2.97 (dd, *J* = 3.20, 11.60 Hz, 1H), 2.83 (appd, J = 12.05 Hz, 1H), 2.38 - 2.48 (m, 2H), 1.82 (appd, *J* = 10.50 Hz, 1H), 1.65 (td, *J* = 3.40, 12.21 Hz, 1H), 1.51 (dq, *J* = 4.10, 11.70 Hz, 1H), 1.43 (appt, *J* = 11.60 Hz, 1H).

**HPLC-MS (ESI-MS):** Calculated: 380.11 for C₁₇H₁₈F₂N₄O₂S [M+H⁺], Found: 381.1. ***t*_{R}** = 4.9 min (HPLC Condition 1).

### Preparation Example 3a.13: Preparation of (R)-5-(2-fluorophenyl)-N-(piperidin-3-yl)-3-ureidothiophene-2-carboxamide

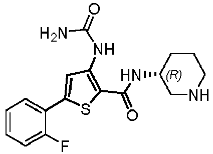

A target compound was obtained by repeating the same procedure as in Preparation Method 3a, except that in Step 1 of Preparation Example 3a.1, 3-chloro-3-(2-fluorophenyl)acrylonitrile (C₉H₅ClFN, 181.60 g/mol, 631.3 mg, 3.48 mmol, 1.1 equiv.) and *tert*-butyl (*R*)-3-(2-(acetylthio)acetamido) piperidine-1-carboxylate (C₁₄H₂₄N₂O₄S, 316.42 g/mol, 1000 mg, 3.16 mmol, 1 equiv.) was used instead of 3-(3-fluorophenyl)-3-chloroacrylonitrile and *tert*-butyl (*S*)-3-(2-(acetylthio)acetamido) piperidine-1-carboxylate. The intermediate *tert*-butyl (*R*)-3-(3-amino-5-(2,4-difluorophenyl)thiophene-2-carboxamido)piperidine-1-carboxylate (C₂₁H₂₆FN₃O₃S, 419.52 g/mol, 960 mg, 2.29 mmol, 1 equiv.) from Step 1 was purified using Condition 1a for Automated Flash Column Chromatography on COMBIFLASH^{™} NextGen 300+ (Teledyne ISCO, Lincoln, NE), while the intermediate *tert*-butyl (*R*)-3-(5-(2,4-difluorophenyl)-3-ureidothiophene-2-carboxamido)piperidine-1-carboxylate (C₂₂H₂₇FN₄O₄S, 462.54 g/mol, 629.4 mg, 1.36 mmol, 1 equiv.) of Step 2 was purified by Condition 2b for Automated Flash Column Chromatography on COMBIFLASH^{™} NextGen 300+. The final product (*R*)-5-(2-fluorophenyl)-*N*-(piperidin-3-yl)-3-ureidothiophene-2-carboxamide (C₁₇H₁₉FN₄O₂S, 362.42 g/mol, 423.5 mg, 1.17 mmol, 37 % (over 3 steps)) was obtained as a dumpy yellow powder with high purity and moderate yield over 3 steps after purification by Condition 5 for Automated Flash Column Chromatography on COMBIFLASH^{™} NextGen 300+.

**¹H NMR** (400 MHz, DMSO-d₆) δ = 9.98 (s, 1H), 8.29 (s, 1H), 7.74 (appd, *J* = 7.95 Hz, 1H), 7.66 (dt, *J* = 1.65, 7.92 Hz, 1H), 7.22 - 7.42 (m, 3H), 6.55 (br. s., 2H), 3.72 (apptq, *J* = 4.40, 8.40 Hz, 1H), 2.86 (dd, *J* = 3.24, 11.68 Hz, 1H), 2.71 (td, *J* = 4.00, 12.20 Hz, 1H), 2.35 (dquin, *J* = 2.20, 11.00 Hz, 2H), 1.74 (td, *J* = 3.20, 8.90 Hz, 1H), 1.54 (td, *J* = 3.50, 12.80 Hz, 1H), 1.44 (dq, *J =* 4.10, 11.00 Hz, 1H), 1.33 (tt, *J* = 4.10, 11.10 Hz, 1H).

**HPLC-MS (ESI-MS):** Calculated: 362.12 for C₁₇H₁₉FN₄O₂S [M+H⁺], Found: 363.1. ***t*_{R}** = 4.6 min (HPLC Condition 1).

### Preparation Example 3a.14: Preparation of (R)-5-(2,4-difluorophenyl)-N-(piperidin-3-yl)-3-ureidothiophene-2-carboxamide

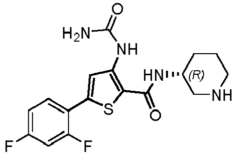

A target compound was obtained by repeating the same procedure as in Preparation Method 3a, except that in Step 1 of Preparation Example 3a.1, 3-chloro-3-(2,4-difluorophenyl)-acrylonitrile (C₉H₅ClF₂N, 199.59 g/mol, 567.7 mg, 2.84 mmol, 1 equiv.) and *tert*-butyl (*R*)-3-(2-(acetylthio)acetamido) piperidine-1-carboxylate (C₁₄H₂₄N₂O₄S, 316.42 g/mol, 1000 mg, 3.16 mmol, 1 equiv.) was used instead of 3-(3-fluorophenyl)-3-chloroacrylonitrile and *tert*-butyl (*S*)-3-(2-(acetylthio)acetamido) piperidine-1-carboxylate. The intermediate *tert*-butyl (*R*)-3-(3-amino-5-(2,4-difluorophenyl)thiophene-2-carboxamido)piperidine-1-carboxylate (C₂₁H₂₅F₂N₃O₃S, 437.51 g/mol, 1174.2 mg, 2.68 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*R*)-3-(5-(2,4-difluorophenyl)-3-ureidothiophene-2-carboxamido)piperidine-1-carboxylate (C₂₂H₂₆F₂N₄O₄S, 480.54 g/mol, 171.3 mg, 0.36 mmol, 1 equiv.) of Step 2 was purified by Condition 2b for Automated Flash Column Chromatography on COMBIFLASH^{™} NextGen 300+. The final product (*R*)-5-(2,4-difluorophenyl)-*N*-(piperidin-3-yl)-3-ureidothiophene-2-carboxamide (C₁₇H₁₈F₂N₄O₂S, 380.41 g/mol, 132.6 mg, 0.35 mmol, 11 % (over 3 steps)) was obtained as a yellow solid with high purity and moderate yield over 3 steps after purification by Condition 5 for Automated Flash Column Chromatography on COMBIFLASH^{™} NextGen 300+.

**¹H NMR** (400 MHz, DMSO-d₆) δ = 10.04 (s, 1H), 8.31 (s, 1H), 7.74 - 7.87 (m, 2H), 7.45 (appddd, *J* = 2.69, 9.14, 11.77 Hz, 1H), 7.22 (appddt, *J* = 0.70, 2.70, 8.50 Hz, 1H), 6.63 (br. s., 2H), 3.80 (apptq, *J* = 4.40, 8.90 Hz, 1H), 2.94 (dd, *J* = 3.24, 11.68 Hz, 1H), 2.79 (td, *J* = 4.00, 12.20 Hz, 1H), 2.43 (dquin, *J* = 1.90, 11.00 Hz, 2H), 1.81 (td, *J* = 3.20, 8.90 Hz, 1H), 1.61 (td, *J=* 4.10, 12.80 Hz, 1H), 1.51 (dq, *J* = 4.10, 11.00 Hz, 1H), 1.40 (tt, *J* = 4.00, 11.10 Hz, 1H).

**HPLC-MS (ESI-MS):** Calculated: 380.11 for C₁₇H₁₈F₂N₄O₂S [M+H⁺], Found: 381.1. ***t*_{R}** = 4.8 min (HPLC Condition 1).

### Preparation Example 3a.15: Preparation of (S)-N-(piperidin-3-yl)-5-(pyridin-3-yl)-3-ureidothiophene-2-carboxamide

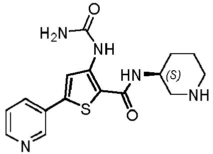

A target compound was obtained by repeating the same procedure as in Preparation Method 3a, except that in Step 1 of Preparation Example 3a.1, 3-chloro-3-(pyridin-3-yl)acrylonitrile (C₈H₅ClN₂, 164.59 g/mol, 304.3 mg, 1.85 mmol, 1 equiv.) was used instead of 3-(3-fluorophenyl)-3-chloroacrylonitrile. The intermediate *tert*-butyl (*S*)-3-(3-amino-5-(pyridin-3-yl)thiophene-2-carboxamido)piperidine-1-carboxylate (C₂₀H₂₆N₄O₃S, 402.52 g/mol, 486.1 mg, 1.21 mmol, 1 equiv.) from Step 1 was purified using Condition 2b for Automated Flash Column Chromatography on COMBIFLASH^{™} NextGen 300+ (Teledyne ISCO, Lincoln, NE), while the intermediate *tert*-butyl (*S*)-3-(5-(pyridin-3-yl)-3-ureidothiophene-2-carboxamido)piperidine-1-carboxylate (C₂₁H₂₇N₅O₄S, 445.54 g/mol, 512.7 mg, 1.15 mmol, 1 equiv.) of Step 2 was purified by Condition 4b for Automated Flash Column Chromatography on COMBIFLASH^{™} NextGen 300+. The final product (*S*)-*N*-(piperidin-3-yl)-5-(pyridin-3-yl)-3-ureidothiophene-2-carboxamide (C₁₆H₁₉N₅O₂S, 345.42 g/mol, 388 mg, 1.12 mmol, 55 % (over 3 steps)) was obtained as a dumpy yellow powder with high purity and moderate yield over 3 steps after purification by Condition 5 for Automated Flash Column Chromatography on COMBIFLASH^{™} NextGen 300+.

**¹H NMR** (600 MHz, DMSO-d₆) δ = 9.96 (s, 1H), 9.57 (app br. s., 2H), 9.09 (app br. s., 1H), 8.79 (d, *J* = 3.22 Hz, 1H), 8.51 (appd, *J* = 7.81 Hz, 1H), 8.41 - 8.47 (m, 2H), 7.92 (t, *J* = 6.20 Hz, 1H), 6.43 - 6.93 (m, 1H), 4.20 - 4.30 (m, 1H), 3.24 (appd, *J* = 9.99 Hz, 1H), 3.14 (appd, *J* = 11.63 Hz, 1H), 2.87 - 2.96 (m, 1H), 2.79 (app br. s., 1H), 1.88 (appd, *J* = 9.58 Hz, 2H), 1.71 - 1.82 (m, 1H), 1.65 (appq, *J=* 10.01 Hz, 1H).

**HPLC-MS (ESI-MS):** Calculated: 345.13 for C₁₆H₁₉N₅O₂S [M+H⁺], Found: 346.2. ***t*_{R}** = 3.1 min (HPLC Condition 1).

### Preparation Example 3a.16: Preparation of 5-(3-fluorophenyl)-N-(piperidin-4-yl)-3-ureidothiophene-2-carboxamide

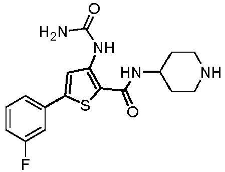

A target compound was obtained by repeating the same procedure as in Preparation Method 3a, except that in Step 1 of Preparation Example 3a.1, *tert*-butyl 4-(2-(acetylthio)acetamido)piperidine-1-carboxylate (C₁₄H₂₄N₂O₄S, 316.42 g/mol, 800 mg, 2.53 mmol, 1 equiv.) was used instead of *tert*-butyl (*S*)-3-(2-(acetylthio)acetamido) piperidine-1-carboxylate. The intermediate *tert*-butyl 4-(3-amino-5-(3-fluorophenyl)thiophene-2-carboxamido)piperidine-1-carboxylate (C₂₁H₂₆FN₃O₃S, 419.52 g/mol, 425.6 mg, 1.01 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl 4-(5-(3-fluorophenyl)-3-ureidothiophene-2-carboxamido)piperidine-1-carboxylate (C₂₂H₂₇FN₄O₄S, 462.54 g/mol, 365.2 mg, 0.79 mmol, 1 equiv.) of Step 2 was purified by Condition 2b for Automated Flash Column Chromatography on COMBIFLASH^{™} NextGen 300+. The final product 5-(3-fluorophenyl)-N-(piperidin-4-yl)-3-ureidothiophene-2-carboxamide (C₁₇H₁₉FN₄O₂S, 362.42 g/mol, 281.3 mg, 0.78 mmol, 31 % (over 3 steps)) was obtained as a yellow solid with high purity and moderate yield over 3 steps after purification by Condition 5 for Automated Flash Column Chromatography on COMBIFLASH^{™} NextGen 300+.

**¹H** NMR (500 MHz, DMSO-d₆) δ = 10.04 (s, 1H), 8.28 (s, 1H), 8.09 (d, *J* = 7.48 Hz, 1H), 7.39 - 7.56 (m, 4H), 7.24 (dt, *J* = 1.80, 8.20 Hz, 1H), 6.64 (br. s., 2H), 3.83 - 3.95 (m, 1H), 3.54 (br. s., 1H), 3.14 (appd, *J* = 12.36 Hz, 2H), 2.72 (t, *J* = 11.60 Hz, 2H), 1.82 (appd, *J* = 10.99 Hz, 2H), 1.61 (appq, *J* = 11.50 Hz, 2H). **HPLC-MS (ESI-MS):** Calculated: 362.12 for C₁₇H₁₉FN₄O₂S [M+H⁺], Found: [M+Na⁺] 385.2; 363.1. ***t*_{R}** = 4.7 min (HPLC Condition 1).

### Preparation Example 3a.17: Preparation of 1-(5-(3-fluorophenyl)-2-(2,6-diazaspiro[3.3]heptane-2-carbonyl)thiophen-3-yl)urea

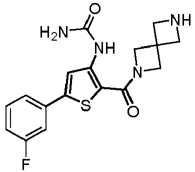

A target compound was obtained by repeating the same procedure as in Preparation Method 3a, except that in Step 1 of Preparation Example 3a.1, *tert*-butyl 6-(2-(acetylthio)acetyl)-2,6-diazaspiro[3.3]heptane-2-carboxylate (C₁₄H₂₂N₂O₄S, 314.41 g/mol, 600 mg, 1.91 mmol, 1 equiv.) was used instead of *tert*-butyl (*S*)-3-(2-(acetylthio)acetamido) piperidine-1-carboxylate. The intermediate *tert*-butyl 6-(3-amino-5-(3-fluorophenyl)thiophene-2-carbonyl)-2,6-diazaspiro[3.3]heptane-2-carboxylate (C₂₁H₂₄FN₃O₃S, 417.50 g/mol, 65.1 mg, 0.16 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl 6-(5-(3-fluorophenyl)-3-ureidothiophene-2-carbonyl)-2,6-diazaspiro[3.3]heptane-2-carboxylate (C₂₂H₂₅FN₄O₄S, 460.53 g/mol, 28.4 mg, 0.06 mmol, 1 equiv.) of Step 2 was purified by Condition 2b for Automated Flash Column Chromatography on COMBIFLASH^{™} NextGen 300+. The final product 1-(5-(3-fluorophenyl)-2-(2,6-diazaspiro[3.3]heptane-2-carbonyl)thiophen-3-yl)urea (C₁₇H₁₇FN₄O₂S, 360.41 g/mol, 16.2 mg, 0.04 mmol, 2 % (over 3 steps)) was obtained as a yellow solid with high purity and low yield over 3 steps after purification by Condition 5 for Automated Flash Column Chromatography on COMBIFLASH^{™} NextGen 300+.

**¹H NMR** (400 MHz, DMSO-d₆) δ = 10.07 (br. s., 1H), 8.27 (s, 1H), 7.96 (appd, *J* = 7.70 Hz, 1H), 7.38 - 7.57 (m, 3H), 7.24 (ddt, *J* = 1.40, 2.20, 8.30 Hz, 3H), 6.63 (br. s., 2H), 3.71 (s, 4H), 3.22 (s, 4H).

**HPLC-MS (ESI-MS):** Calculated: 360.11 for C₁₇H₁₇FN₄O₂S [M+H⁺], Found: 361.2. ***t*_{R}** = 4.9 min (HPLC Condition 1).

### Preparation Example 3a. 18: Preparation of (R)-5-(3-bromophenyl)-N-(piperidin-3-yl)-3-ureidothiophene-2-carboxamide

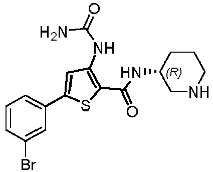

A target compound was obtained by repeating the same procedure as in Preparation Method 3a, except that in Step 1 of Preparation Example 3a.1, 3-(3-bromophenyl)-3-chloroacrylonitrile (C₉H₅BrClN, 242.50 g/mol, 827.7 mg, 3.41 mmol, 0.9 equiv.) and *tert*-butyl (*R*)-3-(2-(acetylthio)acetamido) piperidine-1-carboxylate (C₁₄H₂₄N₂O₄S, 316.42 g/mol, 1200 mg, 3.79 mmol, 1 equiv.) was used instead of 3-(3-fluorophenyl)-3-chloroacrylonitrile and *tert*-butyl (*S*)-3-(2-(acetylthio)acetamido) piperidine-1-carboxylate. The intermediate *tert*-butyl (*R*)-3-(3-amino-5-(3-bromophenyl)thiophene-2-carboxamido)piperidine-1-carboxylate (C₂₁H₂₆SrN₃O₃S, 480.42 g/mol, 1130.4 mg, 2.35 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*R*)-3-(5-(3-bromophenyl)-3-ureidothiophene-2-carboxamido)piperidine-1-carboxylate (C₂₂H₂₇BrN₄O₄S, 480.42 g/mol, 630.1 mg, 1.31 mmol, 1 equiv.) of Step 2 was purified by Condition 2b for Automated Flash Column Chromatography on COMBIFLASH^{™} NextGen 300+. The final product (*R*)-5-(3-bromophenyl)-*N*-(piperidin-3-yl)-3-ureidothiophene-2-carboxamide (C₁₇H₁₉BrN₄O₂S, 423.33 g/mol, 501.9 mg, 1.19 mmol, 31 % (over 3 steps)) was obtained as a yellow solid with high purity and moderate yield over 3 steps after purification by Condition 5 for Automated Flash Column Chromatography on COMBIFLASH^{™} NextGen 300+.

**¹H NMR** (500 MHz, DMSO-d₆) δ = 10.03 (s, 1H), 8.28 (s, 1H), 7.77 - 7.83 (m, 2H), 7.64 (td, *J* = 0.80, 7.80 Hz, 1H), 7.59 (ddd, *J* = 0.76, 1.83, 8.10 Hz, 1H), 7.43 (appt, *J* = 7.90 Hz, 1H), 6.67 (br. s., 2H), 3.75 - 3.86 (m, 1H), 2.94 (dd, *J* = 3.20, 11.60 Hz, 1H), 2.79 (td, J = 3.20, 12.05 Hz, 1H), 2.43 (dquin, *J* = 1.50, 11.00 Hz, 2H), 1.81 (td, *J* = 3.20, 8.85 Hz, 1H), 1.62 (td, *J =* 4.10, 12.80 Hz, 1H), 1.50 (dq, *J* = 4.10, 11.00 Hz, 1H), 1.41 (tt, *J* = 4.00, 11.10 Hz, 1H).

**HPLC-MS (ESI-MS):** Calculated: 422.04 for C₁₇H₁₉SrN₄O₂S [M+H⁺], Found: [⁷⁹Br-M+H⁺] 423.1; [⁸¹Br-M+H⁺] 425.1. ***t*_{R}** = 5.0 min (HPLC Condition 1).

### Preparation Example 3a.19: Preparation of (S)-N-(azepan-3-yl)-5-(3-fluorophenyl)-3-ureidothiophene-2-carboxamide

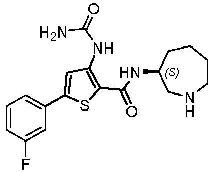

A target compound was obtained by repeating the same procedure as in Preparation Method 3a, except that in Step 1 of Preparation Example 3a.1, *tert*-butyl (*S*)-3-(2-(acetylthio)acetamido)-azepane-1-carboxylate (C₁₅H₂₆N₂O₄S, 330.45 g/mol, 800 mg, 2.42 mmol, 1 equiv.) was used instead of *tert*-butyl (S)-3-(2-(acetylthio)acetamido) piperidine-1-carboxylate. The intermediate *tert*-butyl (*S*)-3-(3-amino-5-(3-fluorophenyl)thiophene-2-carboxamido)azepane-1-carboxylate (C₂₂H₂₈FN₃O₃S, 433.55 g/mol, 192.9 mg, 0.44 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate tert-butyl (*S*)-3-(5-(3-fluorophenyl)-3-ureidothiophene-2-carboxamido)azepane-1-carboxylate (C₂₃H₂₉FN₄O₄S, 476.57 g/mol, 164.6 mg, 0.35 mmol, 1 equiv.) of Step 2 was purified by Condition 2b for Automated Flash Column Chromatography on COMBIFLASH^{™} NextGen 300+. The final product (*S*)-*N-*(azepan-3-yl)-5-(3-fluorophenyl)-3-ureidothiophene-2-carboxamide (C₁₈H₂₁FN₄O₂S, 376.45 g/mol, 125.5 mg, 0.33 mmol, 14 % (over 3 steps)) was obtained as a white-yellow clumpy solid with high purity and low yield over 3 steps after purification by Condition 5 for Automated Flash Column Chromatography on COMBIFLASH^{™} NextGen 300+.

**¹H NMR** (400 MHz, DMSO-d₆) δ = 10.03 (br. s., 1H), 8.28 (s, 1H), 7.78 (d, *J* = 7.58 Hz, 1H), 7.38 - 7.59 (m, 3H), 7.23 (ddt, *J* = 1.50, 2.10, 8.10 Hz, 1H), 6.64 (br. s., 2H), 3.85 - 4.09 (m, 1H), 2.92 (dd, *J* = 4.28, 13.57 Hz, 1H), 2.62 - 2.83 (m, 3H), 1.74 - 1.85 (m, 1H), 1.42 - 1.73 (m, 5H).

**HPLC-MS (ESI-MS):** Calculated: 376.14 for C₁₈H₂₁FN₄O₂S [M+H⁺]. Found: 377.2. ***t*_{R}** = 4.9 min (HPLC Condition 1).

### Preparation Example 3a.20: Preparation of (S)-5-(3-nitrophenyl)-N-(piperidin-3-yl)-3-ureidothiophene-2-carboxamide

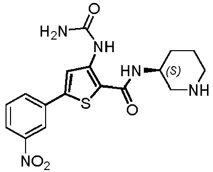

A target compound was obtained by repeating the same procedure as in Preparation Method 3a, except that in Step 1 of Preparation Example 3a.1, 3-chloro-3-(3-nitrophenyl)acrylonitrile (C₉H₅ClN₂O₂, 208.60 g/mol, 469.4 mg, 2.25 mmol, 0.9 equiv.) was used instead of 3-(3-fluorophenyl)-3-chloroacrylonitrile. The intermediate *tert*-butyl (*S*)-3-(3-amino-5-(3-nitrophenyl)thiophene-2-carboxamido)piperidine-1-carboxylate (C₂₁H₂₆FN₄O₅S, 446.53 g/mol, 500 mg, 1.12 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(3-nitrophenyl)-3-ureidothiophene-2-carboxamido)piperidine-1-carboxylate (C₂₂H₂₇FN₅O₆S, 489.55 g/mol, 356.4 mg, 0.73 mmol, 1 equiv.) of Step 2 was purified by Condition 2b for Automated Flash Column Chromatography on COMBIFLASH^{™} NextGen 300+. The final product (*S*)-5-(3-nitrophenyl)-*N*-(piperidin-3-yl)-3-ureidothiophene-2-carboxamide (C₁₇H₁₉N₅O₄S, 389.43 g/mol, 280.1 mg, 0.72 mmol, 29 % (over 3 steps)) was obtained as a flaky yellow powder with high purity and moderate yield over 3 steps after purification by Condition 5 for Automated Flash Column Chromatography on COMBIFLASH^{™} NextGen 300+.

**¹H NMR** (700 MHz, DMSO-d₆) δ = 10.01 (br. s., 1H), 8.42 (s, 1H), 8.35 (t, *J* = 2.04 Hz, 1H), 8.23 (ddd, J = 0.65, 2.30, 8.20 Hz, 1H), 8.11 (ddd, *J* = 0.80, 1.60, 7.74 Hz, 1H), 8.02 (d, *J* = 7.74 Hz, 1H), 7.77 (t, *J* = 8.07 Hz, 1H), 6.71 (br. s., 2H), 3.93 - 4.00 (m, 2H), 3.12 (appdd, *J* = 3.55, 11.94 Hz, 1H), 2.99 (td, *J* = 3.20, 12.69 Hz, 1H), 2.66 (dd, *J* = 10.33, 11.62 Hz, 1H), 2.61 (appt, *J* = 11.20 Hz, 1H), 1.83 - 1.91 (m, 1H), 1.71 - 1.78 (m, 1H), 1.49 - 1.61 (m, 2H).

**HPLC-MS (ESI-MS):** Calculated: 389.12 for C₁₇H₁₉N₅O₄S [M+H⁺]. Found: 390.1. ***t*_{R}** = 4.7 min (HPLC Condition 1).

### Preparation Example 3a.21: Preparation of (S)-5-(3-iodophenyl)-N-(piperidin-3-yl)-3-ureidothiophene-2-carboxamide

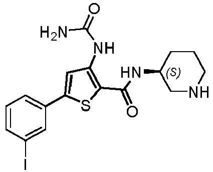

A target compound was obtained by repeating the same procedure as in Preparation Method 3a, except that in Step 1 of Preparation Example 3a.1, 3-chloro-3-(3-iodophenyl)acrylonitrile (C₉H₅ClIN, 289.50 g/mol, 651.4 mg, 2.25 mmol, 0.9 equiv.) was used instead of 3-(3-fluorophenyl)-3-chloroacrylonitrile. The intermediate *tert*-butyl (*S*)-3-(3-amino-5-(3-iodophenyl)thiophene-2-carboxamido)piperidine-1-carboxylate (C₂₁H₂₆IN₃O₃S, 527.42 g/mol, 697.8 mg, 1.32 mmol, 1 equiv.) from Step 1 was purified using Condition 1a for Automated Flash Column Chromatography on COMBIFLASH^{™} NextGen 300+ (Teledyne ISCO, Lincoln, NE), while the intermediate *tert*-butyl (*S*)-3-(5-(3-iodophenyl)-3-ureidothiophene-2-carboxamido)piperidine-1-carboxylate (C₂₂H₂₇IN₄O₄S, 527.42 g/mol, 720 mg, 1.26 mmol, 1 equiv) of Step 2 was purified by Condition 2a for Automated Flash Column Chromatography on COMBIFLASH^{™} NextGen 300+. The final product (*S*)-5-(3-iodophenyl)-*N*-(piperidin-3-yl)-3-ureidothiophene-2-carboxamide (C₁₈H₂₁IN₄O₂S, 470.33 g/mol, 593 mg, 1.26 mmol, 50 % (over 3 steps)) was obtained as a flaky off-white powder with high purity and moderate yield over 3 steps after purification by Condition 5 for Automated Flash Column Chromatography on COMBIFLASH^{™} NextGen 300+.

**¹H NMR** (600 MHz, DMSO-d₆) δ = 10.00 (s, 1H), 8.26 (s, 1H), 7.95 (t, J = 1.66 Hz, 1H), 7.89 (d, *J =* 7.77 Hz, 1H), 7.76 (appd, *J* = 7.86 Hz, 1H), 7.65 (ddd, *J* = 0.80, 2.00, 8.20 Hz, 1H), 7.27 (t, *J* = 7.86 Hz, 1H), 6.66 (br. s., 2H), 3.87 - 3.95 (m, 1H), 3.06 (appdd, *J* = 3.38, 11.74 Hz, 1H), 2.93 (td, *J* = 3.70, 12.49 Hz, 1H), 2.52 - 2.64 (m, 2H), 1.81 - 1.87 (m, 1H), 1.68 - 1.75 (m, 1H), 1.42 - 1.59 (m, 2H).

**HPLC-MS (ESI-MS):** Calculated: 470.03 for C₁₈H₂₁IN₄O₂S [M+H⁺]. Found: 471.1. ***t*_{R}** = 5.2 min (HPLC Condition 1).

### Preparation Example 3a.22: Preparation of (S)-5-(5-fluoro-2-methoxyphenyl)-N-(piperidin-3-yl)-3-ureidothiophene-2-carboxamide

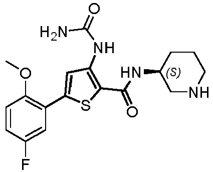

A target compound was obtained by repeating the same procedure as in Preparation Method 3a, except that in Step 1 of Preparation Example 3a.1, 3-chloro-3-(5-fluoro-2-methoxyphenyl)-acrylonitrile (C₁₀H₇ClFNO, 211.62 g/mol, 476.2 mg, 2.25 mmol, 1 equiv.) was used instead of 3-(3-fluorophenyl)-3-chloroacrylonitrile. The intermediate *tert*-butyl (*S*)-3-(3-amino-5-(5-fluoro-2-methoxyphenyl)thiophene-2-carboxamido)piperidine-1-carboxylate (C₂₂H₂₃FN₃O₄S, 449.55 g/mol, 538.8 mg, 1.12 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(5-fluoro-2-methoxyphenyl)-3-ureidothiophene-2-carboxamido)piperidine-1-carboxylate (C₂₃H₂₉FN₄O₅S, 492.57 g/mol, 473.7 mg, 0.96 mmol, 1 equiv.) of Step 2 was purified by Condition 2b for Automated Flash Column Chromatography on COMBIFLASH^{™} NextGen 300+. The final product (S)-5-(5-fluoro-2-methoxyphenyl)-*N*-(piperidin-3-yl)-3-ureidothiophene-2-carboxamide (C₁₈H₂₁FN₄O₃S, 392.45 g/mol, 374 mg, 0.95 mmol, 38 % (over 3 steps)) was obtained as a flaky off-white powder with high purity and moderate yield over 3 steps after purification by Condition 5 for Automated Flash Column Chromatography on COMBIFLASH^{™} NextGen 300+.

**¹H NMR** (400 MHz, DMSO-d₆) δ = 9.96 (s, 1H), 8.34 (s, 1H), 7.90 (d, *J* = 7.95 Hz, 1H), 7.47 (appdd, *J* = 2.10, 9.50 Hz, 1H), 7.17 - 7.27 (m, 2H), 6.60 (br. s., 2H), 3.95 - 4.01 (m, 1H), 3.93 (s, 3H), 3.10 (appdd, *J* = 3.36, 11.80 Hz, 1H), 2.99 (td, *J* = 3.20, 12.70 Hz, 1H), 2.67 (dd, *J* = 10.27, 11.74 Hz, 1H), 2.59 (appt, *J* = 11.10 Hz, 1H), 1.82 - 1.91 (m, 1H), 1.74 (dd, *J* = 3.55, 9.54 Hz, 1H), 1.46 - 1.64 (m, 2H).

**HPLC-MS (ESI-MS):** Calculated: 392.13 for C₁₈H₂₁FN₄O₃S [M+H⁺]. Found: 393.2. ***t*_{R}** = 4.8 min (HPLC Condition 1).

### Preparation Example 3a.23: Preparation of (S)-5-(benzofuran-2-yl)-N-(piperidin-3-yl)-3-ureidothiophene-2-carboxamide

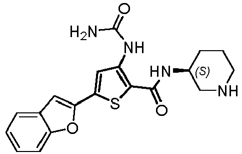

A target compound was obtained by repeating the same procedure as in Preparation Method 3a, except that in Step 1 of Preparation Example 3a.1, 3-(benzofuran-2-yl)-3-chloroacrylonitrile (C₁₁H₆ClNO, 203.63 g/mol, 458.2 mg, 2.25 mmol, 1 equiv.) was used instead of 3-(3-fluorophenyl)-3-chloroacrylonitrile. The intermediate *tert*-butyl (*S*)-3-(3-amino-5-(benzofuran-2-yl)thiophene-2-carboxamido)piperidine-1-carboxylate (C₂₃H₂₇N₃O₄S, 441.55 g/mol, 484.9 mg, 1.10 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(benzofuran-2-yl)-3-ureidothiophene-2-carboxamido)piperidine-1-carboxylate (C₂₄H₂₈N₄O₅S, 484.58 g/mol, 516.2 mg, 1.07 mmol, 1 equiv.) of Step 2 was purified by Condition 2b for Automated Flash Column Chromatography on COMBIFLASH^{™} NextGen 300+. The final product (*S*)-5-(benzofuran-2-yl)-*N*-(piperidin-3-yl)-3-ureidothiophene-2-carboxamide (C₁₉H₂₀N₄O₃S, 384.45 g/mol, 366.6 mg, 0.95 mmol, 38 % (over 3 steps)) was obtained as an off-white powder with high purity and moderate yield over 3 steps after purification by Condition 5 for Automated Flash Column Chromatography on COMBIFLASH^{™} NextGen 300+.

**¹H NMR** (400 MHz, DMSO-d₆) δ = 10.06 (s, 1H), 8.37 (s, 1H), 7.87 (d, *J* = 7.82 Hz, 1H), 7.55 - 7.73 (m, 2H), 7.32 - 7.40 (m, 2H), 7.29 (ddd, J = 0.73, 7.00, 8.00 Hz, 1H), 6.69 (br. s., 2H), 3.74 - 3.88 (m, 1H), 2.90 - 3.03 (m, 1H), 2.81 (td, *J* = 3.20, 12.23 Hz, 1H), 2.38 - 2.49 (m, 2H), 1.77 - 1.89 (m, 1H), 1.64 (td, *J* = 4.00, 12.70 Hz, 1H), 1.36 - 1.58 (m, 2H).

**HPLC-MS (ESI-MS):** Calculated: 384.13 for C₁₉H₂₀N₄O₃S [M+H⁺]. Found: 385.2. ***t*_{R}** = 5.0 min (HPLC Condition 1).

### Preparation Example 3a.24: Preparation of (S)-5-(4-morpholinophenyl)-N-(piperidin-3-yl)-3-ureidothiophene-2-carboxamide

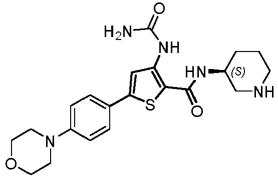

A target compound was obtained by repeating the same procedure as in Preparation Method 3a, except that in Step 1 of Preparation Example 3a.1, 3-chloro-3-(4-morpholinophenyl)acrylonitrile (C₁₃H₁₃ClN₂O, 248.71 g/mol, 559.64 mg, 2.25 mmol, 1 equiv.) was used instead of 3-(3-fluorophenyl)-3-chloroacrylonitrile. The intermediate *tert*-butyl (*S*)-3-(3-amino-5-(4-morpholinophenyl)thiophene-2-carboxamido)piperidine-1-carboxylate (C₂₅H₃₄N₄O₄S, 486.64 g/mol, 103.6 mg, 0.21 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(4-morpholinophenyl)-3-ureidothiophene-2-carboxamido)piperidine-1-carboxylate (C₂₆H₃₅N₅O₅S, 529.66 g/mol, 46 mg, 0.09 mmol, 1 equiv.) of Step 2 was purified by Condition 2b for Automated Flash Column Chromatography on COMBIFLASH^{™} NextGen 300+. The final product (*S*)-5-(4-morpholinophenyl)-*N*-(piperidin-3-yl)-3-ureidothiophene-2-carboxamide (C₂₁H₂₇N₅O₃S, 429.54 g/mol, 15.8 mg, 0.03 mmol, 1 % (over 3 steps)) was obtained as a yellow solid with high purity and low yield over 3 steps after purification by Condition 5 for Automated Flash Column Chromatography on COMBIFLASH^{™} NextGen 300+.

**¹H NMR** (400 MHz, DMSO-d₆) δ = 10.06 (s, 1H), 8.10 (s, 1H), 7.69 (d, *J* = 7.90 Hz, 1H), 7.49 (d, *J* = 8.80 Hz, 2H), 7.01 (d, *J* = 8.93 Hz, 2H), 6.60 (br. s, 2H), 3.81 - 3.92 (m, 1H), 3.74 (t, *J* = 4.40 Hz, 4H), 3.50 (s, 1H), 3.18 (t, *J* = 4.80 Hz, 4H), 3.00 (appd, *J* = 11.13 Hz, 1H), 2.87 (appd, *J* = 11.37 Hz, 1H), 2.52 - 2.59 (m, 2H), 1.77 - 1.86 (m, 1H), 1.63 - 1.71 (m, 1H), 1.49 (appspt, *J* = 10.80 Hz, 1H).

**HPLC-MS (ESI-MS):** Calculated: 429.18 for C₂₁H₂₇N₅O₃S [M+H⁺]. Found: 430.2. ***t*_{R}** = 4.3 min (HPLC Condition 1).

### Preparation Example 3a.25: Preparation of (S)-5-(5-methyl-1-phenyl-1H-pyrazol-4-yl)-N-(piperidin-3-yl)-3-ureidothiophene-2-carboxamide

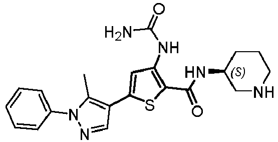

A target compound was obtained by repeating the same procedure as in Preparation Method 3a, except that in Step 1 of Preparation Example 3a.1, 3-chloro-3-(5-methyl-1-phenyl-1*H*-pyrazol-4-yl)acrylonitrile (C₁₃H₁₀ClN₃, 243.69 g/mol, 548.35 mg, 2.25 mmol, 1 equiv.) was used instead of 3-(3-fluorophenyl)-3-chloroacrylonitrile. The intermediate *tert*-butyl (*S*)-3-(3-amino-5-(5-methyl-1-phenyl-1*H*-pyrazol-4-yl)thiophene-2-carboxamido)piperidine-1-carboxylate (C₂₅H₃₁N₅O₃S, 481.62 g/mol, 674.6 mg, 1.40 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate tert-butyl (S)-3-(5-(5-methyl-1-phenyl-1*H*-pyrazol-4-yl)-3-ureidothiophene-2-carboxamido)piperidine-1-carboxylate (C₂₆H₃₂N₆O₄S, 524.64 g/mol, 501.9 mg, 0.96 mmol, 1 equiv.) of Step 2 was purified by Condition 2b for Automated Flash Column Chromatography on COMBIFLASH^{™} NextGen 300+. The final product (*S*)-5-(5-methyl-1-phenyl-1*H*-pyrazol-4-yl)-*N*-(piperidin-3-yl)-3-ureidothiophene-2-carboxamide (C₂₁H₂₄N₆O₂S, 424.52 g/mol, 399.8 mg, 0.94 mmol,38 % (over 3 steps)) was obtained as an off-white powder with high purity and moderate yield over 3 steps after purification by Condition 5 for Automated Flash Column Chromatography on COMBIFLASH^{™} NextGen 300+. **¹H NMR** (400 MHz, DMSO-d₆) δ = 10.13 (s, 1H), 8.05 (s, 1H), 7.95 (s, 1H), 7.71 (d, *J* = 7.82 Hz, 1H), 7.54 - 7.62 (m, 4H), 7.46 - 7.54 (m, 1H), 6.61 (br. s., 2H), 3.79 - 3.89 (m, 1H), 3.62 (br. s., 1H), 2.98 (dd, J *=* 3.06, 11.62 Hz, 1H), 2.84 (td, *J* = 2.50, 12.23 Hz, 1H), 2.50 (br. s., 3H), 2.38 - 2.49 (m, 2H), 1.78 - 1.88 (m, 1H), 1.66 (td, *J* = 3.90, 12.72 Hz, 1H), 1.37 - 1.59 (m, 2H).

**HPLC-MS (ESI-MS):** Calculated: 424.17 for C₂₁H₂₄N₆O₂S [M+H+]. Found: 425.2. ***t*_{R}** = 0.8 min (HPLC Condition 1).

### Preparation Example 3a.26: Preparation of (S)-5-(3-morpholinophenyl)-N-(piperidin-3-yl)-3-ureidothiophene-2-carboxamide

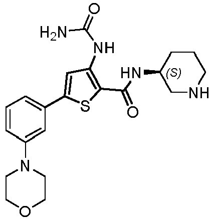

A target compound was obtained by repeating the same procedure as in Preparation Method 3a, except that in Step 1 of Preparation Example 3a.1, 3-chloro-3-(4-morpholinophenyl)acrylonitrile (C₁₃H₁₃ClN₂O, 248.71 g/mol, 559.64 mg, 2.25 mmol, 1 equiv.) was used instead of 3-(3-fluorophenyl)-3-chloroacrylonitrile. The intermediate *tert*-butyl (*S*)-3-(3-amino-5-(3-morpholinophenyl)thiophene-2-carboxamido)piperidine-1-carboxylate (C₂₅H₃₄N₄O₄S, 486.64 g/mol, 129.1 mg, 0.27 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3-(5-(3-morpholinophenyl)-3-ureidothiophene-2-carboxamido)piperidine-1-carboxylate (C₂₆H₃₅N₅O₅S, 529.66 g/mol, 98.7 mg, 0.19 mmol, 1 equiv.) of Step 2 was purified by Condition 2b for Automated Flash Column Chromatography on COMBIFLASH^{™} NextGen 300+. The final product (*S*)-5-(3-morpholinophenyl)-*N*-(piperidin-3-yl)-3-ureidothiophene-2-carboxamide (C₂₁H₂₇N₅O₃S, 429.54 g/mol, 71.6 mg, 0.17 mmol, 7 % (over 3 steps)) was obtained as a yellow solid with high purity and low yield over 3 steps after purification by Condition 5 for Automated Flash Column Chromatography on COMBIFLASH^{™} NextGen 300+.

**¹H NMR** (500 MHz, DMSO-d₆) δ = 10.05 (s, 1H), 8.21 (s, 1H), 7.78 (d, *J* = 7.93 Hz, 1H), 7.32 (appt, *J* = 7.86 Hz, 1H), 7.05 - 7.13 (m, 2H), 7.00 (dd, *J* = 1.98, 8.39 Hz, 1H), 6.62 (br. s., 2H), 3.81 - 3.89 (m, 1H), 3.76 (t, *J* = 4.60 Hz, 4H), 3.17 (t, *J* = 4.90 Hz, 4H), 2.99 (dd, *J* = 2.90, 11.60 Hz, 1H), 2.85 (td, *J* = 3.20, 12.21 Hz, 1H), 2.44 - 2.49 (m, 1H), 1.82 (appd, *J* = 8.54 Hz, 1H), 1.66 (td, *J* = 4.00, 12.82 Hz, 1H), 1.37 - 1.58 (m, 2H).

**HPLC-MS (ESI-MS):** Calculated: 429.18 for C₂₁H₂₇N₅O₃S [M+H⁺]. Found: 430.2. ***t*_{R}** = 4.2 min (HPLC Condition 1).

### Preparation Example 3a.27: Preparation of (S)-5-(4-(1H-imidazol-1-yl)phenyl)-N-(piperidin-3-yl)-3-ureidothiophene-2-carboxamide

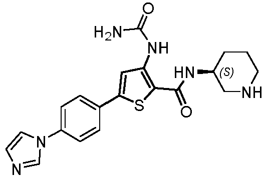

A target compound was obtained by repeating the same procedure as in Preparation Method 3a, except that in Step 1 of Preparation Example 3a.1, 3-(4-(1*H*-imidazol-1-yl)phenyl)-3-chloro-acrylonitrile (C₁₂H₈ClN₃, 229.67 g/mol, 516.8 mg, 2.25 mmol, 1 equiv.) was used instead of 3-(3-fluorophenyl)-3-chloroacrylonitrile. The intermediate *tert*-butyl (*S*)-3-(5-(4-(1H-imidazol-1-yl)phenyl)-3-aminothiophene-2-carboxamido)piperidine-1-carboxylate (C₂₄H₂₉N₅O₃S, 467.59 g/mol, 266.6 mg, 0.57 mmol, 1 equiv.) from Step 1 was purified using Condition 2b for Automated Flash Column Chromatography on COMBIFLASH^{™} NextGen 300+ (Teledyne ISCO, Lincoln, NE), while the intermediate *tert*-butyl *tert*-butyl (*S*)-3-(5-(4-(1H-imidazol-1-yl)phenyl)-3-ureidothiophene-2-carboxamido)piperidine-1-carboxylate (C₂₅H₃₆N₆O₄S, 510.62 g/mol, 40.6 mg, 0.08 mmol, 1 equiv.) of Step 2 was purified by Condition 2c for Automated Flash Column Chromatography on COMBIFLASH^{™} NextGen 300+. The final product (*S*)-5-(4-(1*H*-imidazol-1-yl)phenyl)-*N*-(piperidin-3-yl)-3-ureidothiophene-2-carboxamide (C₂₆H₂₂N₆O₂S, 410.50 g/mol, 30.1 mg, 0.07 mmol, 3 % (over 3 steps)) was obtained as an off-white powder with high purity and low yield over 3 steps after purification by Condition 5 for Automated Flash Column Chromatography on COMBIFLASH^{™} NextGen 300+.

**¹H NMR** (500 MHz, DMSO-d₆) δ = 10.08 (br. s., 1H), 8.33 (s, 1H), 8.27 (s, 1H), 7.81 (appt, *J =* 1.14 Hz, 1H), 7.74 - 7.79 (m, 5H), 7.13 (apps, 1H), 6.65 (br. s, 2H), 3.74 - 3.83 (m, 1H), 2.93 (dd, *J* = 3.40, 11.80 Hz, 1H), 2.77 (td, *J* = 3.70, 12.05 Hz, 1H), 2.38 - 2.48 (m, 2H), 1.76 - 1.86 (m, 1H), 1.61 (td, *J* = 4.20, 12.80 Hz, 1H), 1.46 - 1.56 (m, 1H), 1.34 - 1.44 (m, 1H).

**HPLC-MS (ESI-MS):** Calculated: 410.15 for C₂₆H₂₂N₆O₂S [M+H⁺], Found: 381.2. ***t*_{R}** = 4.8 min (HPLC Condition 1).

### Preparation Example 3a. 28: Preparation of (S)-N-(piperidin-3-yl)-5-(3-(pyridin-4-yl)phenyl)-3-ureidothiophene-2-carboxamide

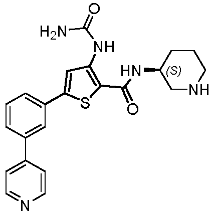

A target compound was obtained by repeating the same procedure as in Preparation Method 3a, except that in Step 1 of Preparation Example 3a.1, 3-chloro-3-(3-(pyridin-4-yl)phenyl)acrylonitrile (C₁₄H₉ClN₂, 240.69 g/mol, 541.6 mg, 2.25 mmol, 1 equiv.) was used instead of 3-(3-fluorophenyl)-3-chloroacrylonitrile. The intermediate *tert*-butyl (*S*)-3-(3-amino-5-(3-(pyridin-4-yl)phenyl)thiophene-2-carboxamido)piperidine-1-carboxylate (C₂₆H₃₆N₄O₃S, 478.62 g/mol, 92.9 mg, 0.19 mmol, 1 equiv.) from Step 1 was purified using Condition 2b for Automated Flash Column Chromatography on COMBIFLASH^{™} NextGen 300+ (Teledyne ISCO, Lincoln, NE), while the intermediate *tert*-butyl (*S*)-3-(5-(3-(pyridin-4-yl)phenyl)-3-ureidothiophene-2-carboxamido)piperidine-1-carboxylate (C₂₇H₃₁N₅O₄S, 521.64 g/mol, 40 mg, 0.08 mmol, 1 equiv.) of Step 2 was purified by Condition 2c for Automated Flash Column Chromatography on COMBIFLASH^{™} NextGen 300+. The final product (*S*)-*N*-(piperidin-3-yl)-5-(3-(pyridin-4-yl)phenyl)-3-ureidothiophene-2-carboxamide (C₂₂H₂₃N₅O₂S, 421.52 g/mol, 31.2 mg, 0.07 mmol, 3 % (over 3 steps)) was obtained as an off-white powder with high purity and low yield over 3 steps after purification by Condition 5 for Automated Flash Column Chromatography on COMBIFLASH^{™} NextGen 300+.

**¹H NMR** (500 MHz, DMSO-d₆) δ = 10.03 (s, 1H), 8.68 (dd, J= 1.60, 4.80 Hz, 2H), 8.35 (s, 1H), 7.96 (t, *J* = 1.60 Hz, 1H), 7.92 (d, *J* = 7.78 Hz, 1H), 7.82 (td, J= 0.90, 7.63 Hz, 1H), 7.78 (dd, J = 1.68, 4.80 Hz, 1H), 7.75 (td, *J* = 1.10, 7.80 Hz, 1H), 7.63 (t, *J* = 7.80 Hz, 1H), 6.67 (br. s, 2H), 3.89 - 4.00 (m, 1H), 3.08 (dd, *J* = 3.28, 11.83 Hz, 1H), 2.94 (td, *J* = 3.40, 12.36 Hz, 1H), 2.54 - 2.66 (m, 2H), 1.82 - 1.89 (m, 1H), 1.69 - 1.76 (m, 1H), 1.46 - 1.62 (m, 3H).

**HPLC-MS (ESI-MS):** Calculated: 421.16 for C₂₂H₂₃N₅O₂S [M+H⁺]. Found: [M-C₅H₁₁N₂+H⁺] 322.0; 422.2. ***t*_{R}** = 3.8 min (HPLC Condition 1).

### Preparation Example 3a.29: Preparation of (S)-N-(piperidin-3-yl)-5-(4-(pyridin-4-yl)phenyl)-3-ureidothiophene-2-carboxamide

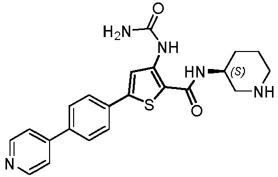

A target compound was obtained by repeating the same procedure as in Preparation Method 3a, except that in Step 1 of Preparation Example 3a.1, 3-chloro-3-(4-(pyridin-4-yl)phenyl)acrylonitrile (C₁₄H₉ClN₂, 240.69 g/mol, 541.6 mg, 2.25 mmol, 1 equiv.) was used instead of 3-(3-fluorophenyl)-3-chloroacrylonitrile. The intermediate *tert*-butyl (*S*)-3-(3-amino-5-(4-(pyridin-4-yl)phenyl)thiophene-2-carboxamido)piperidine-1-carboxylate (C₂₆H₃₆N₄O₃S, 478.62 g/mol, 124.4 mg, 0.26 mmol, 1 equiv.) from Step 1 was purified using Condition 2b for Automated Flash Column Chromatography on COMBIFLASH^{™} NextGen 300+ (Teledyne ISCO, Lincoln, NE), while the intermediate *tert*-butyl (S)-3-(5-(4-(pyridin-4-yl)phenyl)-3-ureidothiophene-2-carboxamido)piperidine-1-carboxylate (C₂₇H₃₁N₅O₄S, 521.64 g/mol, 29.5 mg, 0.06 mmol, 1 equiv.) of Step 2 was purified by Condition 2c for Automated Flash Column Chromatography on COMBIFLASH^{™} NextGen 300+. The final product (*S*)-*N*-(piperidin-3-yl)-5-(4-(pyridin-4-yl)phenyl)-3-ureidothiophene-2-carboxamide (C₂₂H₂₃N₅O₂S, 421.52 g/mol, 21.9 mg, 0.05 mmol, 2 % (over 3 steps)) was obtained as an off-white powder with high purity and low yield over 3 steps after purification by Condition 5 for Automated Flash Column Chromatography on COMBIFLASH^{™} NextGen 300+.

**¹H NMR** (500 MHz, DMSO-d₆) δ = 9.98 (s, 1H), 8.66 (dd, *J* = 1.53, 4.80 Hz, 1H), 8.36 (s, 1H), 8.15 (appt, *J* = 7.00 Hz, 1H), 7.90 - 7.99 (m, 2H), 7.75 - 7.81 (m, 4H), 6.72 (br. s, 2H), 4.09 - 4.24 (m, 1H), 3.21 (appd, *J* = 12.36 Hz, 1H), 2.89 (appt, *J* = 11.29 Hz, 1H), 2.82 (appt, *J* = 10.76 Hz, 1H), 2.63 (appt, *J* = 1.80 Hz, 1H), 2.53 - 2.62 (m, 2H), 1.88 - 1.92 (m, 1H), 1.66 (appquin, *J* = 12.00 Hz, 2H).

**HPLC-MS (ESI-MS):** Calculated: 421.16 for C₂₂H₂₃N₅O₂S [M+H⁺]. Found: 422.2. ***t*_{R}** = 3.7 min (HPLC Condition 1).

### Preparation Example 3a.30: Preparation of 5-(3-fluorophenyl)-N-((3S,5S)-5-fluoropiperidin-3-yl)-3-ureidothiophene-2-carboxamide

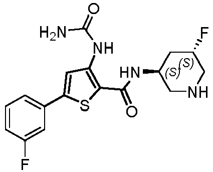

A target compound was obtained by repeating the same procedure as in Preparation Method 3a, except that in Step 1 of Preparation Example 3a.1, *tert*-butyl (3*S*,5*S*)-3-(2-(acetylthio)acetamido)-5-fluoropiperidine-1-carboxylate (C₁₄H₂₃FN₂O₄S, 334.41 g/mol, 836 mg, 2.50 mmol, 1 equiv.) was used instead of *tert*-butyl (*S*)-3-(2-(acetylthio)acetamido) piperidine-1-carboxylate. The intermediate *tert*-butyl (*S*)-3-(3-amino-5-(3-fluorophenyl)thiophene-2-carboxamido)pyrrolidine-1-carboxylate (C₂₁H₂₅F₂N₃O₃S, 437.51 g/mol, 221.6 mg, 0.51 mmol, 1 equiv.) from Step 1 was purified using Condition 2c for Automated Flash Column Chromatography on COMBIFLASH^{™} NextGen 300+ (Teledyne ISCO, Lincoln, NE), while the intermediate *tert*-butyl (*S*)-3-(5-(3-fluorophenyl)-3-ureidothiophene-2-carboxamido)pyrrolidine-1-carboxylate (C₂₂H₂₆F₂N₄O₄S, 480.54 g/mol, 153.8 mg, 0.32 mmol, 1 equiv.) of Step 2 was purified by Condition 2b for Automated Flash Column Chromatography on COMBIFLASH^{™} NextGen 300+. The final product 5-(3-fluorophenyl)-*N*-((3*S*,5*S*)-5-fluoropiperidin-3-yl)-3-ureidothiophene-2-carboxamide (C₁₇H₁₈F₂N₄O₂S, 380.41 g/mol, 118.9 mg, 0.31 mmol, 13 % (over 3 steps)) was obtained as an off-white powder with high purity and low yield over 3 steps after purification by Condition 5 for Automated Flash Column Chromatography on COMBIFLASH^{™} NextGen 300+.

**¹H NMR** (500 MHz, DMSO-d₆) δ = 10.03 (s, 1H), 8.29 (s, 1H), 7.89 (d, *J* = 7.93 Hz, 1H), 7.40 - 7.58 (m, 1H), 7.25 (appdt, *J* = 1.70, 8.20 Hz, 1H), 6.67 (br. s, 2H), 4.79 (d, *J* = 48.22 Hz, 1H), 4.01 - 4.21 (m, 1H), 2.87 - 3.00 (m, 2H), 2.63 (appdd, J= 14.04, 37.38 Hz, 1H), 2.45 - 2.50 (m, 1H), 2.10 (appt, *J* = 11.90 Hz, 1H), 1.75 - 1.93 (m, 1H).

**HPLC-MS (ESI-MS):** Calculated: 380.11 for C₁₇H₁₈F₂N₄O₂S [M+H⁺], Found: 381.1. ***t*_{R}** = 0.8 min (HPLC Condition 1).

### Preparation Example 3a.31: Preparation of (S)-N-(5,5-difluoropiperidin-3-yl)-5-(3-fluorophenyl)-3-ureidothiophene-2-carboxamide

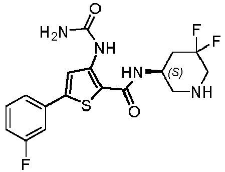

A target compound was obtained by repeating the same procedure as in Preparation Method 3a, except that in Step 1 of Preparation Example 3a.1, (*S*)-5-(2-(acetylthio)acetamido)-3,3-difluoropiperidine-1-carboxylate (C₁₄H₂₂F₂N₂O₄S, 352.40 g/mol, 881 mg, 2.50 mmol, 1 equiv.) was used instead of *tert*-butyl (*S*)-3-(2-(acetylthio)acetamido) piperidine-1-carboxylate. The intermediate *tert*-butyl (*S*)-5-(3-amino-5-(3-fluorophenyl)thiophene-2-carboxamido)-3,3-difluoropiperidine-1-carboxylate (C₂₁H₂₄F₃N₃O₃S, 455.50 g/mol, 235.4 mg, 0.52 mmol, 1 equiv.) from Step 1 was purified using Condition 2a for Automated Flash Column Chromatography on COMBIFLASH^{™} NextGen 300+ (Teledyne ISCO, Lincoln, NE), while the intermediate *tert*-butyl (*S*)-3,3-difluoro-5-(5-(3-fluorophenyl)-3-ureidothiophene-2-carboxamido)piperidine-1-carboxylate (C₂₂H₂₅F₃N₄O₄S, 498.53 g/mol, 254 mg, 0.51 mmol, 1 equiv.) of Step 2 was purified by Condition 2b for Automated Flash Column Chromatography on COMBIFLASH^{™} NextGen 300+. The final product (*S*)-*N*-(5,5-difluoropiperidin-3-yl)-5-(3-fluorophenyl)-3-ureidothiophene-2-carboxamide (C₁₇H₁₇F₃N₄O₂S, 398.40 g/mol, 200.9 mg, 0.5 mmol, 20 % (over 3 steps)) was obtained as an off-white powder with high purity and moderate yield over 3 steps after purification by Condition 5 for Automated Flash Column Chromatography on COMBIFLASH^{™} NextGen 300+. ***t*_{R}** = 4.9 min (HPLC Condition 1)

**¹H NMR** (500 MHz, DMSO-d₆) δ = 10.00 (s, 1H), 8.30 (s, 1H), 7.99 (d, *J* = 7.93 Hz, 1H), 7.39 - 7.59 (m, 3H), 7.25 (ddt, *J* = 0.70, 2.10, 8.50 Hz, 1H), 6.68 (br. s., 2H), 3.99 - 4.14 (m, 1H), 3.33 (br. s., 1H), 2.97 - 3.08 (m, 1H), 2.94 (apptd, *J* = 3.40, 12.66 Hz, 1H), 2.74 (dd, *J* = 13.00, 30.00 Hz, 1H), 2.43 - 2.50 (m, 1H), 2.31 (dd, *J*= 10.99, 21.51 Hz, 1H), 2.04 (appdtd, *J* = 5.50, 15.70, 26.10 Hz, 1H).

**HPLC-MS (ESI-MS):** Calculated: 398.10 for C₁₇H₁₇F₃N₄O₂S [M+H⁺]. Found: 399.1; [M+Na'] 421.1. ***t*_{R}** = 4.9 min (HPLC Condition 1).

### Preparation Example 3a.32: Preparation of N-((3S,5S)-5-fluoropiperidin-3-yl)-5-(5-methyl-1-phenyl-1H-pyrazol-4-yl)-3-ureidothiophene-2-carboxamide

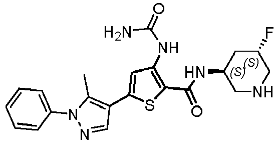

A target compound was obtained by repeating the same procedure as in Preparation Method 3a, except that in Step 1 of Preparation Example 3a.1, 3-chloro-3-(5-methyl-1-phenyl-1*H*-pyrazol-4-yl)acrylonitrile (C₁₃H₁₀ClN₃, 243.69 g/mol, 173.8 mg, 0.7 mmol, 0.9 equiv.) and *tert*-butyl (3*S*,5*S*)-3-(2-(acetylthio)acetamido)-5-fluoropiperidine-1-carboxylate (C₁₄H₂₃FN₂O₄S, 334.41 g/mol, 265 mg, 0.8 mmol, 1 equiv.) was used instead of 3-(3-fluorophenyl)-3-chloroacrylonitrile and *tert*-butyl (*S*)-3-(2-(acetylthio)acetamido) piperidine-1-carboxylate. The intermediate *tert*-butyl (3*S*,5*S*)-3-(3-amino-5-(5-methyl-1-phenyl-1*H*-pyrazol-4-yl)thiophene-2-carboxamido)-5-fluoropiperidine-1-carboxylate (C₂₅H₃₀FN₅O₃S, 499.61 g/mol, 306.8 mg, 0.46 mmol, 1 equiv.) from Step 1 was purified using Condition 2c for Automated Flash Column Chromatography on COMBIFLASH^{™} NextGen 300+ (Teledyne ISCO, Lincoln, NE), while the intermediate *tert*-butyl (3*S*,5*S*)-3-fluoro-5-(5-(5-methyl-1-phenyl-1*H*-pyrazol-4-yl)-3-ureidothiophene-2-carboxamido)piperidine-1-carboxylate (C₂₆H₃₁FN₆O₄S, 542.63 g/mol, 157.3 mg, 0.29 mmol, 1 equiv.) of Step 2 was purified by Condition 2b for Automated Flash Column Chromatography on COMBIFLASH^{™} NextGen 300+. The final product 5 *N*-((3*S*,5*S*)-5-fluoropiperidin-3-yl)-5-(5-methyl-1-phenyl-1*H*-pyrazol-4-yl)-3-ureidothiophene-2-carboxamide (C₂₁H₂₃FN₆O₂S, 442.51 g/mol, 96.7 mg, 0.22 mmol, 28 % (over 3 steps)) was obtained as an off-white powder with high purity and moderate yield over 3 steps after purification by Condition 5 for Automated Flash Column Chromatography on COMBIFLASH^{™} NextGen 300+.

**¹H NMR (600 MHz, DMSO-d6)** δ = 10.12 (s, 1H), 8.05 (s, 1H), 7.94 (s, 1H), 7.76 (d, *J* = 8.04 Hz, 1H), 7.55 - 7.59 (m, 4H), 7.49 (appdt, *J* = 2.77, 5.74 Hz, 1H), 6.61 (br. s, 1H), 4.79 (appd, *J* = 48.23 Hz, 1H), 4.04 - 4.15 (m, 1H), 2.92 (dd, *J* = 12.80, 24.20 Hz, 2H), 2.62 (dd, *J* = 14.30, 37.60 Hz, 1H), 2.48 - 2.49 (m, 3H), 2.47 - 2.48 (m, 1H), 2.09 (appt, *J* = 12.13 Hz, 1H), 1.83 (appdtd, *J* = 2.80, 12.80, 41.00 Hz, 1H) **HPLC-MS (ESI-MS):** Calculated: 442.16 for C₁₇H₁₈F₂N₄O₂S [M+H⁺], Found: 443.2. ***t*_{R}** = 4.8 min (HPLC Condition 1).

### Preparation Method 3b: Synthesis of 2,3-Thiophenecarboxamideureas

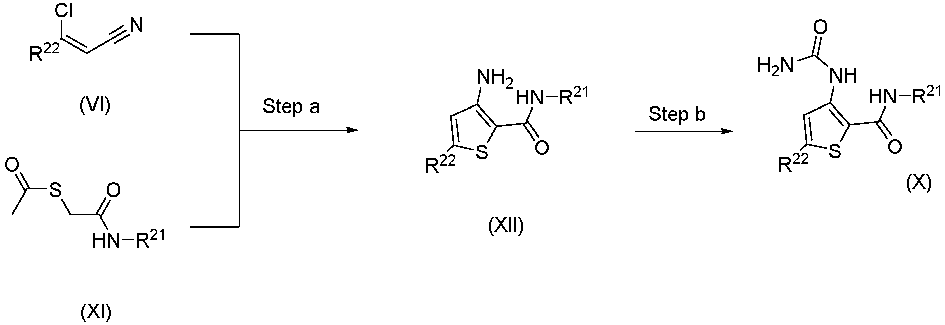

The reaction in above Step a is a process of forming a 2,3,5-substituted amino thiophene core by allowing a β-chlorocinnamonitrile compound represented by Chemical Formula (VI) to react with a thioacetate compound represented by Chemical Formula (XI).
Specifically, the reaction in above Step a is performed at a temperature of 21 °C to 24 °C in the presence of a strong alkoxide base. In this case, the base can be selected from mono-, di-, or tri-C₁₋₆ alkoxide derivatives. As a reaction solvent, a typical organic solvent can be used, and the Examples of the present invention specifically exemplify examples in which tetrahydrofuran (THF) is usually used, but the solvent of the present invention is not limited thereto.

The reaction in above Step b is a process of interconverting a primary amine group into an urea group at the C3 position of the thiophene core by allowing the 2,3,5-substituted amino thiophene compound represented by Chemical Formula (XII) to react with trichloroacetyl isocyanate and subsequentially adding methanolic ammonia solution.
Specifically, the first step of the reaction in above Step b is performed at a temperature of 21 °C to 24 °C in the presence of an isocyanate compound like trichloroacetyl isocyanate. As a reaction solvent, a typical organic solvent can be used, and the Examples of the present invention specifically exemplify examples in which tetrahydrofuran (THF) is usually used, but the solvent of the present invention is not limited thereto.
Specifically, the second step of the reaction in above Step b is performed at a temperature of 0 °C to 5 °C in the presence of a methanolic ammonia solution, which also serves as a solvent.

### [Preparation Examples of Method 3b]

The compounds synthesized in the Examples of the present invention were purified or subjected to structural analysis under the following conditions.
(1) HPLC Condition for Structural Analysis
   - Elution Solvent A: 0.1 % trifluoroacetic acid (TFA)/water
   - Elution Solvent B: 0.1 % trifluoroacetic acid (TFA)/CH₃CN
   - Column: NUCLEODUR^{™} C18 Gravity, 3 µm, 125 × 4 mm analytical column (Macherey- Nagel, Germany)
   - Elution Condition: Elution for 9.5 minutes while varying the concentration of Solvent B from 10 to 100% at a moving rate of 1.0 mL/min.
(2) Condition 1a for Automated Flash Column Chromatography on COMBIFLASH^{™} NextGen 300+ (Teledyne ISCO, Lincoln, NE)
   - Elution Solvent A: petroleum ether 40-60 °C (PE)
   - Elution Solvent B: ethylacetate (EtOAc)
   - Column: FLASHPURE^{™} EXOFLEX^{™} Silica, 24 g, 40 µm irregular, 60 Å pore size, preparative column (Büchi, Switzerland)
   - Elution Condition: Elution for 16 Column Volumes (CV) at a flow rate of 32 mL/min while varying the concentration of Solvent B in the following pattern: 0 % (3 CV) → 60 % (5 CV) → 60 % (5 CV) → 100 % (1 CV) → 100 % (2 CV)
(3) Condition 1b for Automated Flash Column Chromatography on COMBIFLASH^{™} NextGen 300+ (Teledyne ISCO, Lincoln, NE)
   - Elution Solvent A: petroleum ether 40-60 °C (PE)
   - Elution Solvent B: ethylacetate (EtOAc)
   - Column: FLASHPURE^{™} EXOFLEX^{™} Silica, 24 g, 40 µm irregular, 60 Å pore size, preparative column (Büchi, Switzerland)
   - Elution Condition: Elution for 16 Column Volumes (CV) at a flow rate of 32 mL/min while varying the concentration of Solvent B in the following pattern: 0 % (3 CV) → 80 % (5 CV) → 80 % (5 CV) → 100 % (1 CV) →f 100 % (2 CV)
(4) Condition 2a for Automated Flash Column Chromatography on COMBIFLASH^{™} NextGen 300+ (Teledyne ISCO, Lincoln, NE)
   - Elution Solvent A: dichloromethane (DCM)
   - Elution Solvent B: ethylacetate (EtOAc)
   - Column: FLASHPURE^{™} EXOFLEX^{™} Silica, 40 g, 40 µm irregular, 60 Å pore size, preparative column (Büchi, Switzerland)
   - Elution Condition: Elution for 16 Column Volumes (CV) at a flow rate of 40 mL/min while varying the concentration of Solvent B in the following pattern: 0 % (3 CV) → 30 % (3 CV) → 30 % (5 CV) → 100 % (3 CV) → 100 % (2 CV)
(5) Condition 2b for Automated Flash Column Chromatography on COMBIFLASH^{™} NextGen 300+ (Teledyne ISCO, Lincoln, NE)
   - Elution Solvent A: dichloromethane (DCM)
   - Elution Solvent B: ethylacetate (EtOAc)
   - Column: FLASHPURE^{™} EXOFLEX^{™} Silica, 40 g, 40 µm irregular, 60 Å pore size, preparative column (Büchi, Switzerland)
   - Elution Condition: Elution for 18 Column Volumes (CV) at a flow rate of 40 mL/min while varying the concentration of Solvent B in the following pattern: 0 % (3 CV) → 50 % (5 CV) → 50 % (6 CV) → 100 % (4 CV) → 100 % (2 CV)
(6) Condition 2c for Automated Flash Column Chromatography on COMBIFLASH^{™} NextGen 300+ (Teledyne ISCO, Lincoln, NE)
   - Elution Solvent A: 1 % triethylamine (TEA)/dichloromethane (DCM)
   - Elution Solvent B: 1 % triethylamine (TEA)ethylacetate (EtOAc)
   - Column: FLASHPURE^{™} EXOFLEX^{™} Silica, 24 g, 40 µm irregular, 60 Å pore size, preparative column (Büchi, Switzerland)
   - Elution Condition: Elution for 15 Column Volumes (CV) at a flow rate of 32 mL/min while varying the concentration of Solvent B in the following pattern: 0 % (3 CV) → 40 % (5 CV) → 40 % (3 CV) → 100 % (2 CV) → 100 % (2 CV)
(7) Condition 3 for Automated Flash Column Chromatography on COMBIFLASH^{™} NextGen 300+ (Teledyne ISCO, Lincoln, NE)
   - Elution Solvent A: n-pentane (Pen)
   - Elution Solvent B: diethylether (Et₂O)
   - Column: FLASHPURE^{™} EXOFLEX^{™} Silica, 24 g, 40 µm irregular, 60 Å pore size, preparative column (Büchi, Switzerland)
   - Elution Condition: Elution for 16 Column Volumes (CV) while varying the concentration of Solvent B in the following pattern: 0 % (3 CV) → 50 % (5 CV) → 50 % (5 CV) → 100 % (1 CV) → 100 % (2 CV)
(8) Condition 4a for Automated Flash Column Chromatography on COMBIFLASH^{™} NextGen 300+ (Teledyne ISCO, Lincoln, NE)
   - Elution Solvent A: dichloromethane (DCM)
   - Elution Solvent B: methanol (CH₃OH)
   - Column: FLASHPURE^{™} EXOFLEX^{™} Silica, 24 g, 40 µm irregular, 60 Å pore size, preparative column (Büchi, Switzerland)
   - Elution Condition: Elution for 15 Column Volumes (CV) at a flow rate of 32 mL/min while varying the concentration of Solvent B in the following pattern: 0 % (3 CV) → 20 % (5 CV) → 20 % (1 CV) → 100 % (3 CV) → 100 % (3 CV)
(9) Condition 4b for Automated Flash Column Chromatography on COMBIFLASH^{™} NextGen 300+ (Teledyne ISCO, Lincoln, NE)
   - Elution Solvent A: dichloromethane (DCM)
   - Elution Solvent B: methanol (CH₃OH)
   - Column: FLASHPURE^{™} EXOFLEX^{™} Silica, 24 g, 40 µm irregular, 60 Å pore size, preparative column (Büchi, Switzerland)
   - Elution Condition: Elution for 15 Column Volumes (CV) at a flow rate of 32 mL/min while varying the concentration of Solvent B in the following pattern: 0 % (3 CV) → 10 % (5 CV) → 10 % (2 CV) → 100 % (2 CV) → 100 % (3 CV)
(10)Condition 5 for Automated Flash Column Chromatography on COMBIFLASH^{™} NextGen 300+ (Teledyne ISCO, Lincoln, NE)
   - Elution Solvent A: 0.1 % trifluoroacetic acid (TFA)/water
   - Elution Solvent B: 0.1 % trifluoroacetic acid (TFA)/CH₃CN
   - Column: FLASHPURE^{™} EXOFLEX^{™} C18, 20 g, 50 µm spherical, 100 Å pore diameter, preparative column (Büchi, Switzerland)
   - Elution Condition: Elution for 13 Column Volumes (CV) at a flow rate of 40 mL/min while varying the concentration of Solvent B in the following pattern: 10 % (3 CV) → 55 % (3 CV) → 30 % (4 CV) → 100 % (1 CV) → 100 % (2 CV)

### Preparation Example 3b. 1 Preparation of N-cyclohexyl-5-(3-fluorophenyl)-3-ureidothiophene-2-carboxamide

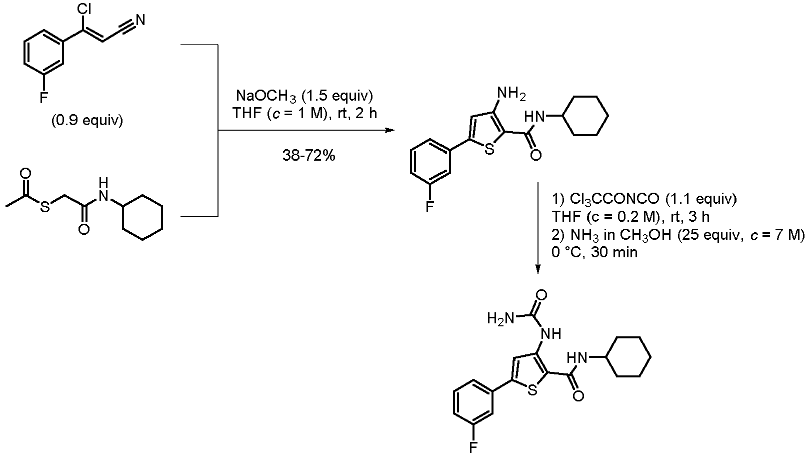

### Step 1. Preparation of 3-amino-N-cyclohexyl-5-(3-fluorophenyl)-thiophene-2-carboxamide

To an oven-dried one-necked round bottom flask a solution of *S*-(2-(cyclohexylamino)-2-oxoethyl) ethanethioate (C₁₀H₁₇NO₂S, 215.32 g/mol, 1500 mg, 6.97 mmol, 1 equiv.) in tetrahydrofuran (THF, C₄H₈O, *c* = 1 M, 7.0 mL) was added under an argon stream at room temperature. After this, solid 3-chloro-3-(3-fluorophenyl)acrylonitrile (C₉H₅ClFN, 181.60 g/mol, 1138.6 mg, 6.27 mmol, 0.9 equiv.) was added in one portion before dropwise addition of a sodium methoxide solution (NaOCH₃, 54.02 g/mol, w = 30% in CH₃OH, 0.97 g/mL, 564.5 mg, 1.9 mL, 3.8 mmol, 1.5 equiv.). Upon completion of addition the reaction mixture turned into a dark red solution, which was stirred at rt for 2 h. When TLC indicated full consumption of all starting materials, the solvent was removed under reduced pressure and the residue was purified by Condition 1a for Automated Flash Column Chromatography using the COMBIFLASH^{™} NextGen 300+ (Teledyne ISCO, Lincoln, NE). The obtained product 3-amino-*N*-cyclohexyl-5-(3-fluorophenyl)-thiophene-2-carboxamide (C₂₁H₂₆FN₃O₃S, 419.52 g/mol, 668 mg, 1.6 mmol, 63 %) was isolated as a brown solid with good yield and sufficient purity for the next step.

**HPLC-MS (ESI-MS):** Calculated: 318.41 for C₂₁H₂₆FN₃O₃S [M+H⁺]. Found: 319.5. ***t*_{R}** = 8.4 min (HPLC Condition 1).

### Step 2. Preparation of tert-butyl (S)-3-(5-(3-fluorophenyl)-3-ureidothiophene-2-carboxamido)piperidine-1-carboxylate

To an oven-dried one-necked round bottom flask a solution of 3-amino-*N*-cyclohexyl-5-(3-fluorophenyl)-thiophene-2-carboxamide (C₁₇H₁₉FN₂OS, 318.41 g/mol, 1361.7 mg, 4.28 mmol, 1 equiv.) in tetrahydrofuran (THF, C₄H₈O, *c* = 0.2 M, 20 mL) was added under an argon stream at room temperature. After this, trichloroacetyl isocyanate (TCANCO, C₃Cl₃NO₂, 188.40 g/mol, 1.2 g/mL, 886.3 mg, 561 µL, 4.7 mmol, 1.1 equiv.) was added dropwise, maintaining the temperature between 20 °C to 30 °C. The solution was then stirred at rt for 3 hours, whereas after 20 minutes the solution turned into a suspension, and a white precipitate formed. After complete consumption of the starting material, indicated by TLC, the solvent was removed under reduced pressure and the resulting precipitate was cooled to 0 °C before being taken into a methanolic ammonia solution (NH₃, 17.03 g/mol, *c* = 7 M in CH₃OH, 1814.6 mg, 21.5 mL, 106.5 mmol, 25 equiv.). The resulting solution was stirred for 30 minutes, and the solvent was removed under reduced pressure. After purification of the crude product by Condition 2a for Automated Flash Column Chromatography using the COMBIFLASH^{™} NextGen 300+ (Teledyne ISCO, Lincoln, NE) *N*-cyclohexyl-5-(3-fluorophenyl)-3-ureidothiophene-2-carboxamide (C₁₈H₂₀FN₃O₂S, 361.44 g/mol, 1536.1 mg, 4.25 mmol, 100 %) could be obtained as a white solid in quantitative yield and high purity over two steps.

**¹H NMR** (400 MHz, DMSO-d₆) δ = 10.09 (s, 1H), 8.28 (s, 1H), 7.91 (appd, *J* = 7.83 Hz, 1H), 7.39 - 7.58 (m, 3H), 7.23 (dt, *J* = 1.50, 8.00 Hz, 1H), 6.63 (br. s., 2H), 3.72 (tq, *J* = 4.40, 8.50 Hz, 1H), 1.77 (appdd, *J* = 12.47, 24.60 Hz, 3H), 1.60 (appd, *J* = 12.59 Hz, 1H), 1.31 (appspt, *J* = 12.00 Hz, 4H), 1.04 - 1.18 (m, 1H). **HPLC-MS (ESI-MS):** Calculated: 361.13 for C₁₈H₂₀FN₃O₂S [M+H⁺], Found: [M+Na⁺] 384.0; 362.0. ***t*_{R}** = 7.6 min (HPLC Condition 1).

### Preparation Example 3b.2: Preparation of (5-(3-fluorophenyl)-N-(pyridin-4-yl)-3-ureidothiophene-2-carboxamide

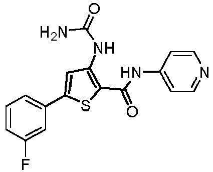

A target compound was obtained by repeating the same procedure as in Preparation Method 3b, except that in Step 1 of Preparation Example 3b.1, *S*-(2-oxo-2-(pyridin-4-ylamino)ethyl) ethanethioate (C₉H₁₀N₂O₂S, 210.26 g/mol, 1390 mg, 6.61 mmol, 1 equiv.) was used instead of *tert*-butyl (*S*)-3-(2-(acetylthio)acetamido) piperidine-1-carboxylate. The intermediate 3-amino-5-(3-fluorophenyl)-*N*-(pyridin-4-yl)thiophene-2-carboxamide (C₂₁H₂₆FN₃O₃S, 419.52 g/mol, 552.9 mg, 1.32 mmol, 1 equiv.) from Step 1 was purified using Condition 4a for Automated Flash Column Chromatography on COMBIFLASH^{™} NextGen 300+ (Teledyne ISCO, Lincoln, NE). The final product (5-(3-fluorophenyl)-*N*-(pyridin-4-yl)-3-ureidothiophene-2-carboxamide (C₁₇H₁₃FN₄O₂S, 356.38 g/mol, 602.3 mg, 1.69 mmol, 26 % (over 2 steps)) was obtained as a yellow-orange solid with high purity and moderate yield over 2 steps after purification by Condition 5 for Automated Flash Column Chromatography on COMBIFLASH^{™} NextGen 300+.

**¹H NMR** (600 MHz, DMSO-d₆) δ = 10.25 (s, 1H), 9.85 (s, 1H), 8.47 (appd, *J* = 6.10 Hz, 2H), 8.38 (s, 1H), 7.74 (dd, *J* = 1.40, 5.30 Hz, 1H), 7.48 - 7.56 (m, 3H), 7.24 - 7.32 (m, 1H), 6.79 (br. s., 2H).

**HPLC-MS (ESI-MS):** Calculated: 356.07 for C₁₇H₁₃FN₄O₂S [M+H⁺], Found: 357.1. ***t*_{R}** = 4.8 min (HPLC Condition 1).

### Preparation Example 3b.3: Preparation of 5-(3-fluorophenyl)-N-(5-methylpyridin-2-yl)-3-ureidothiophene-2-carboxamide

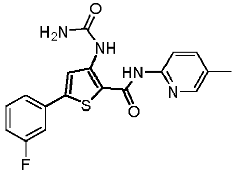

A target compound was obtained by repeating the same procedure as in Preparation Method 3b, except that in Step 1 of Preparation Example 3b.1, *S*-(2-((5-methylpyridin-2-yl)amino)-2-oxoethyl) ethanethioate (C₁₀H₁₂N₂O₂S, 224.28 g/mol, 550 mg, 2.45 mmol, 1 equiv.) was used instead of *tert*-butyl (*S*)-3-(2-(acetylthio)acetamido) piperidine-1-carboxylate. The intermediate 3-amino-5-(3-fluorophenyl)-*N*-(5-methylpyridin-2-yl)thiophene-2-carboxamide (C₁₇H₁₄FN₃OS, 327.38 g/mol, 413.1 mg, 1.26 mmol, 1 equiv.) from Step 1 was purified using Condition 1a for Automated Flash Column Chromatography on COMBIFLASH^{™} NextGen 300+ (Teledyne ISCO, Lincoln, NE). The final product 5-(3-fluorophenyl)-*N*-(5-methylpyridin-2-yl)-3-ureidothiophene-2-carboxamide (C₁₈H₁₅FN₄O₂S, 370.40 g/mol, 463.3 mg, 1.25 mmol, 51 % (over 3 steps)) was obtained as a yellow solid with high purity and moderate yield over 3 steps after purification by Condition 2b for Automated Flash Column Chromatography on COMBIFLASH^{™} NextGen 300+.

**¹H NMR** (500 MHz, DMSO-d₆) δ = 10.17 (s, 1H), 9.83 (app br. s., 1H), 8.31 (s, 1H), 8.22 (app br. s., 1H), 7.92 (d, *J* = 8.39 Hz, 1H), 7.66 (dd, *J* = 1.98, 8.54 Hz, 2H), 7.46 - 7.55 (m, 3H), 7.27 (ddt, *J* = 1.00, 2.60, 8.20 Hz, 1H), 6.60 - 6.86 (m, 2H), 2.28 (s, 3H).

**HPLC-MS (ESI-MS):** Calculated: 370.09 for C₁₈H₁₅FN₄O₂S [M+H⁺], Found: 371.0. ***t*_{R}** = 5.2 min (HPLC Condition 1).

### Preparation Example 3b.4: Preparation of 5-(3-fluorophenyl)-N-(pyrazin-2-yl)-3-ureidothiophene-2-carboxamide

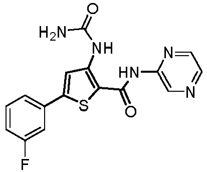

A target compound was obtained by repeating the same procedure as in Preparation Method 3b, except that in Step 1 of Preparation Example 3b.1, *S*-(2-oxo-2-(pyrazin-2-ylamino)ethyl) ethanethioate (C₈H₉N₃O₂S, 211.24 g/mol, 528.1 mg, 2.50 mmol, 1 equiv.) was used instead of *tert*-butyl (*S*)-3-(2-(acetylthio)acetamido) piperidine-1-carboxylate. The intermediate 3-amino-5-(3-fluorophenyl)-*N*-(pyrazin-2-yl)thiophene-2-carboxamide (C₁₅H₁₁FN₄OS, 313.34 g/mol, 286.2 mg, 0.91 mmol, 1 equiv.) from Step 1 was purified using Condition 2b for Automated Flash Column Chromatography on COMBIFLASH^{™} NextGen 300+ (Teledyne ISCO, Lincoln, NE). The final product 5-(3-fluorophenyl)-*N*-(pyrazin-2-yl)-3-ureidothiophene-2-carboxamide (C₁₆H₁₂FN₅O₂S, 357.36 g/mol, 318.5 mg, 0.89 mmol, 36 % (over 2 steps)) was obtained as a dumpy yellow solid with high purity and moderate yield over 2 steps after purification by Condition 5 for Automated Flash Column Chromatography on COMBIFLASH^{™} NextGen 300+.

**¹H NMR** (600 MHz, DMSO-d₆) δ = 10.72 (s, 1H), 9.82 (s, 1H), 9.26 (d, *J* = 1.38 Hz, 1H), 8.47 (dd, *J* = 1.54, 2.45 Hz, 1H), 8.40 (d, *J* = 2.52 Hz, 1H), 8.35 (s, 1H), 7.44 - 7.63 (m, 4H), 7.28 (ddt, *J* = 1.40, 2.40, 8.40 Hz, 1H), 6.76 (br. s., 2H).

**HPLC-MS (ESI-MS):** Calculated: 357.07 for C₁₆H₁₂FN₅O₂S [M+H⁺], Found: [M+Na⁺] 380.1; 358.1. ***t*_{R}** = 6.0 min (HPLC Condition 1).

### Preparation Method 4: Derivatization of 2,3-Thiophenecarboxamideureas

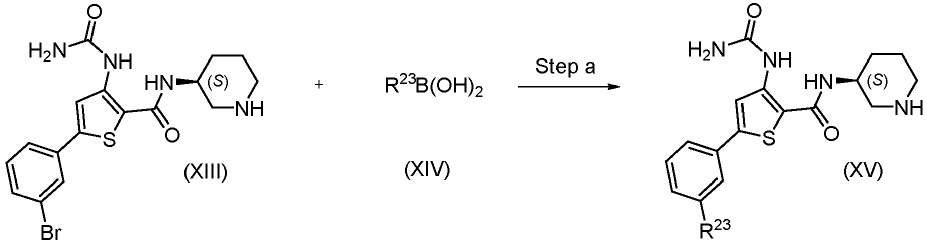

The reaction in above Step a is a process of introducing an R²³ group by allowing the compound represented by Chemical Formula (XIII) to react with the halide compound represented by Chemical Formula (XIV).
Specifically, the reaction in above Step a is performed by heating the mixture to a temperature of 120 °C under a condition in which [1,1'-bis(diphenylphosphino)-ferrocene]palladium(II)dichloride ([PdCl₂(dppf)]) is added in the presence of an inorganic base such as cesium carbonate. The reaction can be performed with a mixture of water and a typical organic solvent, like 1,4-dioxane. The mixed solvent can be used by appropriately mixing water and 1,4-dioxane within a volume ratio range of 5:1 to 1:5.

### [Preparation Examples of Method 4]

The compounds synthesized in the Examples of the present invention were purified or subjected to structural analysis under the following conditions.
(1) HPLC Condition for Structural Analysis
   - Elution Solvent A: 0.1 % trifluoroacetic acid (TFA)/water
   - Elution Solvent B: 0.1 % trifluoroacetic acid (TFA)/CH₃CN
   - Column: NUCLEODUR^{™} C18 Gravity, 3 µm, 125 × 4 mm analytical column (Macherey- Nagel, Germany)
   - Elution Condition: Elution for 9.5 minutes while varying the concentration of Solvent B from 10 to 100% at a moving rate of 1.0 mL/min.
(2) Condition 1 for Automated Flash Column Chromatography with COMBIFLASH^{™} NextGen 300+ (Teledyne ISCO, Lincoln, NE)
   - Elution Solvent A: petroleum ether 40-60 °C (PE)
   - Elution Solvent B: ethylacetate (EtOAc)
   - Column: FLASHPURE^{™} EXOFLEX^{™} Silica, 24 g, 40 µm irregular, 60 Å pore size, preparative column (Büchi, Switzerland)
   - Elution Condition: Elution for 16 Column Volumes (CV) at a flow rate of 32 mL/min while varying the concentration of Solvent B in the following pattern: 0 % (3 CV) → 60 % (5 CV) → 60 % (5 CV) → 100 % (1 CV) → 100 % (2 CV)

### Preparation Example 4.1: (S)-5-(4'-fluoro-[1,1'-biphenyl]-3-yl)-N-(piperidin-3-yl)-3-ureidothiophene-2-carboxamide

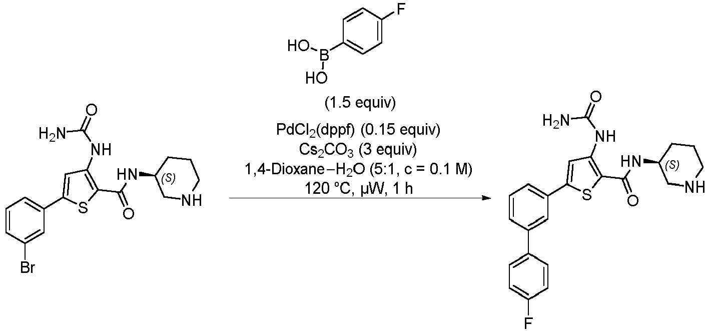

### Step 1. Preparation of (S)-5-(4'-fluoro-[1,1'-biphenyl]-3-yl)-N-(piperidin-3-yl)-3-ureidothiophene-2-carboxamide

In an oven-dried microwave vial (*S*)-5-(3-bromophenyl)-*N*-(piperidin-3-yl)-3-ureidothiophene-2-carboxamide (C₁₇H₁₉BrN₄O₂S, 423.33 g/mol, 30 mg, 0.1 mmol, 1 equiv.) and (4-fluorophenyl)boronic acid (C₆H₆BFO₂, 139.92 g/mol, 14.9 mg, 0.1 mmol, 1.5 equiv.) were dissolved in 1,4-dioxane-H₂O (5:1, *c* = 0.1 M, 1 mL). The solution was degassed with argon for 15 min before adding solid cesium carbonate (Cs₂CO₃, 325,82 g/mol, 69.5 mg, 0.2 mmol, 3 equiv.) and bis(diphenylphosphino)ferrocene]palladium(II)dichloride (PdCl₂(dppf), C₃₄H₂₈Cl₂FeP₂Pd, 731.71 g/mol, 7.4 mg, 10.6 µmol, 0.15 equiv.). The suspension was stirred 1 h at 120 °C in a microwave. After this, the cooled reaction mixture was filtered over CELITE^{™} (Sigma-Aldrich, St. Louis, MO), diluted with water and extracted with ethyl acetate (5x). The combined organic phases were dried over sodium sulfate and concentrated *in vacuo.* The crude product was purified by Condition 1 for Automated Flash Column Chromatography with COMBIFLASH^{™} NextGen 300+ (Teledyne ISCO, Lincoln, NE) to obtain the desired product (*S*)-5-(4'-fluoro-[1,1'-biphenyl]-3-yl)-*N*-(piperidin-3-yl)-3-ureidothiophene-2-carboxamide (C₂₃H₂₃FN₄O₂S, 438.52 g/mol, 19.7 mg, 61 µmol, 61 %) as white solid in moderate yield.

**¹H NMR** (DMSO-d₆ ,600MHz): δ = = 10.03 (s, 1 H), 8.34 (s, 1 H), 7.92 (d, J=7.7 Hz, 1 H), 7.82 (s, 1 H), 7.78 (dd, J=8.6, 5.5 Hz, 2 H), 7.66 (d, J=7.8 Hz, 1 H), 7.68 (d, J=7.7 Hz, 1 H), 7.55 - 7.59 (m, 1 H), 6.67 (br. s., 2 H), 3.97 (br. s., 1 H), 3.10 (d, J=11.6 Hz, 1 H), 2.98 (d, J=12.4 Hz, 1 H), 2.57 - 2.67 (m, 2 H), 1.86 (d, J=9.6 Hz, 1 H), 1.74 (d, J=9.9 Hz, 1 H), 1.55 ppm (d, J=11.6 Hz, 2 H).

**HPLC-MS (ESI-MS):** Calculated: 438.15 for C₂₃H₂₃FN₄O₂S [M+H⁺], Found: 439.2. ***t*_{R}** = 5.7 min (HPLC Condition 1).

### Preparation Example 4.2: (S)-5-(4'-(dimethylamino)-[1,1'-biphenyl]-3-yl)-N-(piperidin-3-yl)-3-ureidothiophene-2-carboxamide

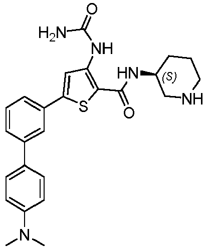

A target compound was obtained by repeating the same procedure as in Preparation Example 4.1, except that in Step 1 of Preparation Example 4.1, (4-(dimethylamino)phenyl)-boronic acid was used instead of (4-fluorophenyl)boronic acid and 0.1 equiv. of bis(diphenylphosphino)ferrocene]palladium(II)dichloride (PdCl₂(dppf) was used instead of 0.15 equiv. The product (*S*)-5-(4'-(dimethylamino)-[1,1'-biphenyl]-3-yl)-*N-*(piperidin-3-yl)-3-ureidothiophene-2-carboxamide (C₂₅H₂₉N₅O₂S, 463.6 g/mol, 20.7 mg, 68 µmol, 68 %) was obtained as white solid in good yield.

**¹H NMR** (DMSO-d₆ ,500MHz): δ = = 10.03 (s, 1 H), 8.27 (s, 1 H), 8.25 (s, 1 H), 7.93 (d, *J*=0.8 Hz, 1 H), 7.86 (d, *J*=7.8 Hz, 1 H), 7.77 (s, 1 H), 7.57 - 7.61 (m, 1 H), 7.45 (s, 1 H), 7.44 (s, 1 H), 6.64 (br. s., 2 H), 4.02 (q, *J*=7.1 Hz, 1 H), 3.88 (s, 3 H), 2.91 - 3.04 (m, 2 H), 2.52 - 2.62 (m, 2 H), 1.98 (s, 1 H), 1.71 - 1.83 (m, 2 H), 1.45 - 1.57 ppm (m, 2 H).

**HPLC-MS (ESI-MS):** Calculated: 463.20 for C₂₅H₂₉N₅O₂S [M+H⁺]. Found: 464.3. ***t*_{R}** = 4.3 min (HPLC Condition 1).

### Preparation Example 4.3 (S)-5-(3-(1-methyl-1H-pyrazol-4-yl)phenyl)-N-(piperidin-3-yl)-3-ureidothiophene-2-carboxamide

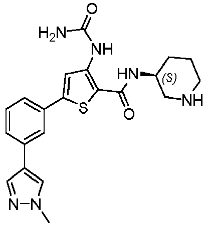

A target compound was obtained by repeating the same procedure as in Preparation Example 4.1, except that in Step 1 of Preparation Example 4.1, 1-methyl-4-(4,4,5,5-tetramethyl-1,3,2-dioxaborolan-2-yl)-1*H-*pyrazole was used instead of (4-fluorophenyl)boronic acid and 0.1 equiv. of bis(diphenylphosphino)ferrocene]palladium(II)dichloride (PdCl₂(dppf) was used instead of 0.15 equiv.. The product (*S*)-5-(3-(1-methyl-1H-pyrazol-4-yl)phenyl)-*N*-(piperidin-3-yl)-3-ureidothiophene-2-carboxamide (C₂₁H₂₄N₆O₂S, 424.52 g/mol, 10.3 mg, 31 µmol, 31 %) was obtained as white solid in low yield.

**¹H NMR** (DMSO-d₆ ,500MHz): δ = 10.11 (s, 1 H), 8.36 (s, 1 H), 7.93 (d, *J*=7.8 Hz, 1 H), 7.81 (t, *J*=1.5 Hz, 1 H), 7.67 (dt, *J*=7.4, 1.6 Hz, 1 H), 7.63 (s, 1 H), 7.61 (s, 1 H), 7.57 - 7.60 (m, 1 H), 7.56 (m, 1 H), 6.90 (s, 1 H), 6.88 (s, 1 H), 6.68 (br. S., 2 H), 3.97 (m, 1 H), 3.11 (m, 2 H), 3.02 (s, 6 H), 2.95 - 2.99 (m, 2 H), 2.80 (s, 1 H), 1.76 - 1.90 (m, 2 H), 1.53 - 1.63 ppm (m, 2 H).

**HPLC-MS (ESI-MS):** Calculated: 424.17 for C₂₁H₂₄N₆O₂S [M+H⁺]. Found: 425.2. ***t*_{R}** = 4.6 min (HPLC Condition 1).

### Example 2: Generation of isogenic sublines of KIT and PDGFRA-mutant GIST

To generate the first set of isogenic sublines of imatinib- and avapritinib-resistant GIST sublines (T1-V654A and T1-D816A), suitable guide sequences were designed using the online tool CRISPOR (Haeussler, M., et al., Evaluation of off-target and on-target scoring algorithms and integration into the guide RNA selection tool CRISPOR. Genome Biol, 2016. 17(1): p. 148). A forward oligo containing the t7 RNA polymerase promoter sequence and the respective guide sequence, as well as a reverse oligo containing the generic single guide RNA (sgRNA) sequence were purchased at MWG Eurofins. For guide RNA design "FE-modified" sequences described by Chen *et al.* (Chen, B., et al., Dynamic imaging of genomic loci in living human cells by an optimized CRISPR/Cas system. Cell, 2013. 155(7): p. 1479-91) were employed. The t7 RNA polymerase DNA template was generated by a fill-in PCR using Q5 high fidelity DNA polymerase (NEB), running 10 cycles of 62 °C / 20 sec and 72 °C / 2 min. sgRNA was then transcribed with t7 RNA polymerase (NEB), according to manufacturer's instructions and precipitated by phenol/chlorophorm extraction, using standard protocols.

GIST-T1 cells were seeded in a T25 flask at low density and after 72h of growth cells were trypsinized, washed, and resuspended in electroporation buffer (Buffer SF, Lonza). 10⁵ cells were mixed with 0.5-0.7 µl Recombinant Cas9 (20µM; NEB), 0.5-0.7 µl *in vitro* transcribed sgRNA (20 µM) per guide, and 0.5-0.7 µl single stranded DNA template (ssODN (single-stranded oligodeoxynucleotide); 500 µM), carrying the desired mutation. Cells were electroporated using the program DN100 on the Amaxa Nucleofector 4D (Lonza). 24-96 hours after electroporation, cells were treated with different KIT inhibitors at 100 nM for selective pressure of the desired mutation. After outgrowth of a resistant population, single cell clones were derived. In-cis mutations of *KIT* were confirmed by Sanger sequencing cDNA of RNA transcripts, using a mutant-allele-specific primer (FW: CAGTGGAAGGTTGATCACAAATGGG (SEQ ID NO: 18).

These cell lines carry the following nomenclature: T1-(KIT-mutation(s)). Where "T1" designates the parental cell line GIST-T1. For example, GIST-T1 with a the original KIT exon 11 mutation and a secondary mutation V654A is termed "T1-V654A", or " T1^{V654A}".

Sequences for KIT mutant sublines (T1-V654A and T1-D816A):

**Table 1: gRNA sequences**

| **Target** | **Guide sequence** | **SEQ ID NO:** |
|---|---|---|
| KIT - exon 13 - 1 | ATGAATATTGTGAATCTACT | 1 |
| KIT - exon 13 - 2 | TGTGATTACCAAGGTAACTC | 2 |
| KIT - exon 17 - 1 | AGAATCATTCTTGATGTCTC | 3 |
| KIT - exon 17 - 2 | GATTCTAATTATGTGGTTAA | 4 |

**Table 2: IVT sequences (IVT sequences = in vitro transcription sequences)**

| **Name** | **Sequence (5'->3')** | **SEQ ID NO:** |
|---|---|---|
| Generic sgRNA forward oligo I | aagcTAATACGACTCACTATA | 5 |
| Generic sgRNA forward oligo II | [N₂₀]GTTTAAGAGCTATGCTGGAAACA | 6 |
| Generic sgRNA forward oligo complete | SEQ ID NO: 5 - G_{[0-2]} - SEQ ID NO: 6 | |
| Generic sgRNA reverse oligo | AAAAAAGCACCGACTCGGTGCCACTTTTTCAAGTTGATAACGGACTAGCC TTATTTAAACTTGCTATGCTGTTTCCAGCATAGCTCTTAAAC | 7 |

| | | |
|---|---|---|
| IVT-gRNA sequences are designed to start with GG, irrespective of their sequence, to initiate robust T7-transcription. N = specific guide sequence (see above). | | |

**Table 3: ssODN sequences**

| **Target** | **ssODN sequence (5'→3')** | **SEQ ID NO:** |
|---|---|---|
| KIT exon 13 | ACAGACAATAAAAGGCAGCTTGGACACGGCTTTACCTCCAATGGTGCAGGCT CCGAGAAGATTCGCAATATTCATGTGATTACCAAGGTAACTCAGGACTTTGA*G | 8 |
| KIT exon 17 | GCCAGAAATATCCTCCTTACTCATGGTCGGATCACAAAGATTTGTGATTTTGGT CTAGCAAGGGCCATCAAGAATGATTCTAATTATGTGGTTAAAGGAAACGT*G | 9 |

Further, cell lines with endogenous PDGFRA mutations (T1α^{D842V}, T1α^{D842V/G652E/V658A}, T1α^{D842V/T6701}, T1α^{D842V/G680R}) were generated by CRISPR/Cas9 mediated gene editing, as described above (Mühlenberg, T., et al., KIT-Dependent and KIT-Independent Genomic Heterogeneity of Resistance in Gastrointestinal Stromal Tumors - TORC1/2 Inhibition as Salvage Strategy. Mol Cancer Ther, 2019. 18(11): p. 1985-1996), with the following deviations: recombinant Cas9 from IDT Labs (60 µM) was used in a 1:2 ratio with specific single guide RNA (sgRNA; IDT). First, PDGFRA^{D842V} was introduced into parental GIST-T1 and cells were selected with IM 200 nM until outgrowth of a resistant population, which carried heterozygous PDGFRA^{D842V}. In a second round of editing, one sgRNA specific for *PDGFRA* resistance mutations (exons 14 and 15) and an ssODN template to introduce the desired mutation (G652E/V658A, T674I, and G680R, respectively) was combined with a guide targeting exon 1 (for G652E/V658A, T674I, and G680R) or mutant exon 11 (for D842V-only) of KIT. Cells were then treated with avapritinib 100 nM and after outgrowth of a resistant population, single cell clones were derived. Heterozygous D842V and in-cis secondary mutations as well as frameshift mutations in mutant exon 1 and 11 of KIT were confirmed by sanger sequencing and/or next generation panel sequencing.

These cell lines carry the following nomenclature: T1-α/a-(PDGFRA-mutaion(s)). Where "T1" designates the parental cell line GIST-T1, "α" or "a" designate that these cells are driven not by KIT but by PDGFRA. For example, a PDGFRA-driven subline of GIST-T1 with a PDFRA-D842V mutation is termed "T1-α-D842V", or "T1-α-D842V", or " T1α^{D842V}". If the cell line carries a further mutation, e.g. G680R, it is termed "T1-α-D842V-G680R".

**Table 4: gRNA sequences (PDGFRA-isogenic sublines)**

| **Target** | **Guide sequence** | **SEQ ID NO:** |
|---|---|---|
| PDGFRA - exon 18 | CGAATCATGCATGATGTCTC | 10 |
| PDGFRA - exon 14 | TGAAGATAATGACTCACCTG | 11 |
| PDGFRA - exon 15 | ATCACAGAGTATTGCTTCTA | 12 |
| KIT - exon 1 | ACCGCGATGAGAGGCGCTCG | 13 |

**Table 5: ssODN sequences**

| **Target** | **ssODN sequence (5'→3')** | **SEQ ID NO:** |
|---|---|---|
| PDGFRA D842V | TCCTCCTGGCACAAGGAAAAATTGTGAAGATCTGTGACTTTGGCCTGGCA AGAGTCATCATGCATGATTCGAACTATGTGTCGAAAGGCAGTGT*A | 14 |
| PDGFRA G652E+V658A | CAAGCTCTCATGTCTGAACTGAAGATAATGACTCACCTGGAGCCACATTTA AACATTGCAAACTTGCTGGGAGCCTGCACCAAGTCAGGTGGGCTCACT*G | 15 |
| PDGFRA T674I | CTTCATTTTCATACCCATCTCCTAACGGCTTTTGTCCCCATAGGGCCGATTT ACATCATCATAGAGTATTGCTTCTATGGAGATTTGGTCAACTATTTGCATAA*G | 16 |
| PDGFRA G680R | GTCCCCATAGGCCCCATTTACATCATCACAGAGTATTGCTTCTATAGAGATT TAGTCAACTATTTGCATAAGAATAGGGATAGCTTCCTGAGCCACCA*C | 17 |

| | | |
|---|---|---|
| *** = PTO (Phosphorothioate) base link** | | |

Using the method described above for generation of PDGFRA-driven GIST cell lines, further KIT-mutant cell lines were generated. These carry, additional secondary mutations (see Table 6) and tertiary mutations (also called AP/AL mutations). These cell lines carry the following nomenclature: T1-(KIT-mutation(s)). Where "T1" designates the parental cell line GIST-T1. For example, GIST-T1 with a the original KIT exon 11 mutation and a secondary mutation N822K is termed "T1-N822K", or " T1^{N822K}". If the cell line harbors a third (AP/AL) mutation it will be termed "T1-V654A-N822K" or "T1-V654A+N822K".

To generate the cell line T1-AY with a "primary" mutation in exon 9, the 57bp deleted in parental GIST-T1 was re-inserted to reconstitute the e11-WT genotype, while inserting the 502_503AYdup mutation. If the cell line harbors an additional KIT mutation, e.g. N822K, it will be termed "T1-AY-N822K" or "T1-AY+N822K".

**Table 6: List of gene editing oligo-nucleotides II**

| **KIT mutation** | **sgRNA** | **SEQ ID NO:** | **ssODN sequence (5'→3')** | **SEQ ID NO:** |
|---|---|---|---|---|
| AYdup | TGTAAGGCTT ACAACGATGT | 19 | A*G*TGCATTCAAGCACAATGGCACGGTTGAA TGTAAGGCTTACAAtGAcGTGGGCAAGACTT CTGCCTATgcctatTTTAACTTTGCATTTAAAGGT AACAACAAAGGTATATTTCTT*T*T | 20 |
| e11 "repair" | GTGGAAGGTT GATCACAAAT | 21 | T*T*TCCCTTTCTCCCCACAGAAACCCATGTAT GAAGTACAGTGGAAGGTTGTTGAGGAGATAA ACGGgAAtAATTATGTTTACATAGACCCAACAC AACTTCCTTATGATCACAAATGGGAGTTTCCC AGAAACAGGCTGAGTTTTGGTCAGTA*T*G | 22 |
| V654A | ATGAATATTGT GAATCTACT | 23 | C*TCAAAGTCCTGAGTTACCTTGGTAATCACAT GAATATTGcGAAcCTtCTTGGAGCCTGCACCA TTGGAGGTAAAGCCGTGTCCAA*G*C | 24 |
| C809G | AGAATCATTCT TGATGTCTC | 25 | C*AGAAATATCCTCCTTACTCATGGTCGGATCA CAAAGATTgGTGATTTTGGTCTAGCtAGAGACA TCAAGAATGATTCTAATTATGTGGTTAAAGGAA AC*G*T | 26 |
| D820A | CAAGAATGAT TCTAATTATG | 27 | A*AGATTTGTGATTTTGGTCTAGCCAGAGACAT CAAGAATGcTTCTAATTATGTcGTTAAAGGAAA CGTGAGTACCCATTCTCTGCTTGACAG*T*C | 28 |
| D820Y | CAAGAATGAT TCTAATTATG | 27 | CACAAAGATTTGTGATTTTGGTCTAGCCAGAG ACATCAAGAATtATTCTAATTATGTcGTTAAAGG AAACGTGAGTACCCATTCTCTGCTTGACAGTC CT*G | 29 |
| N822K | CAAGAATGAT TCTAATTATG | 27 | TACTCATGGTCGGATCACAAAGATTTGTGATTT TGGTCTAGCCAGAGACATCAAGAATGATTCTA AgTATGTGGTTAAAGGAAACGTGAGTACCCAT TCTCTGCTTGAC*A | 30 |

| | | | | |
|---|---|---|---|---|
| *** = phosphorothioate (PTO) bond; lowercase letter = mutation** | | | | |

List of cell lines used for validation assays:

**Table 7: Additional cell lines used in this study.**

| Cell Line | Tumor entity | Driver gene | Mutation(s) |
|---|---|---|---|
| GIST-T1 | GIST | KIT | KIT 560-578de! |
| T1-AYdup | GIST | KIT | KIT-502_503AYdup |
| GIST430 | GIST | KIT | KIT-560-576del |
| GIST430/654 | GIST | KIT | KIT-560-576del + V654A |
| GIST882 | GIST | KIT | KIT-K642E |
| GIST48B | GIST | KIT | V560D+D820A (KIT negative = no protein expression of the KIT mutant) |
| H1781 | lung adenocarcinoma | HER2 | G776delinsVC |
| H1975 | Lung adenocarcinoma | EGFR | L858R + T790M |
| A431 | squamous cell carcinoma | EGFR | overexpression |
| A549 | Lung adenocarcinoma | KRAS | G12S |
| MF-SN1 | Myxofibrosarcoma | none | Complex karyotype |
| LPS141 | Liposarcoma | None | MDM2 amplification |
| LPS853 | Liposarcoma | None | MDM2 amplification |
| SKLMS-1 | Leiomyosarcoma | none | Complex karyotype |
| LMS03 | Leiomyosarcoma | none | Complex karyotype |
| LMS04 | Leiomyosarcoma | none | Complex karyotype |
| LMS05 | Leiomyosarcoma | none | Complex karyotype |

Further information including references about all of these cell lines can be found in the online cell line database www.celloraurus.org .

### Example 3: Cell viability assay

Cell viability assays were performed as previously described (Grunewald, S., *et al., Resistance to avapritinib in PDGFRA-driven GIST is caused by secondary mutations in the PDGFRA kinase domain.* Cancer Discov, 2020). To generate the results presented in Figures 2 and 3, cells were plated at 4000-16000 cells/well in a 96-well plate and cultured overnight. Cells were then incubated in triplicates with media containing inhibitors or solvent control (DMSO) for 72 hours. The sulforhodamine B (SRB) assay was used according to the method of Skehan and colleagues (Skehan, P., et al., New colorimetric cytotoxicity assay for anticancer-drug screening. J.Natl.Cancer Inst., 1990. 82(13): p. 1107-1112).

To generate the results in for the KIT-driven cell lines (GIST-T1; T1-D816E, and T1-V654A) in Figures 1, 6, 7, and 8, cells were plated in triplicates at 400 cells/well in a 384-well plate with media containing inhibitors or solvent control (DMSO) and incubated for 72 hours. The Cell Titer Glo assay (Promega) was applied according to manufacturer's instructions. Absorption or luminescence was measured with a SPARK^{™} Plate reader (Tecan, Switzerland). One measurement of untreated cells at the at the start of treatment served as baseline for growth reduction (GR) calculations. Calculation of GR50 values and curve fitting was performed using the R-package GRmetrics (Hafner, M., et al., Growth rate inhibition metrics correct for confounders in measuring sensitivity to cancer drugs. Nat Methods, 2016. 13(6): p. 521-7), using the mean of >2 independent experiments.

For high-throughput experiments, performed with PDGFRA-driven cell lines (T1-a-D842V; T1-a-D842V+G680R; T1-a-D842V+G652E/V658A) and the KIT/PDGFRA negative cell line GIST48B, as depicted in Figures 1, 6-11, cells were plated at 400 cells/well in a 384-well plate and cultured overnight. Cells were then incubated in triplicates with media containing inhibitors or solvent control (DMSO) for 144 hours. The Cell Titer Glo assay (Promega) was applied according to manufacturer's instructions. Luminescence was measured with an Envision plate reader (PerkinElmer). One measurement of untreated cells at the at the start of treatment served as baseline for growth reduction (GR) calculations. Calculation of GR50 values and curve fitting was performed using quattro workflow (Quattro Research GmbH). Results are shown in Figures 1-3 and 6-11.

### Example 4: in vivo studies

For in vivo studies nude mice were injected in each flank with 1 × 10⁶ cells of the cell lines T1-α-D842V or T1-α-D842V-G652E/V658A. Drug treatments were started after a palpable tumor had formed. Mice were then treated daily by with either AZD7762 (5mg/kg or 10mg/kg) or vehicle control by intraperitoneal injection (IP), or avapritinib (30mg/kg) by oral gavage (PO), for 13 days. Tumor size was measured every other day by caliper and tumor ratio (as a mearue for tumor volume) was calculated by the formula - 0.5 × Length × Width², and normalized to start of treatment (Day0). The results are shown in Figure 4.

Three hours after last treatment mice were sacrificed and tumors were collected. Tumors were homogenized in lysis buffer (1 % NP-40, 50 mmol/L tris(hydroxymethyl)aminomethane (Tris) HCl pH 8.0,100 mmol/L sodium fluoride, 30 mmol/L sodium pyrophosphate, 2 mmol/L sodium molybdate, 5 mmol/L ethylenediaminetetraacetic acid (EDTA) and 2 mmol/L sodium vanadate; freshly adding 0.1 % 10 mg/mL aprotinin and leupeptin as well as 1 % 100 mmol/L PMSF and200 mmol/L sodium vanadate) and then lysed while rotating for 1 hour at 4 °C. Lysates were centrifuged at 4 °C for 30 minutes at 18,000 rcf and protein concentration was determined using the Bio-Rad Protein Assay (Bio-Rad Laboratories). Protein concentration was adjusted to 2 mg/mL, sodium dodecyl sulfate (SDS) loading buffer (Tris-HCl pH 6.7, 10 % SDS, 2.5 % dithiothreitol (DTT), 50 % glycerol, and 0.05 % bromophenol blue) was added and lysates were incubated for 5 minutes at 95°C. Equal amounts of protein (30 µg) were separated on SDS-PAGE Gels (NuPAGE 4 % - 12 %; Life Technologies) and blotted onto nitrocellulose-membranes (GE Healthcare/Amersham-Biosciences). After blocking with Net-G buffer (1.5 M NaCl, 50 mmol/L EDTA, 500 mmol/L Tris, 0.5% Tween 20, and 0.4% gelatine), membranes were incubated at 4 °C overnight with the respective primary antibody. After washing (Net-G), membranes were incubated for 2 hours at room temperature with a secondary antibody (in Net-G) and washed again. Changes in protein expression and phosphorylation as visualized by chemiluminescence were captured and quantified using a FUJI LAS3000system with Science Lab 2001 ImageGauge 4.0 software (FujifilmMedial Systems). b-Actin served as loading control for each membrane (Mühlenberg, T., *et al.,* supra). Antibodies against total PDGFRA and phosphorylated PDGFRA were used ((Cell Signaling Technologies). The results are shown in Figure 12.

### Example 5: HTRF assay

All supplies for the KIT and PDGFRA HTRF assay kit were purchased from CisBio (Bagnols-sur-Cez̋e, France). Active enzymes were purchased from ProQinase (KIT-wt (#0997-0000-1 (010)), KIT-D816H (#1041-0000-1 (002), PDGFRA-wt (#1057-0000-1 (011)), SignalChem (PDGFRA-G680R, #P12-12DG, Lot#X3408-18) and Invitrogen (PDGFRA-D842V (PV4203, #2343231B)). Small volume (25 µL fill volume) white round-bottom 384-well plates were obtained from Greiner Bio-One GmbH (Solingen, Germany).

The biochemical half maximal inhibitory concentrations (IC₅₀) were determined with the TK HTRF KinEASE assay (Cisbio) according to the manufactor's instructions. 5 µL Kinase solution (KIT-wt: 2.53 ng/well, KIT-D816H: 2 ng/well, PDGFRA-wt: 1.68 ng/well, PDGFRA-G680R: 0.38 ng/well, PDGFRA-D842V: 0.0421 ng/well) and 2.5 µL inhibitor solution (8 % DMSO in HTRF buffer) were incubated for 30 min before the reaction was started by addition of 2.5 µL starting solution containing ATP and substrate peptide. ATP concentrations were set at their respective *K*_{M} values (200 µM for KIT-wt, 12 µM for KIT-D816H, 57 µM for PDGFRA-wt, 11 µM PDGFRA-G680R, 14 µM for PDGFRA-D842V). The following substrate concentrations were used: 550 nM for KIT-wt, 1 µM for KIT-D816H, 455 nM for PDGFRA-wt, 886 nM for PDGFRA-G680R and 775 nM for PDGFRA-D842V. After reaction completion (KIT-wt: 20 min, KIT-D816H: 30 min, PDGFRA-wt: 40 min, PDGFRA-G680R: 15 min, PDGFRA-D842V: 15 min), 10 µL of stop solution were added. The fluorescence resonance energy transfer (FRET) signal was measured with an ENVISION^{™} plate reader (PerkinElmer, Waltham, MA, US) (λ ex 620 nm/ *λ* em 665 nm). The quotient of both intensities was recorded at 8 different inhibitor concentrations and data fit to a Hill 4-parameter equation with Quattro software suite (Quattro Research GmbH, Martinsried, Germany). Each reaction was performed in duplicates and at least three independent determinations of each IC₅₀ were made.

The establishment of the HTRF^{®} assay system was carried out following the manufacturer's instructions from PerkinElmer (formerly CisBio) and included the following steps: SEB titration, enzyme titration, enzyme kinetics, substrate and ATP titration, and optimization of the biotin-streptavidin ratio. Both the establishment steps and data collection were performed in white 384-well microplates, each with a capacity of 25 µL (Greiner BioOne GmbH). Liquid transfers were executed using a MultiDropTM Combi Reagent Dispenser (Thermo Fisher Scientific), while ligands dissolved in DMSO were transferred using the Echo 520 Liquid Handler (Labcyte Inc.). Data were read using the microplate reader EnVision 2104 (PerkinElmer). As a low kinase activity was observed during initial establishment attempts, SEB titration was omitted, and 50 nM SEB was directly added to each reaction buffer (excluding KIT-D816H). In principle, both assay establishment and subsequent determination of IC50 values were carried out in predefined buffer systems **(Table 8).**

**Table 8: Composition of the reaction and detection buffers used in the HTRF assay for kinase, substrate, ATP, and fluorophore solutions.**

| | |
|---|---|
| **Reaction buffer** | 5 mM MgCl₂, 1 mM DTT, 0,0001 % TritonX, 50 mM Hepes (pH 7), 40 mM Na₃VO₄, 0,02 % NaN₃, 0,01 % BSA |
| **Detection buffer** | 25 mM Hepes (pH 7), 0,05 % BSA, 0,4 M KF, 10 mM EDTA |

For the determination of IC₅₀ values for various ligands, unless otherwise specified, three independent measurements in duplicate were routinely carried out. The obtained results were presented as means accompanied by corresponding standard deviations. Data analysis was executed using Quattro Software Suite 11.2.0.3 (Quattro Research GmbH). Results are shown in Figures 1, 6-11.

In this procedure, the kinases (5 µL) were incubated with the investigational inhibitors (in 2.5 µL of reaction buffer) at eight distinct concentrations, with each concentration being tested in duplicate, for a duration of 30 minutes prior to the addition of the starting solution (2.5 µL). The starting solution (2.5 µL) encompassed substrate and ATP concentrations as determined during the assay establishment for each respective kinase. Following the predetermined reaction time from the enzyme kinetics phase, the reactions were halted by the addition of the stop solution (10 µL) and allowed to incubate for 60 minutes before measurement. The FRET signal was quantified employing an EnVision plate reader (PerkinElmer, Waltham, MA, USA) set at Em_{665 nm}/Em_{620 nm}. The intensity ratio of both emissions was recorded at eight distinct inhibitor concentrations, and the data were fitted to a 4-parameter Hill equation utilizing the Quattro Software Suite (Quattro Research GmbH, Martinsried, Germany). Each reaction was conducted in duplicate, and a minimum of three independent determinations were performed for each IC₅₀ value.

All details regarding the utilized kinases are documented in Table 9.

**Table 9: Parameters Utilized for Conducting the HTRF^{®} TK KinEASE Assays for Various Kinases to Determine IC50 Values.**

| **Kinase** | **KIT-wt** | **KIT-D816H** | **PDGFRA-wt** | **PDGFRA-G680R** | **PDGFRA-D842V** |
|---|---|---|---|---|---|
| Supplier | Proqinase | Proqinase | Proqinase | SignalChem | Invitrogen |
| Lot | 0997-0000-1 (010) | 1041-00001 (002) | 1057-0000-1 (011) | X3408-18 | 2343231B |
| Enzyme | 2,53 ng / *well* | 2 ng/*well* | 1,68 ng / *well* | 0,38 ng / *well* | 0,0421 ng / *we ll* |
| Substrate | 550 nM | 1 µM | 455 nM | 886 nM | 775 nM |
| ATP | 200 µM | 12 µM | 57 µM | 11 µM | 14 µM |
| Biotin-XL665 | 8:1 | 8:1 | 8:1 | 8:1 | 8:1 |
| Preincubation | 30 min | 30 min | 30 min | 30 min | 30 min |
| Reaction | 20 min | 30 min | 40 min | 15 min | 15 min |
| Stop | 60 min | 60 min | 60 min | 60 min | 60 min |

## Claims

1. Compound of formula (I) or a pharmaceutically acceptable salt thereof for for use in the treatment of a gastrointestinal stromal tumor (GIST) in a patient, 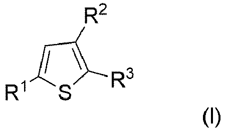 wherein
R¹ is selected from aryl and heterocyclyl, provided R¹ is not thienyl, and wherein R¹ can be optionally substituted on one or more carbon atoms by one or more R⁶; and further wherein if said heterocyclyl contains an -NH- moiety, the nitrogen of said moiety can be optionally substituted by a group selected from R⁷;
R² is -NHC(=O)NH₂;
R³ is -C(=O)NR⁴R⁵;
R⁴ is cycloalkyl or a 5, 6, or 7-membered heterocyclyl ring containing at least one nitrogen atom, wherein R⁴ may be optionally substituted on one or more carbon atoms by one or more R⁶, and wherein if said heterocyclyl contains a -NH- moiety, the nitrogen of said moiety may be optionally substituted by a group selected from R⁷;
R⁵ is H or taken together with R⁴ and the nitrogen atom to which it is attached form a heterocyclic ring, wherein said heterocyclic ring is selected from the group consisting of 4-, 5-, 6-, and 7-membered heterocyclic rings, wherein said heterocyclic ring may be optionally substituted on one or more carbon atoms by one or more R⁶, and wherein if said heterocyclyl contains a -NH- moiety, the nitrogen of said moiety may be optionally substituted by a group selected from R⁷;
each R⁶ and R⁷ is independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, - OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHSO₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁸R⁹, mercapto, -S(C₁₋₆ alkyl), -SO(C₁₋₆ alkyl), -SO₂(C₁₋₆ alkyl), -SO₂-NR⁸R⁹, wherein R⁶ and R⁷ independently of each other can be optionally substituted on one or more carbon atoms by one or more R¹⁰ and on nitrogen of any moiety that contains an NH or NH₂ by R¹¹; R⁸ and R⁹ are each independently selected from halo, nitro, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -CHO, -C(=O)NR¹²R¹³, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR¹²R¹³, mercapto, -S(C₁₋₆ alkyl), -SO(C₁₋₆ alkyl), -SO₂(C₁₋₆ alkyl), -SO₂-NR¹²R¹³;
R¹⁰ and R¹¹ are independently selected from halo, nitro, -NR⁸R⁹, cyano, isocyano, C₁₋₆ alkyl, C₂₋₆ alkenyl, C₂₋₆ alkynyl, aryl, cycloalkyl, heterocyclyl, hydroxy, oxo(=O), -O(C₁₋₆ alkyl), -Oaryl, -OC(=O)alkyl, -NHCHO, N(C₁₋₆ alkyl)CHO, -NHC(=O)NR⁸R⁹, -N(C₁₋₆ alkyl)C(=O)NR⁸R⁹, NHC(=O)alkyl, -NHC(=O)O(C₁₋₆ alkyl), -NHC(=O)OH, -N(C₁₋₆ alkyl)C(=O)(C₁₋₆ alkyl), -NHSO₂(C₁₋₆ alkyl), amidino, -CHO, -C(=O)NR⁸R⁹, -C(=O)(C₁₋₆ alkyl), -C(=O)heterocyclyl, -C(=O)cycloalkyl, -COOH, -C(=O)O(C₁₋₆ alkyl), -C(=O)Oaryl, -C(=O)ONR⁷R⁸, mercapto, -S(C₁₋₆ alkyl), -SO(C₁₋₆ alkyl), -SO₂(C₁₋₆ alkyl), -SO₂-NR⁸R⁹,
R¹² and R¹³ are independently selected from H, C₁₋₆ alkyl, cycloalkyl, aryl, heterocyclyl; alternatively
R¹² and R¹³ together with the nitrogen atom to which they are attached form a heterocyclic ring.

2. The compound for use according to claim 1, wherein R¹ is selected from aryl optionally substituted on one or more carbon atoms by one or more R⁶, wherein, optionally, R¹ is phenyl optionally substituted on one or more carbons atoms by one or more R⁶, wherein R⁶ is preferably halo, more preferably fluoro, wherein, optionally, R¹ is 3-fluorophenyl.

3. The compound for use according to claim 1 or 2, wherein
(1) R⁵ is H; and/or
(2) R⁴ is a 5, 6, or 7-membered heterocyclyl ring containing at least one nitrogen atom, wherein R⁴ can be optionally substituted on one or more carbon atoms by one or more R⁶, and wherein if said heterocyclyl contains a -NH- moiety, the nitrogen of said moiety can be optionally substituted by a group selected from R⁷, wherein, optionally, R⁴ is a 6-membered heterocyclyl ring, preferably 3-piperidinyl, optionally substituted on one or more carbon atoms by one or more R⁶, and wherein the nitrogen of the a -NH- moiety can be optionally substituted by a group selected from R⁷

4. The compound for use according to any one of claims 1 to 3, wherein the compound is 5-(3-fluorophenyl)-N-(piperidin-3-yl)-3-ureidothiophene-2-carboxamide or a pharmaceutically acceptable salt thereof, preferably (S)-5-(3-fluorophenyl)-N-(piperidin-3-yl)-3-ureidothiophene-2-carboxamide or a pharmaceutically acceptable salt thereof, optionally the hydrochloride salt thereof.

5. The compound for use according to any one of claims 1 to 4, wherein the tumor has a drug resistant mutation, optionally an imatinib resistant mutation, sunitinib resistant mutation, regorafenib resistant mutation, ripretinib resistant mutation, avapritinib resistant mutation, or any combination thereof.

6. The compound for use according to any one of claims 1 to 5, wherein the tumor has a KIT 8 exon mutation, a KIT exon 9 mutation, a KIT exon 10 mutation, a KIT exon 11 mutation, a KIT exon 13 mutation, a KIT exon 14 mutation, a KIT exon 17 mutation, a KIT exon 18 mutation, a PDGFRA exon 12 mutation, a PDGFRA exon 13 mutation, a PDGFRA exon 14 mutation, a PDGFRA exon 15 mutation, or a PDGFRA exon 18 mutation, wherein optionally the mutation is a missense mutation, insertion mutation, a deletion mutation, or a combination thereof, wherein, optionally, the mutation is a primary mutation.

7. The compound for use according to any one of claims 1 to 6, wherein the tumor has:
- a KIT exon 9 mutation selected from the group consisting of p.A502_Y503dup, p.Y503_F504insAY, and p.S464L;
- a KIT exon 11 mutation selected from the group consisting of deletions involving codons 557/558, deletions upstream of codons 557/558, deletions downstream of codons 557/558, and deletions affecting codons 557/558;
- as a KIT exon 13 mutation selected from K642E, V65A, and T670I;
- a KIT exon 17 mutation selected from mutations involving codons 809, 816, 820, 822, or 823;
- a KIT exon 18 mutation selected from mutations involving codon 829,
- a PDGFRA exon 12 mutation selected from the group consisting of p.W559_R560del and p.581S;
- a PDGFRA exon 18 mutation selected from the group consisting of p.D842_M84del, p.D842_I843delinsV, and p.D842V.

8. The compound for use according to any one of claims 1 to 7, wherein the tumor has one or more PDGFRA mutations selected from the group consisting of V598F, G605A, G652E, P653Q, V658A, N659K/T, T674R/I, E675G, Y676C, G680R/E, D842V, and !843_D846de!, wherein, optionally,
(1) the tumor has the PDGFRA mutation D842V; or
(2) the tumor has the PDGFRA mutation G680R; or
(3) the tumor has the PDGFRA mutation G652E; or
(4) the tumor has the PDGFRA mutation V658A.

9. The compound for use according to any one of claims 1 to 8, wherein the tumor has one or more KIT mutations selected from the group consisting of S476I, F522C, K642E, V654A, N655K, T670E/I, D816A/E/G/H/N/V, D820A/E/G/H/V/Y, N822I/K/Y, Y823C/D, and A829P, wherein, optionally,
(1) the tumor has the KIT mutation T670I; or
(2) the tumor has the KIT mutation V654A; or
(3) the tumor has the KIT mutation D816A; or
(4) the tumor has the KIT mutation D820A; or
(5) the tumor has the KIT mutation N822K; or
(6) the tumor has the KIT mutation A829P.

10. The compound for use according to of any one of claims 1 to 9, wherein the tumor has at least one primary and at least one secondary mutation, wherein, optionally,
(1) the tumor has a primary KIT exon 9 mutation and a secondary KIT exon 13 mutation, a secondary KIT exon 14 mutation, a secondary KIT exon 17 mutation or a secondary KIT exon 18 mutation; or
(2) the tumor has a primary KIT exon 11 mutation and a secondary KIT exon 13 mutation, a secondary KIT exon 14 mutation, a secondary KIT exon 17 mutation or a secondary KIT exon 18 mutation; or
(3) the tumor has a primary KIT exon 13 mutation and a, a secondary KIT exon 14 mutation, a secondary KIT exon 17 mutation or a secondary KIT exon 18 mutation; or
(4) the tumor has a primary KIT exon 17 mutation and a secondary KIT exon 13 mutation, a secondary KIT exon 14 mutation, or a secondary KIT exon 18 mutation; or
(5) the tumor has a primary PDGFRA exon 12 mutation and, a secondary PDGFRA exon 13 mutation, a secondary PDGFRA exon 14 mutation, a secondary PDGFRA exon 15 mutation, or a secondary PDGFRA exon 18 mutation; or
(6) the tumor has a primary PDGFRA exon 18 mutation and a secondary PDGFRA exon 12 mutation, a secondary PDGFRA exon 13 mutation, a secondary PDGFRA exon 14 mutation, and a secondary PDGFRA exon 15 mutation.

11. The compound for use according to any one of claims 1 to 10, wherein the tumor has compound mutations selected from double-in-cis mutations or triple-in-cis mutations, wherein, optionally,
(1) the tumor has a primary KIT exon 9 mutation and at least one acquired mutation selected from mutations in KIT exon 13, KIT exon 14, KIT exon 17, and KIT exon 18, optionally wherein the tumor has a primary KIT exon 9 mutation and at least one acquired mutation in KIT exon 13 or KIT exon 14 and at least one acquired mutation in KIT exon 17 or KIT exon 18; or
(2) the tumor has a primary KIT exon 11 mutation and at least one acquired mutation selected from mutations in KIT exon 13, KIT exon 14, KIT exon 17, and KIT exon 18; optionally, wherein the tumor has a primary KIT exon 11 mutation and at least one acquired mutation in KIT exon 13 or KIT exon 14 and at least one acquired mutation in KIT exon 17 or KIT exon 18.

12. The compound for use according to any one of claims 1 to 11, wherein the patient was previously treated with one or more kinase inhibitors selected from imatinib, sunitinib, regorafenib, lapatinib, gefinitib, erlotinib, vatlanib, crenolanib, ripretinib, nintedanib, ponartinib, nilotinib, axitinib, cabozantinib, pazopanib, pexidartinib, bezuclastinib, dasatinib, sorafenib, and avapritinib, or a pharmaceutically acceptable salt thereof, wherein, optionally,
(1) the patient was previously treated with imatinib, sunitinib, regorafenib, ripretinib and/or avapritinib; and/or
(2) the patient was previously treated with imatinib; and/or
(3) the patient was previously treated with sunitinib; and/or
(4) the patient was previous treated with regorafenib; and/or
(5) the patient was previously treated with ripretinib; and/or
(6) the patient was previously treated with avapritinib.

13. The compound ofr use according to any one of claims 1 to 12, wherein
(1) the tumor is an advanced gastrointestinal stromal tumor that has progressed from, or the patient was intolerant to, administration of imatinib, administration of sunitinib, administration of regorafenib, administration of ripretinib and/or administration of avapritinib, or wherein the patient has a documented intolerance to one or more of imatinib, sunitinib, regorafenib, ripretinib, and avapritinib; and/or
(2) the tumor is an advanced gastrointestinal stromal tumor that has progressed from, or the patient was intolerant to, a first line administration of imatinib, a second line administration of sunitinib, and an optional third line administration of regorafenib or ripretinib, or wherein the patient has a documented intolerance to one or more of imatinib, sunitinib, regorafenib and/or ripretinib, wherein, optionally, the tumor has a KIT 8 exon mutation, a KIT exon 9 mutation, a KIT exon 10 mutation, a KIT exon 11 mutation, a KIT exon 13 mutation, a KIT exon 14 mutation, a KIT exon 17 mutation, or a KIT exon 18 mutation, preferably a KIT exon 9 mutation selected from the group consisting of p.A502_Y503dup, p.Y503_F504insAY, and p.S464L;
a KIT exon 11 mutation selected from the group consisting of deletions involving codons 557/558, deletions upstream of codons 557/558, deletions downstream of codons 557/558, and deletions affecting codons 557/558;
a KIT exon 13 mutation selected from K642E, V65A, and T670I; or
a KIT exon 17 mutation selected from mutations involving codons 816, 820 or 823; and/or
(3) the tumor is an advanced gastrointestinal stromal tumor that has progressed from, or the patient was intolerant to, administration of avapritinib, or wherein the patient has a documented intolerance to avapritinib, wherein, optionally, the tumor has a PDGFRA exon 12 mutation, a PDGFRA exon 13 mutation, a PDGFRA exon 14 mutation, a PDGFRA exon 15 mutation, or a PDGFRA exon 18 mutation, preferably a PDGFRA exon 12 mutation selected from the group consisting of p.W559_R560del and p.P581S; or
a PDGFRA exon 18 mutation selected from the group consisting of p.D842_M84del, p.D842_I843delinsV, and p.D842V.

14. The compound for use according to any one of claims 1 to 13, wherein the patient is a human patient.

15. The compound for use according to any one of claims 1 to 14, wherein the compound of formula (I) is comprised in a pharmaceutical composition together with a pharmaceutically acceptable carrier.
